# EUROPEAN PATENT SPECIFICATION

(11) **EP 4 248 447 B1**
(45) Date of publication and mention of the grant of the patent: **06.05.2026**
(21) Application number: 21895839.5
(22) Date of filing: 23.11.2021
(51) Int. Cl.: G16B 30/00, G01N 33/50, C12Q 1/68, G01N 33/487, C12Q 1/6853

(54) **ENCODED ASSAYS**
KODIERTE ASSAYS
DOSAGES CODÉS

(30) Priority: 23.11.2020 US 202063116997 P; 16.12.2020 US 202063126414 P; 08.03.2021 US 202163157924 P; 04.05.2021 US 202163183876 P; 17.07.2021 US 202163222963 P; 18.08.2021 US 202163234635 P
(43) Date of publication of application: 27.09.2023
(73) Proprietor: Pleno, Inc., San Diego, CA 92121 (US)
(72) Inventor: VAN ROOYEN, Pieter, San Diego, California 92121 (US); BERTI, Lorenzo, San Diego, California 92121 (US); BRODIN, Jeffrey, San Diego, California 92121 (US); EIDSON, Donald Brian, San Diego, California 92121 (US); STONE, Gavin, San Diego, California 92121 (US); POLLACK, Michael, San Diego, California 92121 (US); ECKHARDT, Allen, San Diego, California 92121 (US)
(74) Representative: Murgitroyd & Company
(86) International application number: PCT/US2021/060647
(87) International publication number: WO 2022/109496

(56) References cited:
- EP-A1- 3 805 408
- US-A1- 2016 010 085
- US-A1- 2020 149 037
- US-A1- 2020 309 784
- TOMASZ KRZYWKOWSKI: "Simultaneous Single-Cell In Situ Analysis of Human Adenovirus Type 5 DNA and mRNA Expression Patterns in Lytic and Persistent Infection", vol. 91, no. 11, 1 June 2017 (2017-06-01), US, XP093155243, ISSN: 0022-538X, Retrieved from the Internet <URL:https://www.ncbi.nlm.nih.gov/pmc/articles/PMC5432871/pdf/e00166-17.pdf> DOI: 10.1128/JVI.00166-17
- XIAOYIN CHEN ET AL: "Efficient in situ barcode sequencing using padlock probe-based BaristaSeq", NUCLEIC ACIDS RESEARCH, vol. 46, no. 4, 28 November 2017 (2017-11-28), GB, pages e22 - e22, XP055751607, ISSN: 0305-1048, DOI: 10.1093/nar/gkx1206
- DINH DIEP ET AL: "Library-free methylation sequencing with bisulfite padlock probes", NATURE METHODS, vol. 9, no. 3, 1 March 2012 (2012-03-01), New York, pages 270 - 272, XP055458110, ISSN: 1548-7091, DOI: 10.1038/nmeth.1871
- CHUNG ET AL.: "Deterministic droplet-based co-encapsulation and pairing of microparticles via active sorting and downstream merging", LAB ON A CHIP, vol. 17, no. 21, 25 October 2017 (2017-10-25), pages 2 - 9, XP055527069, DOI: 10.1039/c7lc00745k
- LAREAU ET AL.: "Droplet-based combinatorial indexing for massive-scale single- cell chromatin accessibility", NATURE BIOTECHNOLOGY, vol. 37, no. 8, August 2019 (2019-08-01), pages 916 - 924, XP036849994, DOI: 10.1038/s41587-019-0147-6

## Description

### 1. FIELD OF THE INVENTION

The invention relates to methods of conducting a methylation specific encoded assay, in which a target analyte is detected based on association of the target with a code, and detection of the code as a surrogate for detection of the target analyte. Methods, systems, cartridges, reagents, and kits for carrying out the encoded assays are also disclosed.

### 2. BACKGROUND OF THE INVENTION

Many assays require high-level of sensitivity and specificity, and are associated with low signal level. Low signal requires amplification (e.g., PCR, immunostaining cascades, and the like) resulting in complex and lengthy protocols, high-level of background and other biases limiting the performance of the assay. There is a need in the art for assays that are easier to read and detect at higher sensitivity than the analyte itself.

Krzywkowski, et al, "Simultaneous Single-Cell In Situ Analysis of Human Adenovirus Type 5 DNA and mRNA Expression Patterns in Lytic and Persistent Infection", J. Virology (2017), vol. 91, no. 11, describes a padlock probe-based rolling-circle amplification technique that enables simultaneous detection and analysis of HAdV-5 genomic DNA and virus-encoded mRNAs in individual infected cells.

WO 2017/177017 A1 provides compositions, methods and systems for quantifying target sequences and identifying target sequence variants.

WO 2018/160397 A1 provides a circular proximity ligation assay in which proximity-probes are employed as bridges to connect two free oligonucleotides via a dual ligation event, resulting in the formation of a circle. The circles can then be quantified by e.g. qPCR.

Diep, et al, "Library-free methylation sequencing with bisulfite padlock probes", Nature Methods (2012), vol. 9, no. 3 describes bisulfite padlock probes with a design algorithm to generate efficient padlock probes, a library-free protocol that reduces sample-preparation cost and time and is compatible with automation, and a bioinformatics pipeline to obtain both methylation levels and genotypes from sequencing of bisulfite-converted DNA.

### 3. SUMMARY OF THE INVENTION

The invention provides a method of conducting a methylation specific assay for a set of targets as defined in the appended claims.

The method may also include subjecting the amplified codes to a cleanup step. The method may also include counting detected codes and estimating target quantity-based counts of detected codes, e.g., estimating target quantity based on a correlation of counts of detected codes and target quantity in the sample analyzed.

In some cases, each code from the set of codes has a length ranging from 3 to 100 nucleotides. In some cases, each code from the set of codes has a length ranging from 3 to 75 nucleotides.

In some cases, each code from the set of codes is a predetermined code. In some cases, each code from the set of codes is selected to avoid interaction with other assay components. In some cases, each code from the set of codes is selected to ensure that it differs from each other code from the set of codes. In some cases, each code from the set of codes is homopolymer free. In some cases, each code from the set of codes is generated from a 4-ary nucleotide alphabet of A, C, G and T. In some cases, the code is generated using a 4-state encoding trellis with 3 transitions per state. In some cases, each code from the set of codes is generated from a 3-ary nucleotide alphabet of a set of three of A, C, G and T. In some cases, the code is generated using a 4-state encoding trellis with 3 transitions per state.

In certain configurations, the recognition elements includes one or more universal primers linked to the code. In certain configurations, the recognition elements includes a TaqMan sequence linked to the code. In certain configurations, each of the recognition elements include one or more unique molecular identifier sequences linked to the code.

In certain configurations, the recognition event includes binding a padlock probe of a set of padlock probes to each target of the set of targets. In some cases, each of the coded targets of the set of coded targets includes a target bound to one of the padlock probes of the set of padlock probes. In certain configurations, the set of targets include nucleic acid targets. In some cases, the recognition element includes a padlock probe including a code. In certain configurations, the padlock probe includes terminal primer sequences complimentary to a segment of one or more targets of the set of targets.

In certain configurations, a first one of the terminal primer sequences includes a terminal one or more nucleotides complementary to one or more target nucleotides; a second one of the terminal primer sequences includes one or more nucleotides complementary to a nucleotide adjacent to the one or more target nucleotides; when the one or more target nucleotides is/are present, the first one of the terminal primer sequences and second one of the terminal primer sequences cover a contiguous sequence of the target nucleic acid; and when the target nucleotide is not present, the first one of the terminal primer sequences and second one of the terminal primer sequences do not cover a contiguous sequence of the target nucleic acid.

In certain configurations, the transformation event includes subjecting the padlock probe to conditions which ligate the termini of the padlock probe in the presence of the target but not in the absence of the target. In certain configurations, the transformation event includes subjecting the recognition element to conditions which effect a ligation of the recognition element in the presence of the target but not in the absence of the target thereby producing a ligated recognition element. In certain configurations, the conditions which effect a ligation include contacting the recognition element with a ligase that effects a ligation of the recognition element in the presence of the target but not in the absence of the target. In some cases, the transformation event includes subjecting the padlock probes of the set of padlock probes to conditions which effect a ligation of the termini of the padlock probes to yield a circularized probe in the presence of the target but not in the absence of the target. In certain configurations, the method includes ligating the first and second of the terminal primer sequences using a ligating reagent that ligates the first and second of the terminal primer sequences only when the target nucleotide is present, thereby yielding a circularized padlock probe.

In certain configurations, the method includes amplifying the ligated recognition element. In some cases, the amplification yields a nanoball including multiple copies of the code. In certain configurations, the method includes amplifying the circularized probe. In some cases, the amplification yields a nanoball including multiple copies of the code. In some cases, amplification is rolling circle amplification. In certain configurations, the detection event includes determining the sequence of the code.

In certain configurations, providing a set of targets includes providing a set of targets from a sample selected from the group consisting of whole blood, lymphatic fluid, serum, plasma, sweat, tear, saliva, sputum, cerebrospinal fluid, amniotic fluid, seminal fluid, vaginal excretion, serous fluid, synovial fluid, pericardial fluid, peritoneal fluid, pleural fluid, transudates, exudates, cystic fluid, bile, urine, gastric fluid, intestinal fluid, fecal samples, liquids containing single or multiple cells, liquids containing organelles, fluidized tissues, fluidized organisms, liquids containing multi-celled organisms, biological swabs and biological washes.

In certain configurations, the targets include wild-type and/or mutated nucleic acid sequences. In certain configurations, the targets include point mutations. In certain configurations, the targets include substitutions, insertions and/or deletions. In certain configurations, the targets include copy number variations. In certain configurations, the targets include extracellular DNA fragments selected for methylation patterns indicative of cancer. In certain configurations, the targets include extracellular DNA fragments. In certain configurations, the targets include extracellular DNA fragments from blood, plasma and/or serum. In certain configurations, the targets are selected to contribute to cancer screening or diagnosis.

Disclosed herein is a method of accumulating targets on beads. The method may include (a) providing on a droplet actuator: a first droplet set including the targets; a set of bead pools, each pool including beads wherein beads of each pool include a different set of bound target capture probes; (b) using droplet operations, contacting one or more droplets of the first droplet set with one or more bead populations and thereby capturing targets with the nucleic acid target capture probes, yielding a second droplet set including bead populations and captured nucleic acid targets; (c) using droplet operations, transporting one or more droplets of the second droplet set into contact with a different bead population of the bead population set, and thereby capturing nucleic acid targets with the nucleic acid target capture probes, yielding a third droplet set including the bead populations and additional captured nucleic acid targets; and repeating step (c) one or more times to yield an nth droplet set wherein n is four or more.

In some cases, the first droplet set includes 100 or more nucleic acid targets. In some cases, the first droplet set includes 1000 or more nucleic acid targets. In some cases, the first droplet set includes 10000 or more nucleic acid targets. In some cases, the first droplet set includes 50000 or more nucleic acid targets. In some cases, the first droplet set includes 100000 or more nucleic acid targets. In some cases, the first droplet set includes 1000000 or more nucleic acid targets. In some cases, the first droplet set includes 10000000 or more nucleic acid targets.

In certain configurations, the first droplet set includes 10 or more droplets. In certain configurations, the first droplet set includes 100 or more droplets. In certain configurations, the first droplet set includes 1000 or more droplets.

In certain configurations, the number of bead pools is equivalent to the number of droplets. In certain configurations, the number of bead pools is more than the number of droplets. In certain configurations, the number of bead pools is less than the number of droplets. In certain configurations, the bead pools include magnetically responsive beads. In some cases, each bead population is provided in a droplet in a filler fluid.

In some cases, the droplet is provided on a droplet actuator. In some cases, the droplet is provided on an electrowetting droplet actuator.

Disclosed herein is an assay substrate including a set of arrays, each array including detection features arranged in n columns and m rows, each detection feature including assay reagents for detecting a nucleic acid code having a sequence is associated with a target but is not a sequence of the target wherein the set of arrays is arranged within a larger array arranged in x columns and y rows.

In certain configurations, in at least one array the assay reagents include primers for amplification of the nucleic acid code. In certain configurations, in at least one array the assay reagents include primers for amplification of a single nucleic acid code. In certain configurations, in a set, the primers of each detection feature are a unique combination of primers relative to other wells in the set. In certain configurations, in a set, the primers in each column of detection features are a unique combination of primers relative to other columns in the set. In certain configurations, in a set, the primers of each row of detection features are a unique combination of primers relative to other rows in the set.

In certain configurations, in a set, the primers of the set collectively include a number of unique primers equal to x + y. In certain configurations, in a set, the primers of the set collectively include a number of unique primers equal to x = y. In certain configurations, in a set, the primers of the set collectively include a number of unique primers equal to x * y. In certain configurations, in a set, the primers of the set collectively include a number of unique primers ranging from x = y to x * y.

In certain configurations, each detection feature includes one forward primer and one reverse primer for a target. In certain configurations, each detection feature includes one forward primer and one reverse primer for a control sequence. In certain configurations, in at least one array the assay reagents include antibodies or parts of antibodies for capture of a polypeptide. In certain configurations, in at least one array the assay reagents include primers for amplification of a nucleic acid code; and in at least one array the assay reagents include antibodies for capture of a nucleic acid code. In certain configurations, the detection feature is selected from tr group consisting of indentations, wells, protrusions, domes, posts, beads, beads-in-wells, spots, hydrophilic spots and combinations of the foregoing.

In certain configurations, the detection feature includes a well. In certain configurations, the detection feature includes an indentation. In certain configurations, the detection feature includes a post. In certain configurations, the detection feature includes a molecular wire.

In certain configurations, in each array of the set of arrays, n and m are each at least about 10. In certain configurations, in each array of the set of arrays, n and m are each at least about 100. In certain configurations, in each array of the set of arrays, n and m are each at least about 1000. In certain configurations, the primers in each of the arrays of the larger array are unique relative to other arrays in the large array.

In certain configurations, in the larger array, either x or y is at least 2. In certain configurations, in the larger array, x + y is at least 10. In certain configurations, in the larger array, x + y is at least 100. In certain configurations, in the larger array, x + y is at least 200. In certain configurations, the combination of n, m, x and y results in at least 1,000 uniquely detectable targets. In certain configurations, the combination of n, m, x and y results in at least 10,000 uniquely detectable targets. In certain configurations, the combination of n, m, x and y results in at least 100,000 uniquely detectable targets. In certain configurations, the combination of n, m, x and y results in at least 1,000,000 uniquely detectable targets.

In certain configurations, the reagents are dried onto or in the detection features. In certain configurations, the target includes a target nucleic acid sequence from a subject. In certain configurations, the target includes a code sequence that corresponds to a target nucleic acid sequence from a subject. In certain configurations, the reagents are chemically coupled to magnetic beads attached to the detection features.

Disclosed herein is a cartridge including the assay substrate provided as a component of a droplet operations device configured to conduct droplet operations on the assay substrate, e.g., dispensing a droplet onto the assay substrate, splitting a droplet on the assay substrate, transporting a droplet onto the assay substrate, and transporting a droplet from array to array on the assay substrate. Disclosed herein is a cartridge including the assay substrate and a substrate including an array of electrowetting electrodes configured to transport a droplet into contact with the assay substrate. Disclosed herein is a cartridge including the assay substrate and a substrate including an array of electrowetting electrodes configured to transport a droplet into contact with each of the arrays of the set of arrays.

Disclosed herein are systems for operating cartridges as disclosed herein. The systems may include an instrument including a controller electronically coupled to and controlling the cartridge. The system may be electronically connected to a computer via a network and programmed to transmit data to the computer. Other components of a system are described herein, e.g., detection elements, temperature control elements, networking elements, and the like.

Disclosed herein are methods for detecting multiple analytes using the assay substrates. The methods may be conducted using droplet operations mediated by a droplet actuator. The methods may include contacting a droplet including a set of targets or a subset of the set of targets with an array of the set of arrays, to capture the targets on the detection features. The methods may include subjecting targets from the set of targets a recognition event at the detection features, in which each target is uniquely recognized by and bound to a recognition element associated with a code, thereby yielding a set of coded targets including the target and the recognition element. The methods may include subjecting each recognition element of the set of coded targets to a transformation event at the detection features, in which a molecular transformation of each recognition element produces a modified recognition element, thereby yielding a set of modified recognition elements comprising the code. The methods may include subjecting each code of the set of modified recognition elements to an amplifying event at the detection features, in which each code is amplified, thereby yielding a set of amplified codes. The methods may include subjecting each amplified code of the set of amplified codes to a detection event, thereby determining the nucleic acid sequence of the code.

In certain configurations, subjecting each amplified code of the set of amplified codes to a detection event is conducted at the detection features. In certain configurations, including using droplet operations to transport a droplet including the amplified codes away from the detection features prior to subjecting each amplified code of the set of amplified codes to a detection event. In certain configurations, subjecting each code of the set of modified recognition elements to an amplifying event includes rolling circle amplification of the code to yield a nanoball including repeated instances of the code. In certain configurations, subjecting each code of the set of modified recognition elements to an amplifying event includes subjecting the code to bridge amplification to yield an oligo patch including repeated instances of the code.

In certain configurations, set of targets include nucleic acid targets. In certain configurations, set of targets include polypeptide targets. In certain configurations, set of targets include polypeptide targets and nucleic acid targets. In certain configurations, each code from the set of codes has a length ranging from 3 to 100 nucleotides. In certain configurations, each code from the set of codes has a length ranging from 3 to 75 nucleotides.

In certain configurations, each code from the set of codes is a predetermined code. In certain configurations, each code from the set of codes is a predetermined code from a dataset. In certain configurations, each code from the set of codes is selected to avoid interaction with other assay components. In certain configurations, each code from the set of codes is selected to ensure that it differs from each other code from the set of codes. In certain configurations, each code from the set of codes is homopolymer free. In certain configurations, each code from the set of codes is generated from a 4-ary nucleotide alphabet of A, C, G and T. In some cases, the code is generated using a 4-state encoding trellis with 3 transitions per state. In certain embodiments, each code from the set of codes is generated from a 3-ary nucleotide alphabet of a set of three of A, C, G and T. In some cases, the code is generated using a 4-state encoding trellis with 3 transitions per state.

In certain configurations, the recognition element includes one or more universal primers. In certain configurations, the recognition element includes a TaqMan sequence. In certain configurations, the recognition element includes one or more unique molecular identifier sequences.

In certain configurations, methylated targets have bases transformed (e.g., bisulfite or enzymatic sample prep) prior to detection (e.g., sequencing, nanopore sequencing, or other sequence determination). In other configurations, methylated targets do not undergo transformation (e.g., bisulfite or enzymatic sample prep) prior to detection (e.g., sequencing, nanopore sequencing or other sequence determination). The disclosure includes detection of any epigenetic modification of nucleic acids.

### 4. BRIEF DESCRIPTION OF DRAWINGS

The features and advantages of the present disclosure will be more clearly understood from the following description taken with the accompanying drawings, which are not necessarily drawn to scale, and wherein:
FIG. 1 is a diagram illustrating an encoding method that uses a 4-state encoding trellis with 3 transitions per state.
FIG. 2 is a diagram illustrating an encoding trellis for a 4-bases-per-cycle pyrosequencing.
FIG. 3 is a diagram illustrating a pyro-code example, followed by a snapshot from a spreadsheet with relevant parameters.
FIG. 4 is a block diagram illustrating an example of droplet actuator system including a droplet operations device.
FIG. 5 is a block diagram of an example of droplet operations device of the microfluidics system including various DMF electrode configurations.
FIG. 6 is a flow diagram of an example of a nucleic acid assay workflow and shows an example of an array detector.
FIG. 7 is a schematic diagram of an example of an array detector shown in FIG. 6, which may be, for example, an AM-DMF CMO array detector.
FIG. 8 is a table indicating a droplet magnetic bead incubation workflow that includes 10 bead enrichment cycles.
FIG. 9 is a schematic diagram showing an example of a coded padlock probe ligation reaction.
FIG. 10 is a schematic diagram showing a universal amplification reaction that may be used to produce linear duplex nucleic acid copies.
FIG. 11 is a schematic diagram of an example of a detection zone layout representing the grid of wells found in each detection zone A-J of CMOS array detector shown in FIG. 7.
FIG. 12 is a schematic diagram of an example showing the use of RNAse H2 primers to increase specificity in a coded padlock probe ligation reaction.
FIG. 13 is a schematic diagram of an example of a process including coded padlock probes incorporating TaqMan probe sequences.
FIG. 14 is a schematic diagram of an example of a coded padlock probe incorporating UMI sequences.
FIG. 15 is a flow diagram of an example of a process, which is a process of methylation detection assay using bisulfite conversion.
FIG. 16 is a flow diagram of an example of a process, which is a workflow for a methylated base detection assay with Roswell molecular wires.
FIG. 17 is a schematic diagram of an example of a process that uses an array detector zone for Roswell molecular wire detection.
FIG. 18 is a schematic diagram of an example of a process, which is an example of a target DNA strand annealing to a Roswell molecular wire capture probe.
FIG. 19 is a schematic diagram showing methylated-cytosine NA binding domain protein binding to a target sequence.
FIG. 20 is a nucleic acid assay workflow for Roswell molecular wire detection.
FIG. 21 is a schematic diagram showing ligation of a padlock probe that includes a molecular wire hybridization sequence.
FIG. 22 is a schematic diagram showing exonuclease digestion of circularized and linear padlock probes.
FIG. 23 is a schematic diagram showing a circularized padlock probe annealing to a molecular wire capture probe.
FIG. 24 is a flow diagram of an example of a process and showing a methylation-specific inhibition of ligation assay workflow using probes, proteins, and/or aptamers.
FIG. 25 is a flow diagram showing an example of a process of detecting methylated nucleic acid using methylation-specific restriction enzymes.
FIG. 26 illustrates a process of using dual hybridization probes in combination with a methylation sensitive endonuclease digestion reaction to differentiate a methylated target sequence and an unmethylated target sequence.
FIG. 27A is a schematic diagram of an example of a code element probe designed to query a target sequence for a variant of interest.
FIG. 27B is a schematic diagram of an example of a set of randomers that may be used to query (probe) a variant site of interest in a targeted sequence.
FIG. 28 is a flow diagram of an example of a method of using a probe that includes a code element combined with a recognition element and a set of coded randomers to query a variant of interest in a target sequence.
FIG. 29A and FIG. 29B illustrate pictorially the steps of the method FIG. 28.
FIG. 30A and FIG. 30B are panels of a schematic diagram showing an example of identifying mismatches and error correction in a variant sequence detection process.
FIG. 31 is a flow diagram of an example of an immuno-PCR assay workflow, wherein a capture antibody is used to detect a protein bound to a target sequence.
FIG. 32 is a schematic diagram showing an example of magnetic beads and capture antibodies bound to the magnetic beads that may be used in an immune-PCR assay.
FIG. 33 is a pair of plots showing qPCR amplification plots after 30 minutes and 2 hours, respectively, of cutting with Hha I.
FIG. 34 is a schematic diagram showing an example of a code element probe and some of the workflow steps in the multiplexed assay.
FIG. 35 is a schematic diagram and a plot showing the specificity of target interaction in the multiplexed assay.
FIG. 36 is a schematic diagram and a plot detection of allele ratio in the multiplexed assay.

### 5. DETAILED DESCRIPTION

### 5.1 Definitions

"A," "an" and "the" include their plural forms unless the context clearly dictates otherwise.

"About" means approximately, roughly, around, or in the region of. When "about" is used with a numerical range, it modifies that range by extending the boundaries above and below the numerical values indicated. "About" can modify a numerical value above and below the stated value by a variance of, e.g., 10 percent up or down (higher or lower).

"Activate," with reference to one or more electrodes, means affecting a change in the electrical state of the one or more electrodes which, in the presence of a droplet, results in a droplet operation.

"And" is used interchangeably with "or" unless expressly stated otherwise.

"Droplet Actuator" means a fluid handling device for use in manipulating droplets. Examples include electrowetting devices, dielectrophoresis devices, robotics devices, microfluidics devices, and manual devices for manipulating droplets.

"Detection features" with reference to the CMOS detector, may be any arrayed topographical feature, including without limitation, indentations, wells, protrusions, domes, posts, beads, beads-in-wells, spots, hydrophilic spots, etc.

"Droplet operation" means any manipulation of a droplet on or by a droplet actuator. A droplet operation may, for example, include: loading a droplet into the droplet actuator; dispensing one or more droplets from a source droplet; splitting, separating or dividing a droplet into two or more droplets; transporting a droplet from one location to another in any direction; merging or combining two or more droplets into a single droplet; diluting a droplet; mixing a droplet; agitating a droplet; deforming a droplet; retaining a droplet in position; incubating a droplet; heating a droplet; vaporizing a droplet; cooling a droplet; disposing of a droplet; transporting a droplet out of a droplet actuator; other droplet operations described herein; and/or any combination of the foregoing. "Electrically connected," "electrical connection," "electrically coupled," and the like are intended to refer to a connection that is capable of transmitting electricity, e.g., a wired connection.

"Electronically connected," "electronic connection," "electronically coupled" and the like are intended to include both wired and wireless connections, including without limitation connections that are capable of transmitting data signals, e.g., electrical signals, electromagnetic signals, and optical signals. A component electronically coupled to another component may located together, e.g., in a common device or instrument, or in the same room or facility, or may be located separately and electronically connected via a network. Similarly, an "electronic signal" means any signal, whether transmitted electrically, optically or wirelessly.

"Filler fluid" means a fluid associated with a droplet operations substrate of a droplet actuator, which fluid is sufficiently immiscible with a droplet phase to render the droplet phase subject to electrode-mediated droplet operations.

"Include," "including," and the like are to be construed in an inclusive sense as opposed to an exclusive or exhaustive sense; that is to say, in the sense of "including, but not limited to."

"Linked" with respect to two nucleic acids means not only a fusion of a first moiety to a second moiety at the C-terminus or the N-terminus, but also includes insertion of the first moiety to the second moiety into a common nucleic acid. Thus, for example, the nucleic acid A may be linked directly to nucleic acid B such that A is adjacent to B (-A-B-), but nucleic acid A may be linked indirectly to nucleic acid B, by intervening nucleotide or nucleotide sequence C between A and B (e.g., -A-C-B- or -B-C-A-). The term "linked" is intended to encompass these various possibilities.

"On" or "loaded on" with respect to a droplet on a droplet actuator indicates that the droplet is arranged on the droplet actuator in a manner which facilitates using the droplet actuator to conduct one or more droplet operations on the droplet, the droplet is arranged on the droplet actuator in a manner which facilitates sensing of a property of or a signal from the droplet, and/or the droplet has been subjected to a droplet operation on the droplet actuator.

"Optimum," "optimal," "optimize" and the like are not intended to limit the disclosed configurations to the absolute optimum state of the aspect or characteristic being optimized but will include improved but less than optimum states.

"Reservoir" means an enclosure or partial enclosure configured for holding, storing, or supplying liquid. A droplet actuator may include reservoirs. For example, a pipette tip or feature on a multiwell plate may be a reservoir. An electrowetting device may include reservoirs, which may be on or off-cartridge reservoirs.

"Sample" means a source of target or analyte. Examples of samples include biological samples, such as whole blood, lymphatic fluid, serum, plasma, sweat, tear, saliva, sputum, cerebrospinal fluid, amniotic fluid, seminal fluid, vaginal excretion, serous fluid, synovial fluid, pericardial fluid, peritoneal fluid, pleural fluid, transudates, exudates, cystic fluid, bile, urine, gastric fluid, intestinal fluid, fecal samples, liquids containing single or multiple cells, liquids containing organelles, fluidized tissues, fluidized organisms, liquids containing multi-celled organisms, biological swabs and biological washes.

"Set" includes sets of one or more elements or objects. A "subset" of a set includes any number elements or objects from the set, from one up to all of the elements of the set.

"Subject" includes any mammal, including without limitation, humans.

"Target" with respect to a nucleic acid includes wild-type and mutated nucleic acid sequences, including for example, point mutations (e.g., substitutions, insertions and deletions), chromosomal mutations (e.g., inversions, deletions, duplications), and copy number variations (e.g., gene amplifications). "Target" with respect to a polypeptide includes wild-type and mutated polypeptides of any length, including proteins and peptides.

"Washing" with respect to washing a surface, such as a hydrophilic surface, means reducing the amount and/or concentration of one or more substances in contact with the surface or exposed to the surface from a droplet in contact with the surface. The reduction in the amount and/or concentration of the substance may be partial, substantially complete, or even complete. The substance may be any of a wide variety of substances; examples include target substances for analysis, and unwanted substances, such as components of a sample, contaminants, and/or excess reagent or buffer. Examples of bead washing protocols are set forth in US Patent 8,637,324, entitled "Bead incubation and washing on a droplet actuator," issued on 2014-01-28, the entire disclosure of which is incorporated herein by reference.

Headings are included herein for reference and to aid in locating the various sections. These headings are not intended to limit the scope of the concepts described with respect to the headings.

### 5.2 Encoded assays

The invention provides encoded assays. At a high level, in an encoded assay, a target analyte ("target") is detected based on association of the target with a code, and detection of the code as a surrogate for detection of the analyte.

In various configurations, an encoded assay may include a recognition event in which a target is uniquely recognized by a recognition element. The recognition event may be effected by submitting targets of a set of targets to a recognition event, in which each target is uniquely recognized by and bound to a recognition element associated with a code, thereby yielding a set of coded targets comprising the target and the recognition element.

In various configurations, an encoded assay may include a transformation event, in which a high-fidelity molecular transformation of the target analyte produces a modified recognition element. The transformation event may be effected by submitting each recognition element of the set of coded targets to a transformation event, in which a molecular transformation of each recognition element produces a modified recognition element, thereby yielding a set of modified recognition elements comprising the code.

In various configurations, an encoded assay may include a detection event, which detects the code as a surrogate for detection of the analyte, e.g., by recognizing or determining the sequence of the code (and optionally other elements). The detection event may include an amplification step in which each code of the set of modified recognition elements is amplified, thereby yielding a set of amplified codes. Amplified codes of the set of amplified codes may have their sequences determined using a variety of techniques, including for example, microarray detection, or nucleic acid sequencing. In some cases, the detection step may be integrated with the amplification step, e.g., as in amplification with intercalating dyes.

In one configuration, the method may include:
(i) submitting each target of a set of targets to a recognition event, in which each target is uniquely recognized by and bound to a recognition element associated with a code, thereby yielding a set of coded targets comprising the target and the recognition element;
(ii) submitting each recognition element of the set of coded targets to a transformation event, in which a molecular transformation of each recognition element produces a modified recognition element, thereby yielding a set of modified recognition elements comprising the code;
(iii) submitting each code of the set of modified recognition elements to an amplifying event, in which each code is amplified, thereby yielding a set of amplified codes;
(iv) submitting each amplified code of the set of amplified codes to a detection event, thereby determining the nucleic acid sequence of the code.

In one configuration, the method may include:
(i) a recognition event in which the target is uniquely recognized by a recognition element, and which associates a code (and optionally other elements) with the target via the recognition element;
(ii) a transformation event, in which a high-fidelity molecular transformation of the target analyte produces a modified recognition element;
(iii) a detection event, which detects the code as a surrogate for detection of the analyte, e.g., by recognizing or determining the sequence of the code (and optionally other elements).

As described in more detail herein, the recognition event, transformation event, coding event, and the detection event may occur sequentially, or combinations of the steps may occur simultaneously, e.g., as a single combined step. For example, the transformation event and the coding event may be simultaneous, such that the sequential process involves (i) recognition event, followed by (ii) transformation event/coding event, followed by (iii) detection event.

To further illustrate the encoded assays:
(i) In the recognition event, the target may be detected by a targeted molecular binding event, such as binding of the target by a complementary sequence or a polypeptide binder.
(ii) In the transformation event, a ligation or a gap-fill ligation may produce the modified recognition element, i.e., a version of the recognition element that is ligated or gap-fill ligated.
(iii) In the coding event, a code reagent may be associated with the modified recognition element based on recognition of the modified recognition element. For example, the novel coded padlock probes of the disclosure may be configured with a sequence that recognizes the modified recognition element and circularize only if the modified recognition element is present.
(iv) In the detection event, the code may involve any means of determining the sequence of the code (and optionally other elements).

The codes may be error corrected and thus easy to distinguish from each other, so they can be detected a low abundance and in the presence of high level of background and in the presence of many other codes.

Since many assays can be converted into codes, the disclosure provides multi-omic assays where a sample is analyzed in multiple parallel workflows that are analyte-dependent and then converge codes that can be then detected simultaneously in a single platform. Parallel assay workflows may be merged into a single workflow, where multiple targets and target-types (e.g., nucleic acids and polypeptides) may be detected simultaneously in a single workflow and also read simultaneously within the same readout platform.

Following detection, codes may be decoded and matched to targets for identification and/or quantification of targets present in the sample.

### 5.3 Code design and decode

The encoded assays of the disclosure make use of codewords or codes. The codes may be sequenced as surrogates in the place of direct analysis of target analytes. As an example, a target analyte may be a particular nucleic acid fragment (e.g., a nucleic acid fragment with a specific mutation); in the assays of the invention, a codeword may be associated with the nucleic acid fragment and the codeword may be read to identify the presence of the nucleic acid fragment in the sample.

For example, a code may be a predetermined sequence ranging from about 3 to about 100 nucleotides or about 3 to about 75 nucleotides. Codes may have sequences selected to avoid inadvertent interaction with other assay components, such as targets, probes, or primers. Code sequences may be selected to ensure that codes differ from each other to permit unique identifiability during the decoding process.

The disclosure includes a dataset or database of codes generated using the methods of the disclosure. The dataset or database may associate the codes with other assay elements, such as primers or probes linked to the probes. The disclosure also includes a method of making a probe set comprising synthesizing probes having the sequences set forth in the dataset or database.

### 5.3.1 Homopolymer-free encoding

In one configuration, the codes are homopolymer-free codes. For standard genomic applications that use a full 4-ary nucleotide alphabet of {ACGT}, the method uses a 4-state encoding trellis with 3 transitions per state.

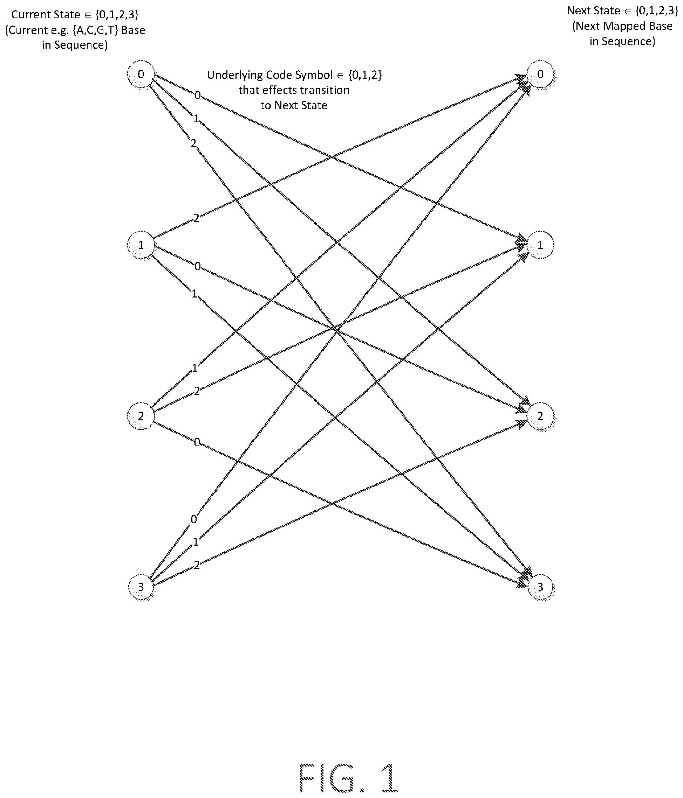

As illustrated in FIG. 1, the current state is the last mapped nucleotide, and the next state is the next (to-be) mapped nucleotide. By forbidding a transition from the current state (say, the 'A' state) in the present trellis section (of 4 states), to the analogous same state (of 'A') in the next trellis section (of 4 states), a repeated mapping to the same nucleotide base--in any generated sequence---is avoided. An 'A' state can only transition to a 'C', 'G', or 'T' state in the next trellis section. Since this involves 3 transitions per state, the mapping trellis is mated to an underlying 3-ary (i.e., ternary-) alphabet error correction code that drives transitions through trellis sections. The underlying (ternary) error correction code is the mechanism that guarantees all generated codewords differ in multiple sequence positions.

A similar method may apply to 3-ary alphabets (where only 3 of the four nucleotide bases, say {CGT} are used), and 5-ary or higher alphabets, where the underlying correction code uses an alphabet of order one less than the mapping alphabet.

In one configuration, codes for the set of codes are selected using a 4-ary alphabet, avoid homopolymers, and every code in the set is different from every other code in the set. The codes may be generated using the trellis method.

In one configuration, codes for the set of codes are selected using a 3-ary alphabet, avoid homopolymers, and every code in the set is different from every other code in the set. The codes may be generated using the trellis method.
(v) In another configuration, a homopolymer-free code composed from a 4-ary nucleotide alphabet of {ACGT} may be generated as follows:
(i) From GF(4) (i.e., the quaternary algebraic alphabet), select an error correction code that will deliver many more codewords than necessary (because some of the generated codewords will later be eliminated);
(ii) Generate all of the codewords for the code;
(iii) Assess the number of repeated symbol locations in each codeword;
(iv) Re-order the list of codewords, sorting by the number of base-repeat instances in each codeword.
(v) From the re-ordered sort, keep only the top K codewords, where K is the desired library size of codewords (this will eliminate the codes with the highest number of polymer-repeats; each repeat will require subsequent fixing that weakens the overall code.)
(vi) For each codeword in the list of survivors, 'smart fix' the repeat positions in each codeword with the following procedure:
   a. Start from the beginning base position in a codeword, and find the first repeat instance of a base;
   b. Go to the second base in the first repeat instance, its base assignment will require change;
   c. If the second base is not at the end of a codeword, look ahead one base position in the codeword, and assess the assignment there;
   d. For the second base (in the repeat), choose a new base assignment that is also different from the base assigned one sample ahead; n that, in addition to removing a length-2 run, this step will also fix a length-3 run;
   e. Process the revised codeword at each remaining repeat location, fixing the second base in each repeat using the process outlined in steps c-d.

This method will eliminate all repeats. The same method can be applied to generate homopolymer codes for 3-ary alphabets (eg., {C, G, T}), and larger 5-ary+ alphabets (such as oligopolymers).

### 5.3.2 Codes optimized for pyrosequencing and similar cyclic serial dispensation schemes

The disclosure provides a locus code-encoding approach for pyrosequencing or similar serial (rather than pooled) primer dispensation methods. The method generates homopolymer-free codes.

When the locus code is encapsulated between header and tail bases, all generated codewords finish decoding at the same time. The technique avoids unexpected spurious hybridizations that change how long in time that a codeword needs to finish its hybridization. This is important because then a sequencer only need sample for a prescribed number of samples to obtain complete data for decoding the samples, regardless of the underlying codeword. This also keeps all codewords candidates aligned, so that the theoretical design distances between codewords are maintained.

The aforesaid synchrony ensures that soft decision block decoding techniques (c.f., disclosures 9 and 10) can be applied during the decoding of its blocks of samples. This soft decision decoding guarantees that SNR requirements are improved by at least 2 dB---and sometimes by many factors-more when the signal strength significantly fades during the reception of codeword samples.

In pyrosequencing, primers are dispensed sequentially (and non-overlappingly) in a cycle, such as G, C, T, A, G, C, T, A, G, C, ... etc. This encoding is quite original because it doesn't directly encode bases; instead, it encodes base POSITIONs within G, C, T, A cycles. Each cycle element can be either populated, or unpopulated---and multiple elements within a cycle can be populated. For this to be implemented, the underlying code must be derived from a binary alphabet, with 1s and 0s. To emphasize, with these codes, more than one base can be hybridized within a single G, C, T, A dispensation cycle. This also implies that sequencing, though serial in nature, can be fast. And with the underlying {0,1} alphabet that underpins and drives the encoding of the populated/unpopulated cycle positions, all codewords are guaranteed to be of the same length---and to finish decoding in the same amount of time.

To provide coding gain, the sequence of 0s and 1s that compose each codeword are derived from constructions of optimal binary error correction codes. Such codes possess many redundant parity bits, and these parity bits are designed such that each codeword varies from each other in multiple positions. This quality results in strong error correction capabilities.

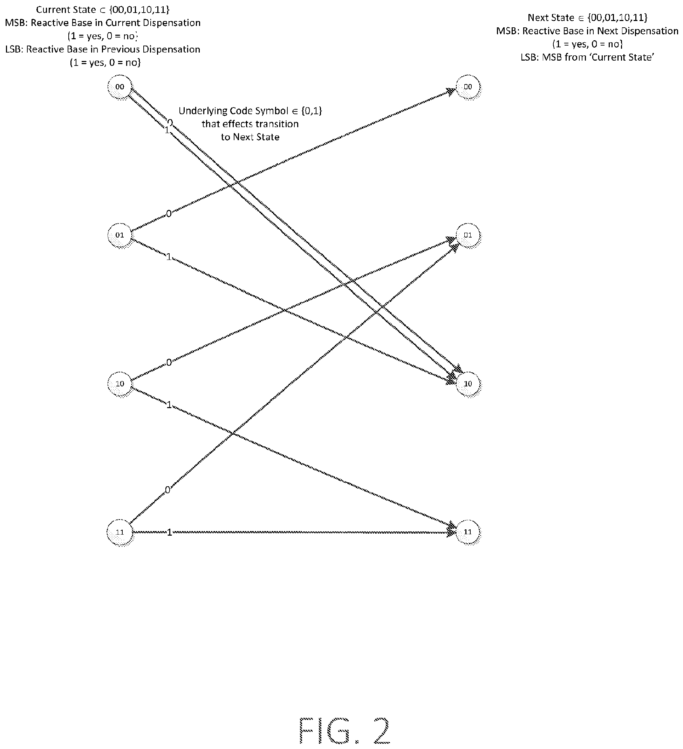

FIG. 2 illustrates an encoding trellis for a 4-bases-per-cycle pyrosequencing. The techniques may be used for encoding 3-cycle, 3-base-alphabet, and 5+-cycle, 5-and-higher-alphabet oligo-polymer hybrid schemes.

Note the use of 4 states in the trellis. Each state represents previous mappings of that last two positions:
(i) both unpopulated, (00);
(ii) both populated, (11);
(iii) newest-populated and older-unpopulated, (10);
(iv) newest-unpopulated and the older populated, (01).

Transitions to next states indicate an update which either does not populate or does populate the next position in a sequence.

4 states are used to correctly implement a pyrosequencing scheme that is homopolymer-free; one position is populated every 3 positions. Note that if 3 consecutive positions were allowed to be unfilled, then the 4^{th} position would need to be filled (because an unzipped hybrid will have an opening to at least one of the four nucleotides). That 4^{th} position being filled would result in generation of a homopolymer (repeat) of bases in a sequence---since the last filled base was the same base in the cycle before.

This aforementioned restriction explains the double transition from the 00 state to the 10 state in the trellis diagram. A current state of 00 transitioning to a next state of 00 would imply 3 positions in a row were unfilled.

Optimal error correction codes are constructed in maximize distance between their sets of codewords. They are not constrained to disallow runs of three consecutive zeros. That would reduce the degrees of freedom they use to maximize distance. By contrast, the mappings to pyro-sequenced positions comply with homopolymer-free and pyrosequencing constraints.

All other transitions in the picture design trellis are natural results of populating a position with a '0' or a '1' and updating the next state to reflect that transition. Since 7 of the 8 transitions in the trellis perfectly express the underlying error correction code's structure, such a code can be quite effective and powerful.

Weakening transitions occur when the underlying code has 3 consecutive zeros. One way to reduce those appearances is to use the sorting methodology described above. This method modestly reduces the library of codes. This method also ensures that the pyro-mapped codewords that best reflect the underlying binary code's structure are faithfully reproduced, while those least reflective are not.

Another method to improve the weakening due to transitions involves breaking up strings of zeros by interleaving the code. Within a code, the (systematic) information section of bits---which precede the redundant section of parity bits---are the bits where the most consecutive zeros are usually seen. One way to eliminate those strings of zeros is to interleave the entire code design, so that the parity and information bits are intermingled. All codewords may be intermingled by the same interleaving pattern. The interleaving technique does not help for the all-zeros codeword, which is generated by almost all linear codes. The all-zeros codeword can be excluded from the codeword set.

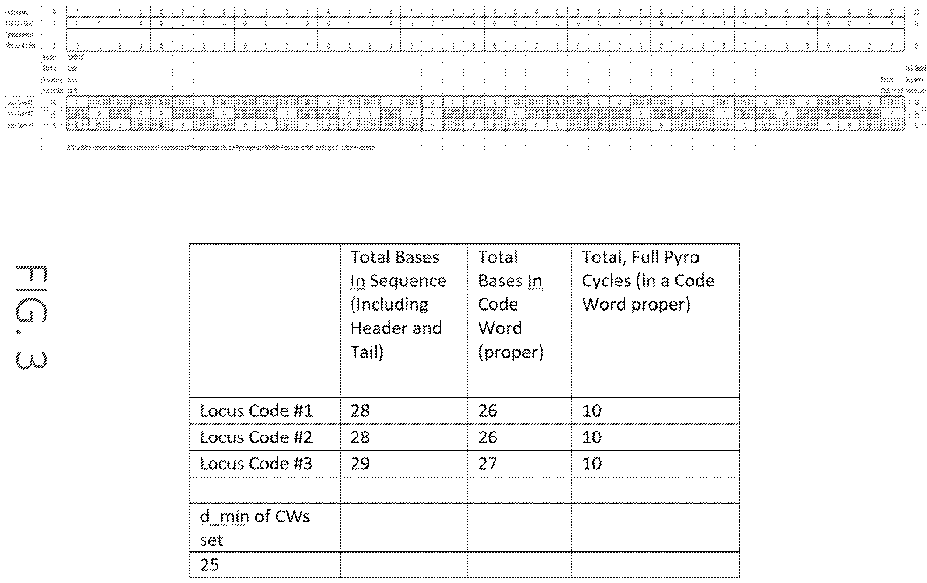

FIG. 3 shows a pyro-code example, followed by a snapshot from a spreadsheet with relevant parameters. The code is a 10-cycle, 40 position code that maps {GCTA} in cycles. It possesses a huge minimum distance between codewords, and is an example code accommodating three codewords. Note that the number of bases assigned to each codeword is not the same, although, clearly, from the illustration, all codewords are of the same time duration, and would finish decoding at the same time. Observe also the usage of populated 'header' and 'tail' positions. These are used to encapsulate the codeword and ensure that it is homopolymer free throughout. These terminating positions may be butted-up against the ends of the codewords for effective encapsulation.

### 5.4 Amplifying and reading codes

In an encoded assay, a target is detected based on association of the target with a code, and detection of the code as a surrogate for detection of the analyte. A variety of techniques may be used to amplify and read the codes. Examples include nanoballs, oligo clusters, oligo amplicons, bead-attached oligos, patterned oligos, and microarrays.

In one configuration, codes of the disclosure are amplified using rolling circle amplification to produce nanoballs that include many duplicates of the probes.

In one configuration, codes of the disclosure are amplified using bridge amplification to produce clusters of oligos on a surface.

In one configuration, codes of the disclosure are amplified on bead surfaces to produce bead-attached oligos.

In one configuration, codes of the disclosure are detected using a patterned array, such as a microarray comprising oligos which are complimentary to the codes.

### 5.5 Assay automation

The encoded assays of the disclosure may be automated by a droplet actuator system. Various steps of the assays may be automated or the entire assays may be automated. Thus, in some cases, a droplet comprising a sample obtained from a subject may be loaded on a droplet actuator system of the invention, and the droplet actuator may execute all sample preparation and assay steps required to obtain an output signal that may be interpreted to provide a useful measurement, such as a diagnostically relevant measurement or a diagnostic result

Examples of systems suitable for automating the assays of the disclosure include fluid handling systems, multiwell plate handling systems, robotic liquid handling systems, robotic dispensers, microfluidic cartridges, such as electrowetting cartridges and traditional pump-based microfluidic cartridges.

Systems of the disclosure may be electronically coupled to cloud based computing systems, such that an assay conducted using a system of the disclosure may produce a digital result that may be transmitted to the cloud where it is used in further calculations, e.g., producing a diagnostic result or an input for a diagnostic analysis.

Various components of systems of the disclosure may be provided in an instrument or cartridge. For example, networking and processing components may be provided on an instrument, while droplet operations components may be provided only cartridge electronically coupled to and controlled by the instrument.

Detection components may be provided on an instrument, while droplet operations components may be provided only cartridge electronically coupled to and controlled by the instrument.

Systems and devices of the disclosure may be used to process an input sample, perform an analysis on the sample, and output a result.

In some cases, sample preparation is performed off-cartridge or on a separate sample preparation cartridge to produce a prepared sample. The prepared sample may, for example, include a concentrated target. The prepared sample may be loaded onto an analysis cartridge for subsequent analysis. In other cases, a raw sample from a subject may be loaded onto an analysis cartridge for on-cartridge sample processing and analysis.

A system of the disclosure may include an instrument and a cartridge. The cartridge may include components for conducting or facilitating droplet operations, such as channel-based microfluidics or digital microfluidics. The instrument may include components for controlling droplet operations on the cartridge, such as controllers, processors, electrical switches and connections.

The cartridge and/or the instrument may include various reaction chambers, fluid sources, sensing mechanisms, thermal control mechanisms, thin-film transistor (TFT) active matrixes, and/or droplet operations features and/or components.

The instrument may in some cases include one or more chambers for storing liquids and components for pumping liquids from the instrument onto cartridge. Liquids may include, for example, filler fluids and reagents or buffers for diluting or reconstituting dried reagents. Pumps and tubes may be provided for transporting liquids from instrument-based reservoirs to cartridge-based reservoirs. In other configurations, the instrument may include the reservoirs or liquids, and all liquids may be loaded onto the cartridge manually or robotically prior to operation of the cartridge by the system.

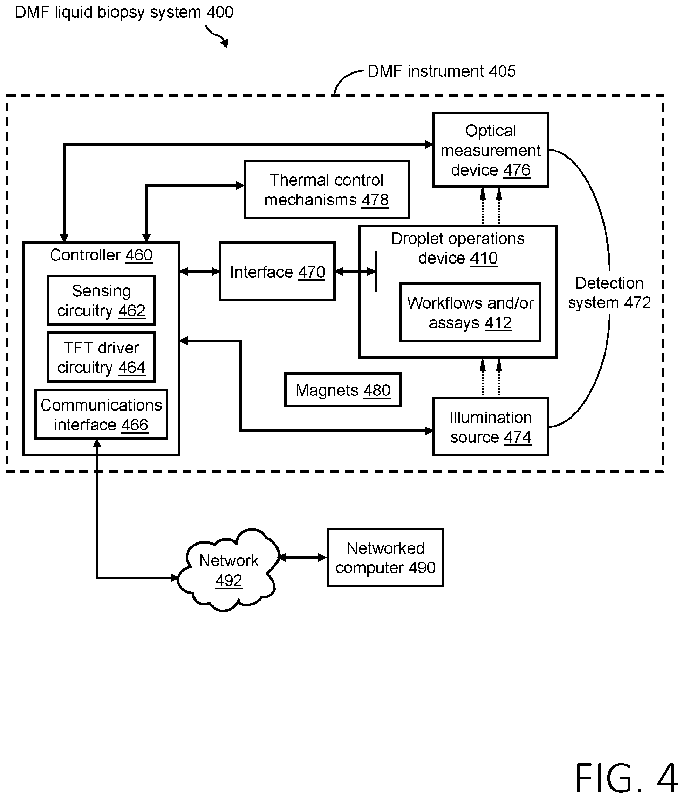

FIG. 4 is a block diagram illustrating an example of droplet actuator system 400 and including a droplet operations device 410. Examples of droplet operations devices include pumps, channels, reservoirs, robotics, and microfluidic cartridges, such as electrowetting cartridges and traditional pump-based microfluidic cartridges.

In some cases, droplet operations device 410 may be provided as a separate cartridge or as a component of a separate cartridge, such as a digital microfluidics cartridge or a channel-based microfluidics cartridge or a combination of fluidics and channel based cartridges. In some configurationsa single instrument may include the capability of controlling coupling to multiple droplet operations devices.

Droplet operations device 410 may, for example, be provided as a disposable and/or reusable cartridge. Droplet operations device 410 may be pre-loaded with liquid or dried reagents. Droplet operations device 410 may be used to perform various workflows and/or assays 412.

Droplet actuator system 400 may include components, such as a controller 460, an interface 470, a detection system 472, and thermal control mechanisms 478. Controller 460 may be any suitable controller or processor electronically coupled to and controlling other components of the disclosure. Controller 460 may include multiple controllers or processors. Although FIG. 4 illustrates the controller is part of the instrument, it should be noted that any one or more controllers or processors may be electronically coupled to and controlling various components of the system remotely, e.g., via a network.

Controller 460 may be electronically coupled to the various hardware components of microfluidics system 400, such as to droplet operations device 410, detection system 472, thermal control mechanisms 478, and magnets 480. For example, controller 460 may be electronically coupled to droplet operations device 410 via interface 470. Interface 470 may be, for example, a pluggable interface for mechanically and electrically coupling Instrument to droplet operations device 410.

Detection system 472 may be include any sensing mechanism suitable for sensing a property of droplet operations device 410 or of a substance on or in droplet operations device 410. For example, detection system 472 may be used to sense an analyte in a droplet on droplet operations device 410. Data collected by detection system 472 may be used to determine the presence or absence or quantity of a property of the droplet operations device 410, or a substance on the droplet operations device 410. For example, data collected by system 472 may be used to determine the presence or absence or quantity of an analyte in a droplet on droplet operations device 410. Controller 460 may be electronically coupled to and controlling detection system 472.

Detection system 472 may be, for example, an optical measurement system that includes an illumination source 474 and an optical measurement device 476. For example, detection system 472 may be a fluorimeter that provides both excitation and detection. In this example, illumination source 474 and optical measurement device 476 may be arranged with respect to droplet operations device 410 to permit detection of an analyte (or code) in a particular region of droplet operations device 410. Controller 460 may be electronically coupled to and controlling illumination source 474 and an optical measurement device 476.

Components of detection system 472 may be provided as part of cartridge or as part of the instrument or maybe distributed between cartridge and instrument. Detection system 472 may include various components not shown, such as lenses and other optical elements, and optical fibers.

Illumination source 474 may be, for example, a light source for the visible range (400-800 nm), such as, but not limited to, a white light-emitting diode (LED), a halogen bulb, an arc lamp, an incandescent lamp, lasers, and the like. Illumination source 474 may be any color light that is useful for detecting a particular analyte or analytes (e.g., codes or copies of the code) in microfluidics system 400. Optical measurement device 476 may be used to sense light. Optical measurement device 476 may be, for example, a charge coupled device, a photodetector, a spectrometer, a photodiode array, or any combinations thereof. Droplet actuator system 400 may include multiple detection systems 472 (e.g., multiple illumination sources 474 and/or multiple optical measurement devices 476) to support multiple detection spots.

Instrument 405 may include certain feedback mechanisms, such as impedance and/or capacitance sensing or imaging circuits, that may be used to determine or confirm the outcome of a droplet operation. Instrument 405 may include certain sensing circuitry 462 for managing any feedback mechanism. Controller 460 may be electronically coupled to and controlling sensing circuitry 462. In one example, a signal may be generated or detected by a capacitive sensor that can detect droplet position, velocity, and size. In another example, droplet operations device 410 may include a camera or other optical device to provide an optical measurement of the droplet position, velocity, and size. These droplet sensing mechanisms may be used to trigger controller 460 to re-route the droplets at appropriate positions. This feedback may be used to create a closed-loop control system to optimize droplet actuation rate and verify droplet operations are completed successfully. Controller 460 may include thin-film transistor (TFT) driver circuitry 464 for controlling, for example, a TFT-based active matrix that may be provided in droplet operations device 410.

Certain chemical and biological processes require precise and stable temperature control for optimal efficiency and performance. Thermal control mechanisms 478 may be any mechanisms for controlling the operating temperature of droplet operations device 410. For example, thermal control mechanisms 478 may be resistive heaters and/or thermoelectric (e.g., Peltier) devices arranged in thermal contact with droplet operations device 410 or provided as part of droplet operations device 410. Controller 460 may be electronically coupled to and controlling thermal control mechanisms 478.

Magnets 480 may be, for example, permanent magnets and/or electromagnets. In the case of electromagnets, controller 460 may be used to control the electromagnets 480. Magnets 480 may be provided on moveable platforms arranged to move the magnets 480 into proximity with or away from droplet operations device 410. Any actuation means may be used to move magnets 480. In another embodiment, magnets 480 may be fixed in a specific location and droplet operations device 410 may be moveable relative to magnets 480. In another embodiment, magnets 480 may be fixed in a specific location and droplet operations device 410 may also be fixed in operation relative to magnets 480. In this case, droplet operations may be used to transport magnetically responsive materials into and out of the magnetic field of the magnets to manipulate such materials in assays of the invention.

Instrument 405 may be electronically to a network. For example, a communications interface 466 of controller 460 may be in electronic communication with a networked computer 490 via a network 492. Networked computer 490 may be, for example, any centralized server or cloud-based server. Network 492 may be, for example, a local area network (LAN) or wide area network (WAN) for connecting to the internet.

Communications interface 466 may be any wired and/or wireless communication interface for connecting to a network (e.g., network 492) and by which information may be exchanged with other devices connected to the network. Examples of wired communication interfaces may include, but are not limited to, USB ports, RS232 connectors, RJ45 connectors, Ethernet, and any combinations thereof. Examples of wireless communication interfaces may include, but are not limited to, an Intranet connection, Internet, cellular networks, ISM, Bluetooth^{®} technology, Bluetooth^{®} Low Energy (BLE) technology, Wi-Fi, Wi-Max, IEEE 402.11 technology, ZigBee technology, Z-Wave technology, 6LoWPAN technology (i.e., IPv6 over Low Power Wireless Area Network (6LoWPAN)), ANT or ANT+ (Advanced Network Tools) technology, radio frequency (RF), Infrared Data Association (IrDA) compatible protocols, Local Area Networks (LAN), Wide Area Networks (WAN), Shared Wireless Access Protocol (SWAP), any other types of wireless networking protocols, and any combinations thereof.

Controller 460 may, for example, be a general-purpose computer, special purpose computer, personal computer, microprocessor, or other programmable data processing apparatus. Controller 460 may provide processing capabilities, such as storing, interpreting, and/or executing software instructions, and controlling the overall operations of microfluidics system 400. The software instructions may comprise machine readable code stored in non-transitory memory that is accessible by the controller 460 for the execution of the instructions. Controller 460 may be configured and programmed to control data and/or power aspects of system 400. Data storage (not shown) may be built into or provided separate from controller 460, e.g., as part of a network, such as a cloud-based network.

Controller 460 may be used to manage any functions of microfluidics system 400. For example, controller 460 may be used to manage the operations of sensing circuitry 462, TFT driver circuitry 464, communications interface 466, detection system 472 (e.g., illumination source 474 and optical measurement device 476), thermal control mechanisms 478, magnets 480, and any other instrumentation (not shown) in relation to droplet operations device 410.

With respect to droplet operations device 410, controller 460 may in some configurationscontrol droplet manipulation by activating/deactivating electrodes.

Controller 460 may be used to authenticate droplet operations device 410, to verify that droplet operations device 410 is not expired, to confirm the cleanliness of droplet operations device 410 by running a certain protocol for that purpose, and so on.

In other configurationsof microfluidics system 400, the functions of controller 460, sensing circuitry 462, TFT driver circuitry 464, communications interface 466, detection system 472 (e.g., illumination source 474 and optical measurement device 476), thermal control mechanisms 478, magnets 480, and/or any other instrumentation may be electronically integrated directly into droplet operations device 410 rather than provided separately from droplet operations device 410.

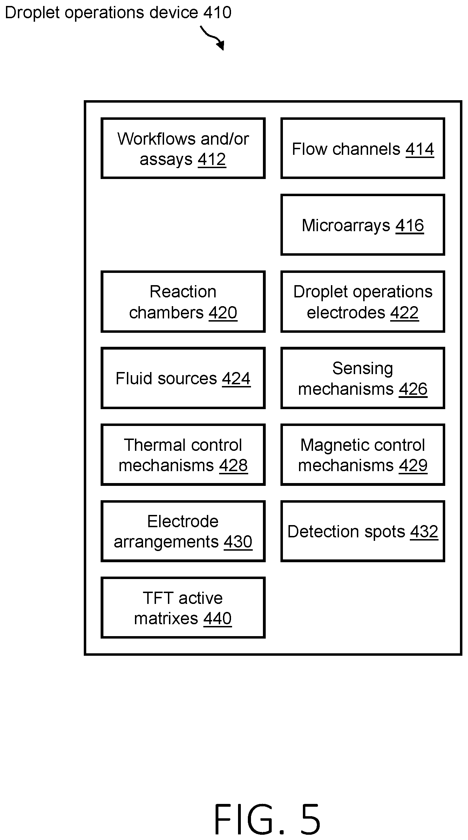

In various configurations, the microfluidics system 400 provides droplet operations device 410 that may support automated processes to manipulate, process and/or analyze biological materials. For example, droplet operations device 410 may support an automated process with respect to performing encoded described herein. More details of an example of droplet operations device 410 are shown and described herein with reference to FIG. 5.

### 5.5.1 Droplet operations device

FIG. 5 is a block diagram of an example of droplet operations device 410 of the microfluidics system 400 including various DMF electrode configurations. Generally, DMF devices consist of two substrates separated by a gap that forms a chamber in which the droplet operations are performed. In one example, a DMF device may include a printed circuit board (PCB) substrate and a glass or plastic substrate separated by a gap. Accordingly, droplet operations device 410 may include one or more reaction (or assay) chambers 420. The one or more reaction chambers 420 may be supplied by any arrangements (e.g., lines, paths, arrays) of droplet operations electrodes 422 (i.e., electrowetting electrodes). In one example, droplet operations device 410 may include a 1,000 x 1,000 array (approximately 2 inches square) of droplet operations electrodes 422. In one example, each of the droplet operations electrodes 422 of the array may be about 40 µm square.

A droplet operations gap of droplet operations device 410 (e.g., the one or more reaction chambers 120) may be filled with a filler fluid. The filler fluid may be a nonconductive immiscible fluid, such as a gas (e.g., air) or a liquid (e.g., an oil). Example oils may include silicone oil, hexane, and perfluorinated liquids.

Reaction chambers 420 and arrangements of droplet operations electrodes 422 of droplet operations device 410 may be supplied by any arrangements of fluid sources 424. Fluid sources 424 may be any fluid sources integrated with or otherwise fluidly coupled to droplet operations device 410. Fluid sources 424 may include any number and/or arrangements of, for example, on-cartridge reservoirs, off-cartridge reservoirs, blister packs, fluid ports, and the like, and any combinations thereof. Fluid sources 424 may include any liquids, such as reagents, buffers, and the like, needed to support the workflows and/or assays 412 and/or any other processes of droplet operations device 410.

Droplet operations device 410 may include sensing mechanisms 426. Sensing mechanisms 426 may be any components and/or elements built into droplet operations device 410, such as impedance or capacitance sensing. For example, sensors may be embedded at each droplet operations electrode 422 location to measure impedance, which enables monitoring and closed-loop control of certain droplet operations. Examples of other types of sensors may include temperature sensors, optical sensors, electrochemical sensors, voltage sensors, and current sensors. Sensing mechanisms 426 may be driven and/or controlled by sensing circuitry 462 of controller 460.

Droplet operations device 410 may include thermal control mechanisms 428. Thermal control mechanisms 428 may be any components and/or elements built into droplet operations device 410 to support any type of thermal control mechanisms 478. For example, closed loop control may be provided by thermal sensors embedded within the heater/cooler and a calibration step may be used to correlate the temperature within the heater/cooler to the temperature within the droplet operations gap of droplet operations device 410. In another example, resistive heaters may be integrated within droplet operations device 410. Examples include resistive wires or meandering traces at particular locations on the droplet operations devices and/or discrete packaged components, such as surface mount resistors attached directly to droplet operations device 410. In another example, Joule heating or radiation may be used to heat the liquid droplets. Thermal control mechanisms 428 may be driven and/or controlled by controller 460.

Droplet operations device 410 may include TFT active matrices 430. For example, a TFT active matrix 430 may be provided in relation to an arrangement of droplet operations electrodes 422. Any TFT active matrix 430 of droplet operations device 410 may be driven and/or controlled by TFT driver circuitry 464 of controller 460. Active matrix DMF devices based on TFT can enable particularly flexible and high-throughput DMF devices to be realized. In TFT, individual transistors (i.e., CMOS) are fabricated underneath each electrode (i.e., pixel) enabling electronics, such as switches and sensors, to be embedded at each electrode location. The embedded switches enable row-column based addressing which significantly reduces the number of connections to the device and allows arbitrarily large arrays of electrodes to be independently operated with a fixed number of electrical inputs to the device. The embedded TFT circuitry also enables sensors (e.g., sensing mechanisms 426) to be embedded at each electrode location. Again, for example, for measuring impedance which enables monitoring and closed-loop control of certain droplet operations. An example of TFT active matrix technology that may be suitable for forming a TFT active matrix 430 in droplet operations device 410 may be the TFT active matrix technology described in U.S. Patent No. 7,163,612, entitled "Method, apparatus and article for microfluidic control via electrowetting, for chemical, biochemical and biological assays and the like," issued on January 16, 2007.

Droplet operations device 410 may include any arrangements of flow channels 414 integrated into any arrangements of reaction chambers 420 and/or droplet operations electrodes 422..

Droplet operations device 410 may include one or more microarrays 416 integrated into any arrangements of flow channels 414, reaction chambers 420, and/or droplet operations electrodes 422. A microarray 416 can be any type of microarray for performing assays. Examples of microarrays include, but are not limited to, DNA microarrays (e.g., cDNA microarrays, oligonucleotide microarrays, BAC microarrays and SNP microarrays), MMChips, protein microarrays, peptide microarrays, tissue microarrays, cellular microarrays, small molecule microarrays, chemical compound microarrays, antibody microarrays, carbohydrate arrays, phenotype microarrays, reverse phase protein microarrays, and the like.

A microarray 416 may include an arrangement (e.g., an array) of capture sites on a substrate. In one example, the capture sites may be analyte capture elements, wherein each capture site or groups of capture sites may be functionalized to capture different analytes. The substrate may be, for example, a glass or silicon substrate. In the case of a glass substrate, detection system 472 may be a florescence-based optical detection mechanism. In the case of a silicon substrate in which microarray 416 is provided on a semiconductor surface, detection system 472 may be an electrical signal-based detection mechanism.

Additionally, droplet operations device 410 may be based wholly or partially on other DMF formats that are not based on traditional electrode arrays. For example, (1) Optical: In optoelectrowetting (OEW), a highly resistive a-Si:H layer switches the voltage on a virtual electrode defined by the pattern of illumination; (2) Magnetic: Ferrofluidic droplets or magnetic-bead containing droplets are manipulated by translating a permanent magnet or by using an array of electromagnets to create a magnetic field gradient. In a related implementation, droplets are manipulated indirectly by using a magnetic field to deform a film which creates topographical variation causing droplets to be operated on by gravitational forces; (3) Thermocapillary: Surface-tension driven flow based on a gradient of temperature. Example implementation is a PCB with an array of surface-mount resistors attached to the backside; and (4) Surface-acoustic wave.

Droplet operations device 410 may use a variety of insulators, such as polyimide, parylene, SU-8, Si3N4, SiO, SiOC, PDMS, Ta2O5, Al2O3, BST, ETFE. Droplet operations device 110 may use a variety of insulators, hydrophobic coatings to establish hydrophobic conditions suitable for electrowetting, such as Cytop, Teflon AF, Fluoropel, Aquapel, SiOC. Substrates for droplet operations device 110 may, for example, include printed circuit board/FR4, glass, silicon, plastic, and paper. Coatings may be used for forming electrodes, such as ITO, PEDOT, and CNT.

Droplet operations device 410 may be formed using a variety of approaches. For example, certain simple configurations may be made using a single conductive layer in which all electrodes, wires and pads are formed. These configurations may, for example, be manufactured using lithography, screen-printing, inkjet printing, etc. Other configurations may be made using PCB manufacturing techniques, which provides multiple layers of electrical interconnects (e.g., 2-layer, 4-layer, 6-layer, 8-layer, etc.), enabling more complex designs and smaller features. Board-level integration with electronic components. Silicon manufacturing techniques may be used to make TFT aspects of droplet operations device 410.

### 5.5.2 Array detector

The encoded assays of the disclosure may make use of a CMOS detector. The CMOS detector may, for example, be fabricated using a glass or silicon substrate. In some cases, microscale droplets may be transported using droplet operations onto the CMOS detector for analysis.

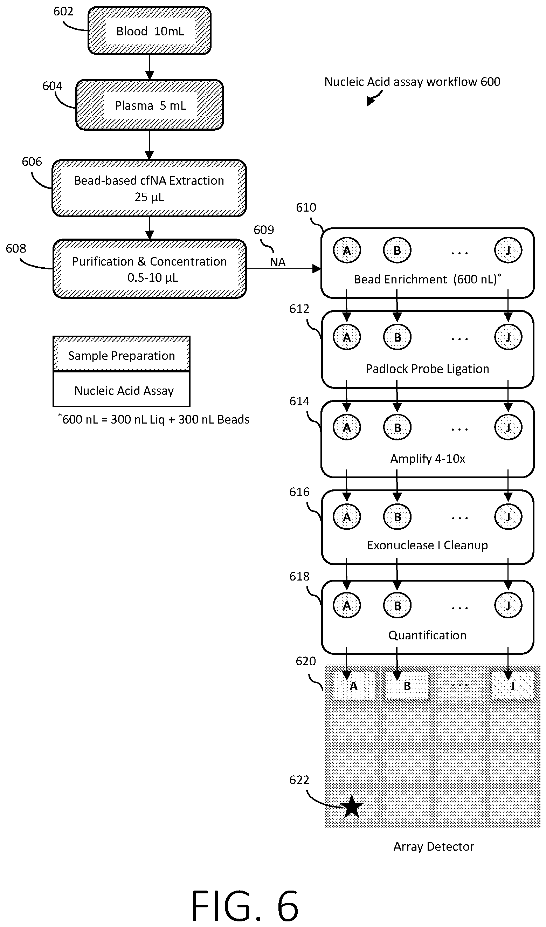

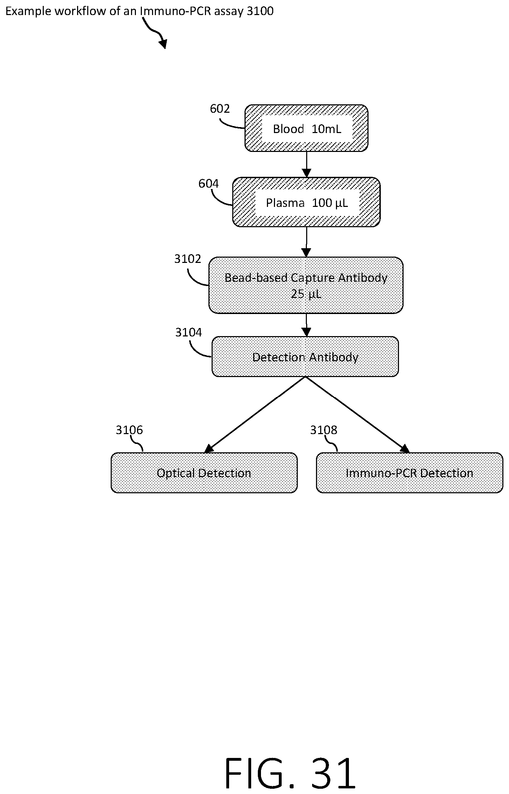

An example of an array detector is shown in FIG. 6. In this example, the array detector 620 is a 4 x 4 16-zone CMOS array detector. In one configuration, the detector may be composed of a square or rectangular array containing 2, 3, 4, 5, 6, 7, 8, 9, 10 or more detector zones per side. In one example, each of a plurality of detection droplets A-J may be transported using droplet operations to a specific section of the array detector designed to detect the contents of that specific droplet. There may be several zones on the detector used to detect different droplets and used for different assays. Most of the array detector 620 may be configured for PCR detection. In one embodiment, one array detector zone 622, may be configured for detection of proteins in an immunoassay (see FIG. 31) which do not use PCR detection. This PCR-free zone may be located in any of the array sectors.

Detection droplet A, shown as a vertical line shaded circle may be transported to a corresponding array detector zone A, also shown with vertical line shading. In detection droplet A only a specific number of target mutant alleles may be captured. Array detector zone A may be designed to specifically detect only those target mutant alleles captured by detection droplet A. In one configuration, droplet operations may be used to move each detection droplet to its respective array detector zone on the CMOS chip. As an example of an assay detecting 100 targets with detector zones of the array detector used for the example assay, detection droplet A may contain only targets 1-10 if they are present in the original sample and array detector zone A may be used to detect only those targets 1-10. Detection droplet B may contain only targets 11-20 if they are present in the original sample and array detector zone B may be used to detect only those targets 11-20. Correspondingly, detection droplet J may contain only targets 91-100 if they are present in the original sample and array detector J may be used to detect only those targets 91-100. Since only a limited amount of nucleic acid may be extracted from the sample and therefore a limited amount of any particular target mutant allele may be present, it is undesirable to spread one drop over the entire detector. Therefore, only specific target molecules may be sent to a specific portion of the detector. This allows for a higher concentration of molecules of specific targets to be detected and allows for an increased probability of detecting the target nucleic acid sequence.

After being transferred to the CMOS chip, droplet operations may be used to transport each of the detection droplets A-J to a specific zone of the array detector 620.

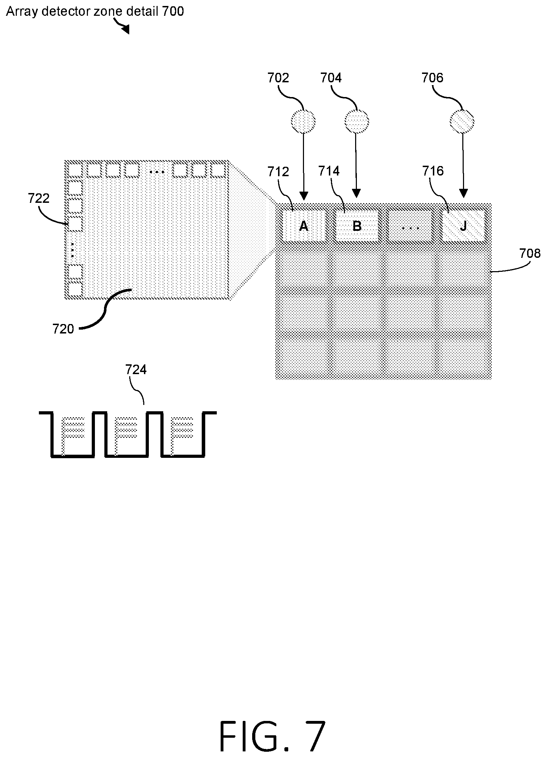

FIG. 7 is a schematic diagram of an example of an array detector shown in FIG. 6, which may be, for example, an AM-DMF CMO array detector. In this example, array detector 708 includes 10 columns of zones which are labeled A, B, ... J (712, 714, and 716 respectively) and a number of horizontal rows of detection zones. In one configuration, the array detector contains a 10 x 10 array of different detection zones. In another configuration, the array detector contains a 100 x 100 array of different detection zones.

In FIG. 7, the vertical line shaded (702), horizontal line shaded (704) and diagonal line shaded (706) circles represent detection droplets A, B through J respectively. The diagram in FIG. 7 is used to represent the movement of all detection droplets A-J. One of ordinary skill in the art will extrapolate this example to as many detection droplets, detection zones and individual detectors as needed for an assay. Once on the CMOS chip, the detection droplets A-J may be transported to a specific location on the chip. Droplet A (702) may be transported to its corresponding zone A (712), detection droplet B (704) may be transported to its corresponding zone B (714), and detection droplet J (706) may be transported to its corresponding zone J (716).

In one configuration, each zone in the array detector may be designed to indirectly or directly detect specific linear double stranded NA PCR products. In this configuration, each zone may receive the corresponding droplet which contains those specific linear dsNA PCR products for that detection zone. In one configuration, if the original sample does not contain certain targets for a particular zone, then no signal will be produced during the detection step in that zone.

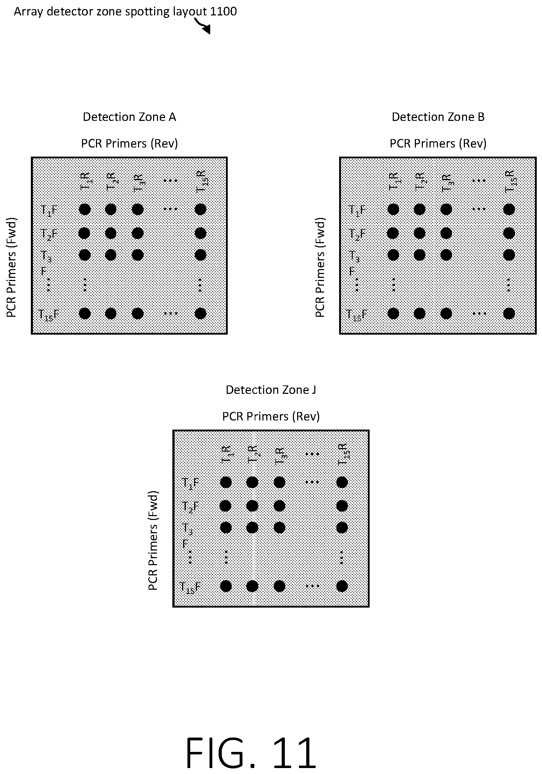

FIG. 7 shows an expanded view 720 of PCR array detector zone A 712. In this view, the white boxes 722 illustrate a few of the hundreds of possible detection features (detectors) located in array detector zone A alone. In one configuration, each array detector zone contains a grid of 10 x 10 detector reaction wells 722. In another configuration, each array detector zone contains a grid of 100 x 100 detector reaction wells 722. In another configuration, each array detector zone contains a grid of 1000 x 1000 detector reaction wells 722. In another configuration, each array detector zone contains a grid of more than 1000 x 1000 detector reaction wells 722. With a grid of 10 x 10 zones each containing a grid of 10 x 10 detection features per zone, thousand individual detectors are possible. In another example, with a grid of 100 x 100 zones each containing a grid of 100 x 100 detection features per zone, million individual detectors are possible. Individual reaction wells are shown in further detail in detection feature 724. Each detection feature may have a dried reagent spot containing an intercalating dye, for example SYBR Green (SYBR Gold), and a unique combination of forward/reverse primers to carry out PCR detection of the targets specific to that feature. Combinations of forward and reverse primers for detection of target sequences is described in more detail with reference to FIG. 11.

Since there may be a limited amount of nucleic acid extracted from the sample and a correspondingly limited amount of any particular target, it may be undesirable to spread one droplet over the entire detector. Therefore, only specific target molecules found in specific droplets may be sent to a specific portion of the detector. This allows for a higher concentration of molecules of specific targets to be detected and allows an increased probability of detecting the target nucleic acid sequence.

### 5.6 Nucleic acid assays

### 5.6.1 Nucleic acid detection workflow

Referring again to FIG. 6 is a flow diagram of an example of a nucleic acid assay workflow 600. Nucleic acid assay workflow 600 is described below for a DMF system 400; however, it will be appreciated that other systems, such as robotics systems or other microfluidics systems, may be used.

In step 602, a whole blood sample (e.g., 10 mL) may be collected from a subject. In one configuration, the subject may be a human. The subject may be a human or non-human animal. A syringe or pipette may be used to load the whole blood sample into a blood draw collection tube containing an anticoagulant such as EDTA.

In step 604, the plasma fraction of the whole blood sample is isolated. In one example, the blood draw collection tube may be centrifuged on-bench to separate the plasma fraction from the cellular components of whole blood. A syringe or pipette may be used to transfer 5 mL, for example, of the plasma fraction into a sample reservoir on the droplet operations device.

In one configuration, dried internal controls may be provided as one or more dried reagent spots inside the sample reservoir. The internal controls may include, for example, non-human or synthetic positive and/or negative control nucleic acid sequences. Droplet operations may be used to pulse the plasma sample in the sample reservoir to ensure complete rehydration and uniform mixing of the internal controls with the plasma sample. In one configuration, one droplet of the combined plasma / rehydrated internal control sample may be transported using droplet operation to an appropriate section of the droplet operations device (e.g., a detector) to quantitatively test for the complete rehydration of the internal controls.

Droplet operations may be used to split-off one or more droplets from the combined plasma / internal control sample to produce one or more plasma droplets that may be used in subsequent liquid biopsy assays. In one configuration, a plasma droplet may be used in an immunoassay, which will be discussed further hereinbelow.

In one configuration, the nucleic acids in a plasma droplet may be extracted, purified, and concentrated for use in a single mutant allele detection assay. For example, a proteinase K (ThermoFisher, Waltham, MA) digestion step may be used to digest proteins present in the plasma droplet (e.g., nucleases). In one configuration, proteinase K may be supplied as a dried reagent spot on a certain droplet operations electrode of the droplet operations device. Droplet operations may be used to transport the plasma droplet to the dried proteinase K reagent spot and pulse the plasma droplet to ensure complete rehydration and uniform mixing of the proteinase K reagent in the plasma droplet. In one configuration, a heat denaturation step (e.g., 94-98°C for 20-30 seconds) may be used to denature double-stranded nucleic acid into single-stranded nucleic acid. In the case of microRNA may not be required as denaturation is unnecessary.

In step 606, a bead-based extraction protocol is used to capture single-stranded cell-free nucleic acids (cfNA) such as cell-free DNA (cfDNA) and/or cell-free microRNA in the plasma droplet. In some configurations, the bead-based cfNA extraction protocol uses magnetically responsive nucleic acid capture beads. One of numerous sources of suitable examples of modified magnetic beads for nucleic acid capture includes those from Cytiva Lifesciences (Marlborough, MA). In one configuration, the magnetically responsive nucleic acid capture beads may be provided as a dried reagent spot on a certain droplet operations electrode. In some configurations, the dried magnetic bead reagent spot may further include a DNA polymerase. In one example, the plasma droplet (e.g., a proteinase K treated plasma droplet) may be transported to the dried magnetic bead reagent spot and pulsed to ensure complete rehydration and mixing of the magnetic bead reagent in the plasma droplet and binding of cfNAs. Any cfNA in the plasma droplet may be bound to the magnetic beads during this process, producing a nucleic acid-bead droplet. The approximate volume of the nucleic acid-bead droplet may, for example, be about 25 µl. A magnetic bead washing protocol may be used to wash the macroscale nucleic acid-beads droplet and replace any contents not bound to the magnetic beads with a buffer.

The nucleic acid-bead droplet (step 606) may be transported to a region of the cartridge to perform a nucleic acid assay (discussed immediately below) or to another region of the cartridge to perform a methylation assay discussed further below.

For example, one or more magnets may be engaged to bind the cfNA containing magnetic beads in place. Droplet operations may then be used to replace any material (e.g., proteins and other cellular components) not bound to the magnetic beads with buffer (e.g., a Tris buffer). Droplet operations may be used to add enough volume of buffer to produce, for example, a 25 µL droplet.

In step 608, purification and concentration protocols may be performed to produce a cfNA droplet. For example, while maintaining the magnetic field, droplet operations may be used to remove all but 0.5 to 10 µL of surrounding solution from the bound macroscale nucleic acid-beads droplet to produce a smaller nucleic acid-beads droplet with a droplet volume of, for example, 0.5-10 µL (500-10,000 nL). In one configuration, the remaining volume in the macroscale nucleic acid-beads droplet may be transported to a waste reservoir using droplet operations. Using onboard thermal controls, the macroscale nucleic acid-beads droplet may be heated to 80°C to release the bead-bound nucleic acid into the buffer solution, producing a 0.5-10 µL (500-10,000 nL) cfNA droplet. In one configuration, Qiagen elution buffer (Germantown, MD) may be used to release the bead-bound nucleic acid. In another configuration, a buffer pH change may be used to release nucleic acids from ChargeSwitch Magnetic Beads purchased from ThermoFisher Scientific (Waltham, MA).

In one configuration, a denaturation reaction may be performed to produce single-stranded cfNA. For example, using droplet operations, the cfNA droplet may then be transported to a dried NaOH reagent spot on a certain droplet operations electrode. Droplet operations may be used to pulse the cfNA droplet over the dried NaOH spot to ensure complete rehydration of the NaOH spot, uniform mixing and complete nucleic acid denaturation. This denaturation reaction produces a single-stranded denatured-nucleic acid droplet. In some embodiments, cfNA denaturation may be accomplished by heating the cfNA droplet to 95 °C for 2-10 minutes. In some embodiments, cfNA denaturation may be accomplished by using droplet operations to merge the cfNA droplet with a dried formamide reagent spot. Droplet operations may be used to pulse the cfNA droplet over the dried formamide spot to ensure complete rehydration, uniform mixing and complete nucleic acid denaturation.

In step 609, a droplet volume reduction protocol is performed. For example, in certain nucleic acid assays (described hereinbelow), coded padlock probes may be used to hybridize to specific nucleic acid targets of interest.

In order to increase hybridization kinetics, the sample volume may be further reduced. Accordingly, in one configuration of a nucleic acid assay workflow 600, droplet operations may be used to split the cfNA droplet (e.g., a denatured-nucleic acid droplet) into several smaller droplets. For example, the denatured nucleic acid droplet from the step 608 may have a volume in the range of 0.5 µL to 10 µL (500-10,000 nL). Dividing a droplet of this volume into, for example, 10 smaller droplets produces 10 droplets ranging in volume from 50 nL to 1,000 nL. In one configuration, the denatured-nucleic acid droplet may be split using droplet operations into 10 droplets of 300 nL each to produce ssNA droplet A through ssNA droplet J. In another configuration, the single larger droplet may be split into four separate droplets. In another configuration, the single larger droplet may be split into more than 100 individual droplets. Splitting the denatured-nucleic acid droplet into droplets of 300 nL each will be used for illustrative purposes only for the remainder of this text. Any reference below to the number of droplets and each cfNA droplet's starting volume at step 608 is for illustrative purposes only and the person will be able to apply the following configurations to different numbers of droplets with different volumes. Each ssNA droplet A-J may proceed to step 610 for a bead enrichment process.

### 5.6.2 Bead enrichment

In some configurations of the disclosure, it may be desirable to capture specific nucleic acid targets using a bead capture process.

### 5.6.2.1 Direct capture

It may be desirable to capture specific nucleic acid targets from the droplets (ssNA droplet A-J) using specific, complementary, linear, probes attached to magnetic beads using a direct bead enrichment process.

In step 610, specific cfNA targets are captured using a direct magnetic bead enrichment process. For example, each 300 nL binding buffer containing ssNA droplet A-J may be combined with, for example, one of populations of magnetic beads (B_{A}, B_{B}, ..., B_{J}). Droplet operations may be used to transport each ssNA droplet A-J to one of specific reagent spots containing specific complementary probes attached to magnetic beads B_{A}-B_{J}. In one configuration, each population of magnetic beads (B_{A}-B_{J}) may be 300 nL in volume, for example, and each ssNA droplet may be 300 nL in volume, for example. When one ssNA droplet A-J (300 nL each) is merged with one magnetic bead population (B_{A}, B_{B}, ... , B_{J}) (300 nL each) a droplet volume of 600 nL is produced for each droplet-bead population group. The droplet-bead populations may be referred to as NA-probe-beads A-B_{A}, NA-probe-beads B-B_{B}, ... NA-probe-beads J-B_{J}, where the first letter A-J indicates the droplet and the next two letters indicate which bead population has been combined with the specific droplet. In the example of NA-probe-beads A-B_{A}, ssNA droplet A has been combined with magnetic bead population B_{A}.

For example, using droplet operations, the 300 nL ssNA droplet A is transported into contact with the 300 nL magnetic beads population B_{A}, ssNA droplet B is transported into contact with magnetic beads population B_{B} and ssNA droplet J is transported into contact with magnetic beads population B_{J}. In one configuration, the magnetic beads may be immobilized to the PCB cartridge at the time of manufacturing by drying using forced air. In one configuration for using a direct target nucleic acid capture method, a biotinylated probe+ligand may be coupled to a magnetic bead containing streptavidin (ThermoFisher, Waltham, MA). In another configuration, there may be populations of magnetic beadstreptavidin---biotin---probe complexes (B_{A}-B_{J}) immobilized onto the PCB cartridge. Each of the probe complexes may be complementary to a specific target nucleic acid sequence. For example, each of the 10 pools containing the magnetic bead complexes (B_{A}-B_{J}) may contain specific probes used to capture, for example, 1/10^{th} of the nucleic acid targets in the original sample. The nucleic acid assay may be set up to detect a different number of nucleic acid targets. The nucleic acid target may, for example, be a target mutant allele. In one configuration, the assay may detect more than 10 different mutant allele targets. In another configuration, the assay may detect 100 or more different mutant allele targets. In another configuration, the assay may detect 1000 or more different mutant allele targets. In another configuration, the assay may detect 10,000 or more different mutant allele targets. In another configuration, the assay may detect 100,000 or more different mutant allele targets.

In an example of the nucleic acid assay used to detect 1000 different mutant allele targets in the original whole blood sample, the first population of magnetic beadstreptavidin---biotin---probe complexes (B_{A}) may contain probes 1-100 and may capture the matched nucleotide targets 1-100 present in droplet A, producing NA-probe-beads A-B_{A}; bead population B_{B} may contain probes 101-200 and may capture any matched nucleotide targets 101-200 present in droplet B, producing NA-probe-beads B-B_{B}; and bead population B_{J} may contain probes 901-1,000 and may capture any matched nucleotide targets 901-1,000 present in droplet J, producing NA-probe-beads J-B_{J}. In one configuration, the biotinylated probes may capture their matched nucleic acid targets in each respective droplet to which they are initially exposed.

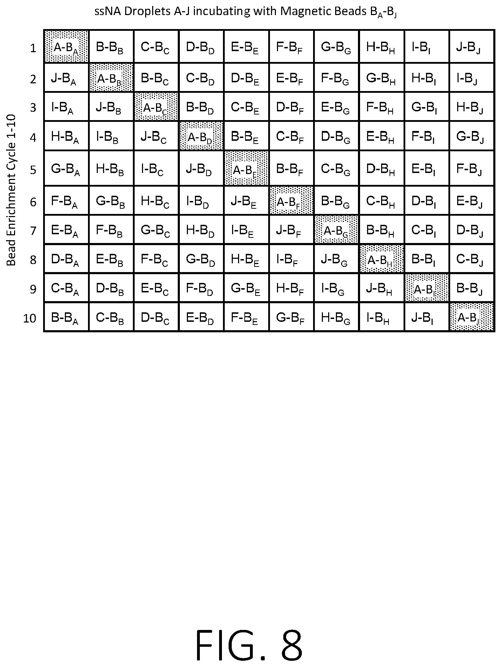

FIG. 8 is a table indicating a droplet magnetic bead incubation workflow 800 that includes 10 bead enrichment cycles. For example, during bead enrichment cycle 1, when ssNA droplet A is merged with and incubated with bead population B_{A} (capture probe beads A), a NA-probe-beads droplet A-B_{A} may form, where A represents the droplet "A" and "B_{A}" represents the first of 10 rehydrated bead populations. Any ssNA target sequence present in ssNA droplet A which can bind with the specific linear oligonucleotide probes attached to bead population B_{A} may do so. The remaining nucleic acid targets of interest do not have a complementary binding probe in this bead pool and will therefore remain in solution. Likewise, when ssNA droplet B merges and incubates with bead B_{B}, a NA-probe-beads droplet B-B_{B} may form. As a final example, when ssNA droplet C merges with bead B_{C}, an NA-probe-beads droplet C-B_{C} may form. Each bead population may have different oligonucleotide capture probes attached which may be used to capture 10% of the molecules sought. If there are, for example, 100 different target molecules in the original blood sample (step 602), each of the bead populations may have different capture probes such that each bead pool may capture different targets. Thus, bead population B_{A} may capture any nucleotide targets 1-10 present in droplet A, bead population B_{B} may capture any nucleotide targets 11-20 present in droplet B, and bead population B_{J} may capture any nucleotide targets 91-100 present in droplet J. This process may be scaled from 100 to 1,000 to 10,000 or 100,000 or more different target mutant alleles, where each of the bead populations will capture 10% of the target nucleic acid present in the original blood sample. In the case of 10,000 different mutant alleles present in the original blood sample, bead population B_{A} may capture any nucleotide targets 1-1000 present in droplet A, bead population B_{B} may capture any nucleotide targets 1001-2000 present in droplet B, and bead population B_{J} may capture any nucleotide targets 9001-10,000 present in droplet J.

Bead population B_{A} may be used to pull out targets 1-10 from droplet A. However, if present in the original sample, targets 1-10 may also be present in droplets B through J. In the subsequent cycles of the bead enrichment process, each droplet may be exposed to each subsequent bead population in turn. For example, using droplet operations, each droplet may be transported to the next bead population and may incubate for a time. The transport of droplet A, for example, relative to the fixed bead populations is shown by the dotted fill in FIG. 8. In the second bead enrichment cycle, FIG. 8 row 2, droplet A incubates with bead pool B_{B}, droplet B incubates with bead pool B_{C} and droplet J incubates with bead pool B_{A}. Therefore, bead pool B_{A} may capture any targets 1-10 from droplet J, bead pool B_{B} may capture any targets 11-20 from droplet A, and bead pool B_{C} may capture any targets 21-30 from droplet B. That is, in this example of 100 total targets, bead pool B_{A} may only capture targets 1-10 out of each droplet and bead pool B_{B} may only capture targets 11-20 out of each droplet.

Since there may be a limited amount of nucleic acid in the original blood sample, it may be desirable for the droplets in a detection reaction (i.e., exposed to the detector) to be as concentrated as possible. A benefit of magnetic bead incubation workflow 800 is that a sample may be interrogated for many different targets at once because there may be many different bead populations. Furthermore, having many different bead populations may help to concentrate the signal. A further benefit of this methodology may be that capturing the nucleic acid targets on each bead population is a diffusion limited process. By limiting the droplet volume to 300 nL, for example, and incubating droplets in parallel, the time required for diffusion is reduced and therefore may reduce the overall analysis time.

Using droplet operations, the incubation procedure may be repeated and then each droplet may be transported to the next enrichment cycle station. This process may be repeated for the remaining 8 cycles. When the bead enrichment phase is completed, ssNA droplets A-J may have incubated with each of different bead populations B_{A}-B_{J}. Each bead-probe population may have captured substantially all of the respective mutant allele nucleotide targets in each droplet.

### 5.6.2.2 Indirect capture

In some configurations of the disclosure, ssNA droplets A-J may be transported to one of dried reagent spots containing biotinylated capture probes which may be complementary to specific nucleic acid targets. Droplet operations may be used to pulse each of the droplets until complete rehydration of the dried reagent and uniform mixing occurs. After rehydration and mixing, the biotinylated capture probes may have captured all of or substantially all of the matched mutant allele targets in that droplet. This forms target-probe-biotin complexes in each of the droplets. Each of these 10 droplets with target-probe-biotin complexes potentially therein, may be transported using droplet operations to one of dried reagent spots containing streptavidin-coupled magnetic bead populations. Droplet operations may be used to pulse each of the droplets until complete rehydration of the dried reagent and uniform mixing occurs. A magnet located in or nearby the DMF instrument may be used to keep the magnetic bead population secured in place. The streptavidin magnetic beads may capture biotinylated probes which have bound a target nucleic acid sequence and any free biotinylated probes. Similarly, to the protocol discussed above in the direct capture method, each of the 10 droplets may be transported using droplet operations to each of the magnetically bound streptavidin-bead complex populations and each bead population may capture the corresponding target mutant alleles in each droplet.

In some configurations, the magnetic beads may have covalently coupled immobilized capture probes which act to capture specific nucleic acid sequences present in solution. The magnetic beads may be activated with a certain functional group and the capture probes may have a complementary functional group. Coupling the two functional groups, for example in a dehydration process, chemically binds the probes to the beads.

### 5.6.3 Nucleic acid probe bead washing

In step 610, one or more magnets may be engaged to hold each bead population (B_{A}, B_{B}, ..., B_{J}) in place. Droplet operations may be used to wash away any surrounding material not bound to the beads. For example, any molecules not bound to each of the NA-probe-beads droplets may be replaced by a buffer, for example a Tris buffer. This step completes the nucleic acid purification process by first hybridizing the target nucleic acid to the complementary probe attached to magnetic beads and then removing contaminants. The captured nucleic acid targets may then be released from the capture beads.

In some configurations, the captured and enriched nucleic acid targets may be released from the magnetic beads using alkali, such as NaOH or heat, producing target droplets A-J.

In some configurations, the captured and enriched nucleic acid targets may be released from the magnetic beads using an elution buffer. For example, droplet operations may be used to merge two buffer droplets (e.g., Tris buffer droplets) over a dehydrated Qiagen bead elution buffer reagent spot. Droplet operations may be used to pulse the buffer droplets over the Qiagen bead elution buffer reagent spot (Qiagen, Germantown, MD) to ensure complete rehydration and uniform mixing of the buffer reagent. This produces two elution buffer droplets. Keeping the magnetic field active, droplet operations may be used to split the elution buffer droplets into separate droplets. Subsequently, droplet operations may be used to merge and pulse one elution buffer droplet with each NA-probe-beads droplet, causing the captured nucleic acid sequences to elute into the droplet to produce target droplets A-J. In an example 1000 target assay, target droplet A may contain captured targets 1-100 eluted off NA-probe-beads droplet A, target droplet B may contain any captured targets 101-200 eluted off NA-probe-beads droplet B, and target droplet J may contain captured targets 901-1000 eluted off NA-probe-beads droplet J.

### 5.6.4 Coded padlock probe ligation

Referring again to FIG. 6, In step 612, droplet operations may be used to transport each of the target droplets A-J to one of dried reagent spots containing coded padlock probes complementary to the targets found in each respective target droplet. In one example, the coded padlock probes may be custom synthesized by Integrated DNA Technologies (IDT, Coralville, IA). Each coded padlock probe reagent spot may include dried custom synthesized coded padlock probes and High Fidelity Taq DNA Ligase (New England Biolabs, Ipswich, MA). Droplet operations may be used to pulse each of the target droplets A-J over its corresponding coded padlock probe reagent spot to ensure complete rehydration and uniform mixing of the reagents.

Each dried coded padlock probe reagent spot may contain from 1 to 10,000 or more coded padlock probes synthesized to capture specific target mutant alleles of interest. Each target droplet A-J may contain a portion of the specific targets. For example, in a sample containing 10,000 different mutant allele targets, target droplet A may contain targets 1-1,000; target droplet B may contain targets 1,001-2,000; ... and target droplet J may contain targets 9,001-10,000. Each target droplet A-J may interact with its corresponding pool of coded padlock probes for the hybridization and ligation steps. For example, a first pool may contain the complementing coded padlock probes to targets 1-1,000; a second pool may contain the complementing coded padlock probes to targets 1,001-2,000; ... and a tenth pool may contain the complementing coded padlock probes to targets 9,001-10,000. In some configurations, the coded padlock probes will ligate only if both ends of the probe may be complimentary to the target sequence of interest and anneal with no mismatched nucleotide pairs. In one example, high fidelity HiFi Taq DNA Ligase may be used ligate the nick in the ends of the padlock oligonucleotide probe after both ends have hybridized to the complimentary target nucleic acid sequence.

### 5.6.5 5'-arm & 3'-arm of coded padlock probes

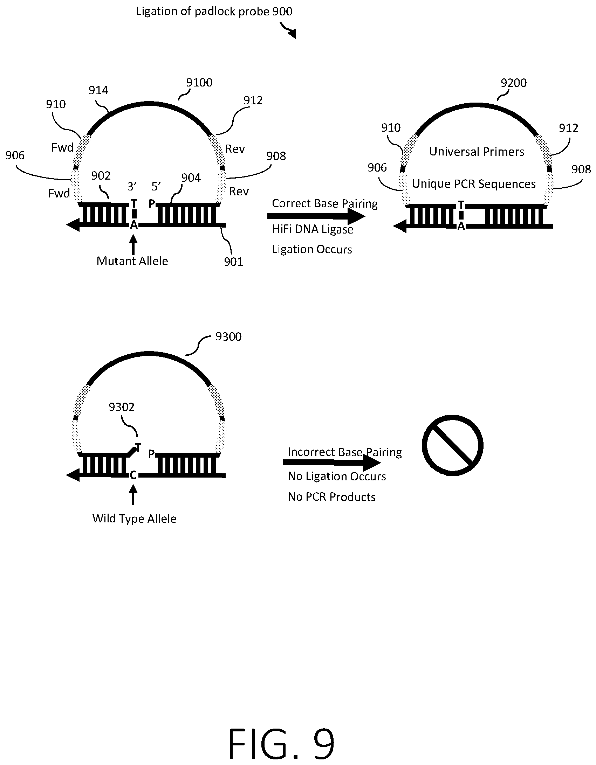

FIG. 9 is a schematic diagram showing an example of a coded padlock probe ligation reaction. Upon rehydration, mixing, and incubating (e.g., at 50-70°C for 20-40 seconds or longer) the two ends or arms of the complementary coded padlock probes, i.e., end 902 and end 904 may anneal to the target mutant allele sequence 901 of interest. In this example, the coded padlock probe nucleotide (T) which is complementary to the mutant allele may correctly hybridize to the target mutant allele (A), leaving a nick between the 3' coded padlock probe complement (T) and the exposed phosphate on the 5'-end of the coded padlock probe.

Each coded padlock probe may be synthesized to include a pair of specific oligonucleotide "arms", arm 902 and arm 904, located at the distal ends of the coded padlock probe. These oligonucleotide probes may be complementary to each side of the target mutant allele and may be designed to capture one specific target. In one configuration, the synthesized coded padlock probes have a 3' nucleotide (e.g., a T in FIG. 9) which complements the mutant nucleotide (e.g., an A in FIG. 9) and an exposed 5'-phosphate at the opposite end of the coded padlock probe. The two ends of the coded padlock probe may be synthesized to be a perfect complement to the mutant nucleic acid sequence (mutant allele sequence 901) and they may anneal perfectly to the target sequence. If the pair of probe ends perfectly hybridize to the target nucleic acid sequence, then the 3' end of the probe may be immediately adjacent to the 5' end of the probe located at the opposite end of the entire coded padlock probe. The coded padlock probe may then circularize with one nick in the sequence, as shown in Fig. 9. If the circularized coded padlock probe has the correct base pairing, then the high-fidelity ligase present in the rehydrated droplet may immediately seal the nick between the 3'-complementary nucleotide and the 5'-free phosphate, forming a closed loop 9200 hybridized to the mutant sequence. In one embodiment, HiFi Taq DNA Ligase may immediately repair the nick of any probes correctly hybridized to the target mutant allele sequence. In step 612 of FIG. 6, the coded padlock probe ligation reaction produces coded padlock probe droplets A-J.

If a coded padlock probe hybridizes to a wildtype allele sequence 9300, there may be a mismatched nucleotide pair which does not hybridize in the double-stranded region where the nucleic acid otherwise hybridized to the coded padlock probe 9302; in this example a T and C nucleotide are incorrectly paired and no ligation occurs when there is incorrect base pairing.

In one configuration, the 5'-free-phosphate arm 904 of the coded padlock probe may be longer than the 3'-arm 902 of the coded padlock probe. It is the 5'-arm 904 of the coded padlock probe which is responsible for most of the coded padlock probe's hybridization to the mutant sequence. It is known in the art that the hybridization of the 3'-complementary arm 902 is transient in nature. The hybridization of this 3'-arm is more of a temperature dependent equilibrium process, where the 3'-arm 902 repeatedly hybridizes to and then releases from the mutant sequence. If the 5'-arm 904 is longer than the 3'-arm 902 then the coded padlock probe will bind tightly to the mutant sequence, leaving a shorter, transient 3'-arm 902. However, it is the 3'-end of the probe which acts as a surrogate detector by binding to the mutant allele. Because the 3'-arm of the coded padlock probe may hybridize and release at a temperature dependent rate, the complementing fit must be perfect in order for the high-fidelity ligase to seal the nick between the two ends of the coded padlock probe before the 3'-arm has a chance to release again. Thus, the equilibrium nature of the 3'-arm 902 of the probe may increase the specificity of the coded padlock probe because it challenges the system to become more selective. In one embodiment, an array of 3'-arm and 5'-arm lengths may be created in order to find the length combination with the highest specificity. In some embodiments, selecting the optimal 3'- and 5'-arm lengths for a probe may provide improved specificity in capturing a mutant target using coded padlock probes.

### 5.6.6 Array of 3'-probe & 5'-probe arm lengths

In one configuration, an array of lengths for each of the 3'-probes and 5'-probes may be created to optimize the hybridization for various targets. In one configuration, the 3'-probe arm has a larger number of nucleotides than the 5'-probe arm. In one configuration, the 5'-probe arm has the same number of nucleotides as the 3'-probe arm. In a preferred configuration, the 5'-probe arm has a larger number of nucleotides than the 3'-probe arm. In one configuration, the 3'-probe arm is 2 or more, 10 or more, 20 or more, 30 or more, or 40 or more nucleotides in length, where one nucleotide at the 3' end of the probe is the exact complement to the mutant allele target. In another configuration, the 5'-probe arm is 3 or more, 11 or more, 21 or more, 31 or more or 41 or more nucleotides in length. In another configuration, the 5'-end of the probe (arm 904) is a phosphate group available to be covalently bound to the 3'-probe arm nucleotide.

### 5.6.7 Relative hybridization & temperature dependence of the 3'-probe equilibrium

In one configuration, an array of all possible combinations of nucleotide bases may be tested to determine the relative hybridization of a correctly matching mutant/probe allele vs. the relative hybridization of an incorrectly matched mutant/probe allele.

In another configuration, the optimum hybridization temperature may be determined for each target/coded padlock probe combination. For example, as the temperature increases, the amount of time the 3'-arm of the probe will spend hybridized to the mutant allele may be reduced (i.e., the transient nature of this 3'-probe end may increase, spending less time hybridized to the target) and therefore the specificity of the coded padlock probe and the high-fidelity ligase will increase. However, in one configuration, the increase in temperature may not exceed the melting temperature of the nucleic acid target or the coded padlock probe. In this configuration, the temperature which produces the highest fidelity ligation without exceeding the target nucleic acid melting temperature may be determined. This temperature is referred to as the fidelity temperature. In another configuration, coded padlock probes and mutant targets with similar fidelity temperatures may be grouped together in the same sample pool. This may allow each sample pool to be heated to the temperature which provides the highest ligation fidelity without exceeding that pool's melting temperature.

### 5.6.8 Hybridization kinetics of the coded padlock probes - 3' arm

Coded padlock probes may reduce hybridization kinetics relative to other hybridization detection methods. When one end of the coded padlock probe hybridizes to the target mutant allele, the other end of the coded padlock probe may be in close proximity. This means the total hybridization of both ends of the coded padlock probe may proceed much faster than using two separate probes. In this configuration, two molecules may bond instead of the increased time required for three separate molecules to bond (i.e., the target nucleotide sequence and two separate probes hybridizing to either end of the target mutant nucleotide). Reducing the sample volume as discussed above may also be used to greatly reduce the hybridization kinetics. For example, in step 610 of FIG. 6 (i.e., the bead enrichment step), the entire macroscale sample volume, for example 0.5-10 µL, may be fractionated into equally sized smaller volumes of 300 nL, for example (or 10 µL per droplet reduced to 30 nL per droplet).

By reducing the sample volume and maximizing the sample concentration as much as physically and biologically possible, the hybridization kinetics of hybridizing the coded padlock probes to the target sequence may be significantly reduced. With nanoliter sized droplets, at least one end of the coded padlock probe may hybridize to the target of interest rapidly. This may require second-order reaction kinetics. Hybridizing the other end of the coded padlock probe to the target nucleic acid sequence may become a second-order kinetic process. Using the coded padlock probe may harness these faster second-order kinetic processes instead of requiring much slower third-order reaction kinetics involving: a) an independent oligonucleotide probe #1, b) an independent oligonucleotide probe #2 and c) the target mutant allele of interest. Second, if the entire macroscale sample volume was sent to the detector, a much larger volume would flood the entire sensor. This may be less desirable due to the extremely small amount of nucleic acid in the sample volume. The reduced concentration due to a much larger sample volume decreases the probability of detecting the target mutant allele. If instead the sample is divided into groups of 10, for example, the fractionated sample may interact with only 10% of the sensor. The detection sensor will be described in more detail below. Each 10% section of the sensor may have 10% of the total target population in a much smaller volume. This means the assay essentially focuses all the detection events which may increase the signal to noise and thereby may increase the probability of detecting the target of interest.

### 5.6.9 Coded padlock probe design

Each coded padlock probe may be synthesized to include a pair of specific oligonucleotide "arms", arm 902 and arm 904, located at the distal ends of the coded padlock probe which may be complementary to and may hybridize with a target sequence containing a single nucleic acid base mutation. Arms 902 and 904 may be referred to as primers.

In one configuration, the 3'-arm 902 of the coded padlock probe may include the nucleotide complementary to the mutated nucleotide base. If the pair of oligonucleotide arms perfectly attach to the target nucleic acid sequence, then the 3' end of the coded padlock probe may be immediately adjacent to the 5' end of coded padlock probe. The probe may then be ligated by a high-fidelity ligase to cause the probe to circularize. Once circularized the probe may be used for rolling circle amplification.

Medially to the complementary probe arms 902 and 904, the coded padlock probe may include a specific (unique) pair of forward and reverse PCR primer sequences 906 and 908, respectively (shown in light gray), which may be used to create a uniquely identifying PCR tag for identification of specific target sequences. For example, combinations of specific (unique) forward and reverse PCR oligonucleotide primers complimentary to the forward and reverse PCR primer sequences 906 and 908 may be used to create unique combinatorial forward and reverse PCR detection reactions that may be used to generate uniquely identifying PCR tags which can be used for detection and identification of specific target sequences.

In some configurations, medially to the unique PCR tag forward and reverse primer sequences, the coded padlock probe may further include universal forward and reverse amplification primer sites 910 and 912, respectively (shown in medium gray). These primer sites may be used during an amplification step (e.g., step 614 of FIG. 6 described below) after the coded padlock probes have hybridized to the mutant or target allele and the HiFi ligase has ligated and circularized the coded padlock probe (e.g., circularized probe 9200). The universal forward and reverse primer sequences 910 and 912 shown in medium gray may be used to amplify the target nucleic acid to a level such that sufficient nucleic acid may be present in each droplet to be detected in the array detector 620 (described below in step 618). The forward and reverse universal amplification sequences 910 and 912, respectively may be universal in that the exact same primer sequences may be present in every coded padlock probe, permitting all targets to be amplified in parallel.

The coded padlock probes may include sequences which may not be extended, e.g., non-extendable sequence 914. These sequences may, for example, be located between generic sequences 910 and 912. In some configurations, incorporating a non-extendable sequence 914 into the coded padlock probes may prevent generic rolling circle amplification (RCA) of the padlock.

Coded padlock probe detection assays may be designed to use RCA. In some cases, this may be a disadvantage to single base mutation detection because RCA may occur with only one PCR primer. If PCR amplification is allowed to occur using RCA and only one PCR primer, uniquely detecting single base mutations may be more difficult. Furthermore, RCA typically produces significantly less signal compared to other PCR methods. When detecting single base mutations, it is important to have sufficient signal.

Therefore, in some configurations of the disclosure, RCA may be intentionally prevented. Using the combinatorial forward and reverse primers with the spatial separation of different detection zones (as described hereinbelow) allows for the detection of tens to hundreds of thousands of different mutant targets.

In one configuration, a blocking molecule is inserted into non-extendable sequence 914 which prevents RCA.

In another configuration, rolling circle amplification (RCA) may be used in the universal amplification step.

In one configuration, non-extendable sequence 914 of the coded padlock probe contains a phosphate-deoxyribose backbone without a nitrogenous base. During universal amplification, a polymerase enzyme may stop when it encounters an abasic site.

In another configuration, the non-extendable sequence 914 is a polyethylene glycol (PEG) spacer. In one configuration, the PEG spacer includes 2 or more, 4 or more, 6 or more, 8 or more or 10 or more PEG monomer units. In a preferred configuration, the PEG spacer contains 6 monomer units. During an amplification step (e.g., step 614 of FIG. 6) the polymerase may encounter the PEG chain and terminate extension.

In another configuration, the non-extendable sequence 914 may be, for example, a restriction endonuclease Notl sequence (GCGGCCGC) or other rare restriction endonuclease sequences, the frequency of which are extremely rare in the human genome. For example, after the coded padlock probe successfully hybridizes to the mutant target of interest and the HiFi Ligase successfully circularizes the coded padlock probe, universal amplification may occur. During amplification, the complement to the Notl sequence (CGCCGGCG) may hybridize to the coded padlock probe. A PEG spacer or restriction site (e.g., Notl site) may be inserted at the time of manufacture.

Referring again to step 612 of FIG. 6, a coded padlock probe containing a PEG blocker or a Notl restriction enzyme cleavage site may be circularized. In one example of the Notl method, each of the 10 coded padlock probe droplets A-J may be transported using droplet operations to a dried reagent spot containing an amount of Notl restriction enzyme. Droplet operations may be used to pulse each of the coded padlock probe droplets A-J over the dried reagent spot to ensure complete rehydration and uniform mixing of the enzyme in each droplet. After the enzyme has rehydrated, it may cleave the Notl sequence (e.g., non-extendable sequence 914) at the 5'-CG of the Notl sequence pair which may open the ring creating a linear double-stranded product. In the example incorporating a PEG sequence blocker, the polymerase may extend over the primers and may not extend through the PEG sequence, creating a linear double-stranded product. Both the PEG and Notl non-extendable sequences 914 may be used to prevent rolling circle amplification.

In some configurations of the disclosure, two independent target-specific primers may be used instead of a coded padlock probe. Each of the independent primers may be designed to perfectly match a mutant allele target. In this configuration, the target may hybridize with two separate oligonucleotides, requiring intermolecular reaction kinetics.

### 5.6.10 Universal amplification

Referring again FIG. 6, in step 614, each of the coded padlock probe droplets A-J may be amplified in a universal amplification reaction. For example, the coded padlock probe droplets A-J (from step 612) may be transported to one of the reagent spots containing dried dNTPs, generic PCR primers which complement the universal, generic forward and reverse PCR primer sequences 910 and 912 (described with reference to FIG. 9) and DNA polymerase. Droplet operations may be used to pulse the coded padlock probe droplets A-J over each reagent spot to ensure complete rehydration and uniform mixing, producing pre-amplification droplets A-J. After rehydration and mixing, the pre-amplification droplets A-J may be heated in a PCR initialization step (e.g., heated to 94-96°C for 2-10 minutes) to activate the DNA polymerase and to denature nucleic acids in the mixture. To begin a PCR cycle 1, the pre-amplification droplets A-J may be heated, for example, to 94-98°C for 20-30 seconds to ensure denaturation of the target nucleic acids to single-stranded products. At this stage in step 614, the target mutant nucleic acid sequence is a linear single-stranded product, while the coded padlock probe is a single-stranded circularized sequence. At this stage, a first rehydrated primer may bind to its complementary generic (universal) primer sequence (e.g., generic primer sequence 912) of the coded padlock probe. Subsequently, after the first PCR cycle, the second primer may bind to its complementary generic (universal) primer sequence e.g., generic primer sequence 910).

The reaction temperature of each pre-amplification droplet A-J may be lowered to an annealing temperature (e.g., 50-65°C for 5-40 seconds) to allow the primers to anneal to the circularized coded padlock probes. After the primers hybridize to the forward and reverse universal amplification sequences on the coded padlock probe, the rehydrated DNA polymerase incorporates dNTPs onto the primers in an elongation step (e.g., 55-72°C for Taq DNA Polymerase for approximately 20-60s). In the first cycle, this elongation step occurs proceeding in the 5' to 3' direction from generic primer sequence 912 to generic primer sequence 910 to synthesize a new nucleic acid strand. In some configurations, the amplification reaction may be stopped when it encounters a non-extendable sequence 914 e.g., a PEG blocker or the cleaved ends of the Notl restriction site and is not amplified. In one configuration, this PCR amplification process may be repeated for 3 or more cycles to generate enough target nucleic acid to be detected. In other configurations, generic PCR amplification may proceed for 20 or more cycles or 40 or more cycles. After for example 4-10 amplification cycles, each of the pre-amplification droplets A-J may be held at 72°C for 5-15 minutes to ensure complete strand extension.

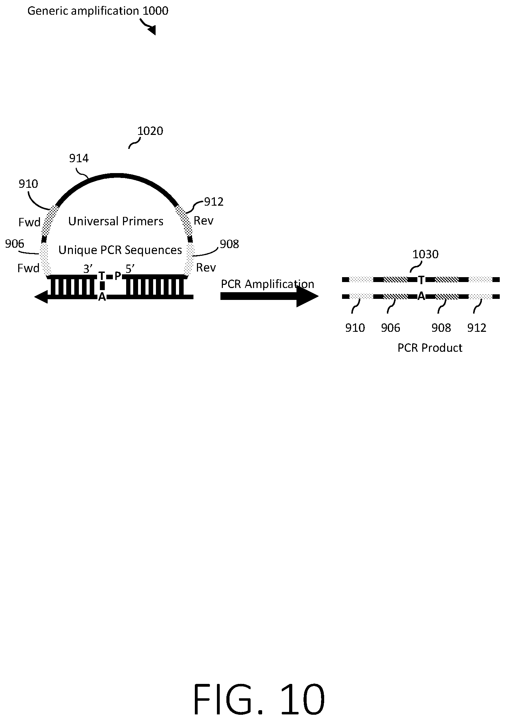

The universal amplification step 614 produces linear duplex nucleic acid copies (see FIG. 10, 1030 that include the generic (universal) primer sequences 910 & 912 and the combinatorial forward and reverse PCR detection primer sequences (906 and 908) which may be used to create a uniquely identifying PCR tag for identification of specific target sequences as shown in FIG. 10. The amplified droplets produced in step 614 are now referred to as amplified droplets A-J This step may produce excess nucleic acid in each droplet relative to that required to populate the detector array. The exact amount of nucleic acid in each droplet may be determined in a subsequent step, discussed below, and this nucleic acid may be diluted to a concentration range required by the detector (e.g., a fluorescence detector).

The amount of PCR product generated may be a function of how many coded padlock probes hybridize to the same target mutant allele. If there is a larger concentration of one specific mutant allele, but only one or two instances of other mutant alleles, the amplification of the mutant allele with the large concentration may overwhelm the detection system. In this instance, it may be desirable to physically reduce the number of coded padlock probes available to the droplet containing the highly concentrated target. Reducing the number of coded padlock probes available to the highly concentrated target may physically reduce the amplification and prevent detector saturation.

Reducing the number of coded padlock probes available to the droplet is an example method of tuning the signal at the detector if it is known in advance that a mutant allele is over-represented in the sample. The signal may be generated in genome equivalents, such that the signal may have relatively equal representation across all targets in same sample. A quantification step, described below, may be required.

After amplification of the coded padlock probes (step 614), linear duplex PCR products may be formed (as described with reference to FIG. 10) that include generic (universal) primer sequences 910 and 912 and the one or more pairs of unique PCR tag sequence primers 906 and 908. The same forward and reverse universal primer sequences 910 and 912 may be used on every coded padlock probe in the assay to prevent bias, thereby ensuring all targets are amplified uniformly and to the same degree. PCR products may only be produced upon successful ligation by the HiFi DNA Ligase. As shown in FIG. 9, 9300, when the coded padlock probe anneals to a wildtype allele, the ligase may not be able to seal the buckled nick due to the incorrect base pairing and therefore no PCR products may be produced. This process may be dependent on the fidelity of the ligase enzyme connecting only the correctly paired probe and target molecules. A high-fidelity ligase, commercially available may be selected for this reaction. In the event the ligase incorrectly seals the nick of in a wildtype allele hybridization event, a cleanup step using the RNAse H2 protocol may be used to prevent incorrect amplification in step 614. The RNAse H2 protocol is described in more detail below.

### 5.6.11 Exonuclease cleanup

Coded padlock probes which have hybridized with a target mutant allele sequence may be circularized and after amplification there may be linear, double-stranded nucleic acid sequences which may be resistant to exonuclease I digestion. Any unreacted nucleic acid material remaining in the droplets may be a linear single stranded nucleic acid product which may be digested by the exonuclease I enzyme. This difference in sensitivity to the exonuclease I enzyme differentiates desired products from undesired products (or "waste") and provides an efficient cleanup step.

Referring again to FIG. 6, in step 616, an exonuclease cleanup process is performed to remove any unreacted single-stranded nucleic acid. For example, the remaining single-stranded contents of each amplified droplets A-J from step 614 may be digested by the exonuclease I enzyme which may cleave individual single stranded oligonucleotides in the 3' to 5' direction. This may include any unreacted open (not circular) coded padlock probes, any unreacted forward and reverse primers, left over mutant allele sequences and any other contents in the droplet which may be single-stranded or have an exposed 3' end. The exonuclease digestion may reduce any susceptible components to 1-2 nucleotide residues. Double-stranded PCR amplicons and circularized coded padlock probes may not be digested.

In one example, each of the amplified droplets A-J from step 614 may be transported using droplet operations to a dried exonuclease I spot for an exonuclease sample cleanup. Droplet operations may be used to pulse the amplified droplets A-J with the dried exonuclease I spot to ensure rehydration and mixing, producing digested droplets A-J. During the digestion reaction, the exonuclease I enzyme may digest unused PCR primers, unligated coded padlock probes and target nucleic acids into 1-2 nucleotide segments. Linear, single-stranded nucleic acids remaining in the droplet may also be digested into 1-2 nucleotide segments. The only products which may remain after reaction with the exonuclease I enzyme may include closed circular molecules or any double-stranded nucleic acid product. Remaining circularized molecules may not interfere with the subsequent steps in the single nucleotide mutation detection assay.

In some configurations, each of the digested droplets A-J may be heated at 80°C for approximately one minute to fully inactivate the exonuclease I enzyme, producing cleanup droplets A-J. This step, in combination with the exonuclease digest, may remove the need for a bead-based purification process because unwanted material may be reduced to small nucleotide residues. Furthermore, the heat treatment may remove the enzyme itself which may otherwise interfere with a PCR detection step, described below.

### 5.6.12 Pre-detection quantification

In some configurations, it may be useful to determine the total amount of nucleic acid in each of the cleanup droplets A-J. When determining the presence or absence of each of the target mutant nucleic acid sequences, it may be important to have the appropriate amount of nucleic acid for the detector. If there is insufficient nucleic acid, a real concern when sampling few mutant nucleic acid strands, the sensor may fail to produce a detectable signal. If there is too much nucleic acid in the sample, the sensor may saturate.

In step 618, a quantification process may be performed. For example, the amount of nucleic acid present in each of the cleanup droplets A-J may be quantified by incorporating a fluorescent dye into the dsNA strands and measuring the change in fluorescence. When surrounded by only water or aqueous buffer solutions, a fluorescent dye exposed to an excitation wavelength will release the energy as heat or vibrations. When in the presence of nucleic acid molecules, certain fluorescent dyes such as SYBR Green or SYBR Gold (Thermo Fisher, Waltham, MA) may bind in the hydrophobic regions. When exposed to an appropriate excitation wavelength, the energy absorbed by the fluorescent dye may be released as light at a higher wavelength. This method may be used as an extremely sensitive, fast and powerful way of quantifying the presence of nucleic acid molecules, resolving, for example, on the order of picograms of nucleic acid per 100 µL sample volume. The pre-detection quantification step may determine whether an appropriate number of amplification cycles have been performed to send the appropriate amount of nucleic acid to the sensor. For example, excess nucleic acid may be produced in the amplification step (step 614) and the quantification step 618 may be used to determine what dilution of the droplet may be needed to produce the desired nucleic acid concentration to send to the detector. Droplet operations may then be used to dispense an appropriate volume of buffer from a buffer reservoir and transport the required volume of buffer to appropriately dilute the cleanup droplets A-J. Droplet operations may be used to pulse the buffer and cleanup droplets A-J to ensure appropriate dilution of the sample droplet.

### 5.6.13 Background fluorescence of the buffer

A background fluorescence measurement of the DNA intercalating dye used in a pre-quantification step may be made and stored on-board for determining the amount of nucleic acid in a droplet. For example, droplet operations may be used to transport 1 nL of a rehydration buffer (e.g., a Tris buffer) over a dried spot of SYBR Green or SYBR Gold Master Mix. Droplet operations may be used to pulse the buffer droplet over the dried dye spot to ensure complete rehydration and uniform mixing. The fluorescence of this droplet may be measured using a CCD camera, for example, at 520 nm to obtain the background fluorescence signal due to only the buffer in the cleanup droplets A-J (step 616) and the intercalating dye. After measurement, the fluorescence may be stored on-board and the background fluorescence droplet may be transported to a waste reservoir.

### 5.6.14 Quantifying amount of nucleic acid in the sample

In step 618, each of the cleanup droplets A-J (step 616) may be transported using droplet operations to a quantification section of the PCB cartridge for quantification. In this quantification step, the amount of total nucleic acid in each of the droplets may be determined using a fluorescence measurement. In one example, droplet operations may be used to separate 1 nL from each of the cleanup droplets A-J (from step 616) and transport each droplet separately to one of dried reagent spots containing SYBR Green or SYBR Gold Master Mix. Droplet operations may be used to pulse the cleanup droplets A-J over the dried dye spot to ensure complete rehydration and uniform mixing of the intercalating dye, producing quantification droplets A-J. Immediately after the cleanup droplets A-J come in contact with the dried SYBR Green (SYBR Gold) master mix reagent spot, the fluorescence of each drop may be captured using an on-cartridge CCD camera at, for example, a wavelength of 520 nm for 5 minutes.

In some configurations of the disclosure, the fluorescence of each of the quantification droplets A-J may be compared to an embedded, laboratory-generated, standard curve of PCR cycles as a function of fluorescence for varying nucleic acid concentrations. In this configuration, a standard curve of PCR cycles as a function of fluorescence for varying nucleic acid concentrations may be stored on cartridge. The fluorescence of each of the quantification droplets A-J may be compared to the stored standard curve to determine the total nucleic acid present in each droplet. After the total amount of nucleic acid in each of the quantification droplets A-J is determined, a calculation may be performed by the processor to determine the exact dilution necessary to ensure the nucleic acid concentration in each droplet is within the range dictated by the fluorescence detector.

In another configuration, 3 or 4 or 5 or more than 5 nucleic acid standards of known concentration may be spotted as dried reagents on the cartridge and may be used to generate a standard curve in-situ. For example, one droplet of buffer may be dispensed from an on-board buffer reservoir using droplet operations and then transported to a dried spot of intercalating dye, for example SYBR Green (SYBR Gold) master mix. In a next step of this configuration, droplet operations may be used to separate the rehydrated SYBR Green (SYBR Gold) master mix reagent spot into a certain number of droplets that corresponds to the number of dried nucleic acid standards provided on the cartridge. In this example, the number of rehydrated intercalating dye droplets may be 3 or 4 or 5 or more than 5 rehydrated dye droplets. Droplet operations may be used to transport each of the rehydrated SYBR Green (SYBR Gold) master mix droplets to each of the nucleic acid standard spots. Droplet operations may be used to pulse each of the intercalating dye droplets over each of the nucleic acid standard spots. After complete rehydration and uniform mixing, fluorescence may occur and a standard curve may be generated of nucleic acid concentration vs. fluorescence signal. The amount of nucleic acid in each of the quantification droplets A-J (step 618) may then be compared to the standard curve to determine the amount of nucleic acid in each quantification droplet.

In another configuration, one concentrated standard nucleic acid spot may be dried on the cartridge, rehydrated and then diluted into 3, or 4, or 5, or more than 5 nucleic acid standard droplets. For example, droplet operations may be used to dispense one buffer droplet from an on-board buffer reservoir and transport the buffer droplet to the dried, concentrated standard nucleic acid spot. Droplet operations may then be used to pulse the buffer droplet over the concentrated nucleic acid standard spot to ensure complete rehydration and unform mixing. Droplet operations may then be used to separate the rehydrated concentrated nucleic acid standard droplet into 3, or 4, or 5, or more than 5 droplets.

In some configurations, droplet operations may be used to transport buffer droplets to each of the 3, or 4, or 5, or more than 5 nucleic acid standard droplets to dilute them to the appropriate concentration.

In another configuration, one droplet of buffer may be dispensed from an on-board buffer reservoir and transported using droplet operations to merge with one dried intercalating dye reagent spot (e.g., SYBR Green (SYBR Gold)). Droplet operations may be used to pulse the one droplet of buffer over the dried intercalating dye reagent spot to ensure complete rehydration and uniform mixing.

Droplet operations may be used to split the one droplet of rehydrated intercalating dye buffer reagent into the same number droplets as the number of nucleic acid standard droplets that may be provided. In one configuration, the dyed buffer reagent droplet may be split into 3, or 4, or 5, or more than 5 droplets.

Droplet operations may be used to transport each of the dyed buffer reagent droplets to merge with each of the nucleic acid standard droplets. Droplet operations may be used to pulse the merged dyed buffer and nucleic acid standard droplets to ensure uniform mixing. Once the nucleic acid has incorporated the intercalating dye, fluorescence may occur. In one configuration, a standard curve may be generated of nucleic acid concentration vs. fluorescence signal. In one configuration, the amount of nucleic acid in each of the quantification droplets A-J (step 618) may be compared to the standard curve to determine the amount of nucleic acid in each quantification droplet A-J.

After concentration of nucleic acid in each of the quantification droplets A-J (step 618) is obtained, a calculation may be performed by an on-board processor to determine the exact dilution necessary to ensure the nucleic acid concentration in each droplet may be within the range dictated by the fluorescence detector. For example, if the fluorescence detector determines an appropriate amount of nucleic acid has been created in the amplification step (step 614), droplet operations may be used to transport the quantification droplets A-J to the array detector 620. In one configuration of the quantification step, if the processor determines there is insufficient nucleic acid present in each of the quantification droplets A-J, an error flag may be called which may stop the entire assay and return an error message for the end user.

In one configuration, if the fluorescence detector determines there is excess nucleic acid present in the sample such that the droplet would saturate the array detector, a calculation may be performed by the instrument to determine an appropriate dilution needed for each droplet. In some configurations, amplifying the sample nucleic acid to more than the amount needed by the detector may be desired. Based on the dilution factor determined in the quantification step 618, a calculation may be performed to determine what volume of buffer (e.g., Tris buffer) may be needed to appropriately dilute each of the quantification droplets A-J. For example, droplet operations may be used to transport the appropriate volume of buffer to each quantification droplet A-J. Droplet operations may be used to pulse each of the quantification droplets A-J with its respective dilution buffer droplet to ensure uniform mixing and dilution, producing diluted droplets A-J.

In some cases, it may be known in advance that in the original sample there is a high abundance of a particular target relative to the other targets. In this configuration, the final PCR product may be a function of how many coded padlock probes anneal to the same target sequence. In this configuration, the number of coded padlock probes contacting the target strands may be physically reduced which may stunt amplification to prevent saturation of the detector from one target sequence.

In step 618, droplet operations may be used to separate a small volume of approximately 1 to 10 pL from each diluted droplet A-J for analysis, producing picoscale droplets A-J. Droplet operations may be used to transport each of the picoscale droplets A-J to one of dried reagent spots which contain all the requisite reagents, except the PCR primers and the fluorescent intercalating dye, required for PCR-initiated fluorescence detection on an array detector 620. These reagents include, for example, TaqPath ProAmp master mix, Taq DNA polymerase, each of the four dNTPs, stabilizers, buffers, enzymes such as RNAse H2 enzyme, and MgCl₂. Droplet operations may be used to pulse each of the picoscale droplets A-J to ensure complete rehydration and uniform mixing of the reagents with the nucleic acid in the sample. This step produces detection droplets A-J.

### 5.6.15 Transfer the sample droplet to CMOS chip

The detection droplets may be transported to an array detector for detection of target nucleic acids. For example, the detection droplets may be transported using droplet operations, such as electrowetting-mediated droplet operations.

Referring again to FIG. 7 is a schematic diagram of an array detector zone in a detector array. In the above example of detecting 100 targets, the detection features 722 and 724 in array detector zone A 720 may contain the dried intercalating dye and forward and reverse primers necessary to detect nucleic acid targets 1-10 found in detection droplet A. The detection features in array detector zone B may contain the dried intercalating dye and forward and reverse primers necessary to detect nucleic acid targets 11-20 found in detection droplet B. Each of the remaining detection droplets C-J may be transported to their respective array detector zones C-J to detect the specific targets 21-100, for example. Continuing the example of detecting 100 targets, the first row of detection features, 722 and 724, in array detector zone A 720 may contain the dried intercalating dye and forward and reverse primers to detect nucleic acid target 1. The second horizontal row of detection features in array detector zone A 920 may contain the dried intercalating dye and the forward and reverse primers necessary to detect nucleic acid target 2. The last horizontal row of detection features 722 and 724 in array detector zone A 720 may contain the spotted dried intercalating dye and the forward and reverse primers necessary to detect nucleic acid target 10. This pattern may be repeated for array detector zone B and nucleic acid targets 11-20 and similarly for the other array detector zones C-J, the corresponding detection droplets C-J and the corresponding nucleic acid targets 21-100.

In one configuration, two or more rows in each zone are used to detect each target. In one configuration, each detection feature in each zone is used to detect a unique target. In one configuration, all wells in a particular zone are used to detect one target.

### 5.6.16 Rehydrate primers

Droplet operations may be used to transport each detection droplet A-J to the appropriate detector zone A-J. When the droplet lands in the detector zone, it may flood the detection features located in that zone. Droplet operations may be used to pulse the liquid in each detector zone and in each detection feature to ensure complete rehydration and uniform mixing of each detection droplet A-J with the dried forward and reverse primers and the intercalating dye pre-spotted in or on each detection feature. Immediately upon rehydration and mixing with the nucleic acid containing droplets, the intercalating dye may begin to fluoresce. This fluorescence may be used to detect the presence of mutant allele targets and is discussed further below.

In one configuration, the sample pool may include 10,000 targets split into 10 pools (droplets) transported to different detection zones, where each detection zone contains 1000 targets each. In one embodiment, a unique PCR tag may be used for each separate target sought. If a unique PCR tag is used for each target, in this example 1000 different PCR tags would be required to discriminate between each of the 1000 targets in one detection feature. The same 1000 PCR tags may be used in each detection zone because the spatial separation may be used to discriminate targets in different zones. For example, zone A may use 1000 tags to detect targets 1-1000 and zone B may use the same 1000 tags to detect targets 1001-2000. Using 1000 unique PCR tags may increase manufacturing and production costs and may significantly increase manufacturing time as described below. In one configuration, each target may be detected by a unique combination of two primers reused over all zones which significantly reduces the number of primers needed. The unique combinatorial PCR tags (generated using the unique PCR tag sequence primers 906 and 908) on the coded padlock probes are shown in light gray in FIG. 9. The combination of forward and reverse primers which hybridize to the unique PCR tags may be pre-spotted in or on each detection feature.

### 5.6.17 Combinatorial fwd/rev pcr primers spotted in detection features

FIG. 11 is a schematic diagram of an example of a detection zone layout 1100 representing the grid of wells (722 & 724) found in each detection zone A-J of CMOS array detector 708 shown in FIG. 7. During the manufacture of the CMOS chip, an array of forward and reverse PCR primers and an intercalating dye such as SYBR Green, SYBR Gold, or Eva Green may be spotted in or on each detection feature and dried using for example forced air. In each zone, an array of fifteen, for example, forward PCR primers T₁F through T₁₅F may be spotted in or on each detection feature of the rows and an array of fifteen, for example, reverse primers T₁R through T₁₅R may be spotted in or on each detection feature of the columns. Therefore, each detection feature may contain a unique combination of at least two dried primers, at least one forward and at least one reverse primer.

In FIG. 11 the array of forward PCR primers is indicated by T₁F through T₁₅F and the array of reverse primers is indicated by T₁R through T₁₅R. Each of the 15 forward primers may be a unique sequence in the group of forward primers and each of the 15 reverse primers may be a unique sequence in the group of reverse primers.

In one configuration, the forward primer of any one coded padlock probe may be selected from one of 15 forward primers and the reverse primer of the same coded padlock probe may be selected from one of 15 reverse primers, as shown in FIG. 11. For example, one uniquely identifiable coded padlock probe may have forward primer 1 (T₁F) and reverse primer 1 (T₁R). Another uniquely identifiable coded padlock probe may have forward primer 1 (T₁F) and reverse primer 2 (T₂R). In one configuration, the incorporation into the coded padlock probe of these primer tags in a combinatorial fashion may be used to create a unique identifier which may be used to differentiate individual coded padlock probes. The combination of 15 forward and 15 reverse primers allows the possibility of having 225 unique combinatorial PCR tags which may be used to detect the presence of 225 unique mutant allele targets per zone. This combinatorial approach may allow one to use fewer complementary primers in the detection step. The PCR forward and reverse tags, 906 and 908 respectively, may be repeated in all zones; however they may be used uniquely in each subpool or within one zone. Because the different zones of the detector may be spatially separated, the same pair of PCR primers in one zone may be used to detect a different target mutant allele than the same pair of PCR primers located in a different zone.

In one configuration, the 15 unique forward primers may be different from the 15 unique reverse primers, creating a total of 30 unique primers. In another configuration, the 15 unique forward primers may be the same primers used for the 15 unique reverse primers creating a total of 15 unique primers.

In one configuration, the exact location of the combinatorial primers is known. If the matching target arrives from a detection droplet and floods the corresponding detection feature, PCR amplification may occur. After a sufficient quantity of target molecules are generated and after a sufficient quantity of intercalating dye is incorporated a fluorescent signal may occur. The result may be a binary detection output signal. While T₁F+T₁R may be used in the upper left corner, for example, of each zone, a fluorescence signal from the upper left corner of different zones may indicate the presence of a different target.

In one configuration, one or more wells in each zone may contain unique PCR tag sequences which may be used to detect the internal positive and negative control sequences.

### 5.6.18 Mapping detection feature Primers & Detected Targets

In one configuration, the location of which targets may be detected in which specific detection features may be precisely mapped. Spatially separating the same combinatorial array of PCR primers allows an increased number of targets to be detected, while using fewer number of PCR primers. In one configuration, using this combinatorial approach, 15 forward and 15 reverse primers may be combined to detect 225 unique targets per zone. In one configuration, there may be a 10x10 array of zones, which creates the possibility of detecting a total of 225,000 unique targets over all zones using only 15 unique primer sequences. In one configuration, each detection zone may contain a 316 x 316 array of unique forward and reverse primers. In one configuration, a 316 x 316 array of unique primers may be combined with an array detector having a 10 x 10 array of zones to allow the unique identification of approximately 10 million unique mutant allele targets.

In one configuration, reusing the primers improves the amount of detectable targets while improving the manufacturing time. Spotting a primer in a detection feature is a fast process, where approximately 1,500 drops may be spotted per second. However, the wash cycle required between each solution change is a slow process of approximately 1.5 minutes. Therefore, it is advantageous to make use of each primer as many times as possible to reduce the time required for the washing cycle. If for example, 100,000 unique PCR primers were used to detect 100,000 unique targets, the amount of time spent on the wash cycle alone would require years. In one configuration, primer T₁F may be spotted 15 times in every detection feature of the first row of each zone. In one embodiment, the delivery lines are then washed and another primer, for example T₂F, may be spotted 15 times in every detection feature of the second row of each zone. In this embodiment, the time required for the washing step is minimized.

In one configuration, the forward and reverse primers may be dried inside each detection feature. In one configuration, the forward and reverse primers may be chemically coupled to the inside of the detection feature. In another configuration, the forward and reverse primers may be attached to magnetic beads and held in place with a magnet below the wells.

### 5.6.19 Fluorescence detection

In one configuration, droplet operations may be used to transport a droplet of buffer to a detection feature containing dried PCR primers and an intercalating dye to determine a background noise fluorescence signal. A fluorescence measurement may be taken at 520 nm for example and collected for approximately 1 or more, 10 or more, 20 or more, 30 or more or 60 or more seconds. The background fluorescence signal may be collected and stored on the cartridge.

In one configuration, droplet operations may be used to transport each of the detection droplets A-J to the correct section of the array detector zones. Droplet operations may then be used to pulse each of the detection droplets A-J to rehydrate and uniformly mix the combinatorial PCR primers and the intercalating dye with the nucleic acid sample. Immediately upon rehydration and mixing with the nucleic acid containing droplets, the intercalating dye may begin to fluoresce in the range of the background noise.

In one configuration, PCR amplification may be performed to increase the amount of nucleic acid in each droplet until a fluorescence signal is obtained beyond that of the background noise. In this configuration, the wells in each zone may be heated to 95°C for 5 minutes to activate the DNA polymerase. In one configuration, the zones may be heated to 94-98°C for 20-30 seconds to convert the double stranded nucleic acid present in the droplet to single-stranded nucleic acid. In an configuration, the rehydrated primers pre-spotted in the detection feature may anneal to the complementary sequence of the PCR tag sequences in the original coded padlock probes. In one configuration, the temperature of the zones may be reduced to 50-65°C to allow for optimal primer annealing. In another configuration, the temperature of each zone may be increased to 72-78°C to allow optimal template extension by Taq polymerase. This step may proceed for a certain length of time. In one configuration, 15-20 PCR cycles may be performed. Subsequently a fluorescence measurement may be collected using a standard fluorescence detector. In another configuration, PCR amplification may be allowed to continue for 2 additional cycles before another fluorescence measurement is collected. In one configuration, a fluorescence measurement may be collected after cycle 32 for example. In one configuration, a fluorescence measurement may be collected after 45 cycles. In one configuration, a fluorescence measurement may be collected after every PCR cycle. In one configuration, a fluorescence measurement may be collected after every third PCR cycle. The fluorescence signal as a function of PCR cycle number may be stored on cartridge. The on-cartridge processor may include an algorithm which determines when the fluorescence signal increases above a predetermined threshold indicating a positive result for that target. In one configuration, a fluorescence map may be made corresponding to specifically mapped detection features and which may be used to detect the presence of mutant target alleles. Since a precise map of each zone may be created at the time of reagent spotting, one may precisely determine which target mutant allele is fluorescing.

In one configuration, a fluorescence endpoint assay may be conducted where PCR amplification may be allowed to proceed for 45 cycles. In this configuration, a fluorescence signal may be collected after the 45 cycles have completed.

### 5.6.20 Increased specificity using RNase H2 additional step

In one configuration, HiFi DNA Ligase may seal nicks in the coded padlock probes which have correctly hybridized to a mutant allele. The product may then be sent to an amplification step and further on to detection. HiFi Taq DNA Ligase is the highest fidelity ligase available commercially. However, this ligase is not error-free. If the HiFi Taq DNA Ligase erroneously seals a nick in a coded padlock probe which has hybridized to a wildtype allele, an error has been incorporated. It would be disadvantageous to amplify this product. Therefore, in one configuration, a "post-processing" step after coded padlock probe ligation (step 612 of FIG. 6) and before amplification (step 614 of FIG. 6) using the RNase H2-dependent PCR protocol may be subsequently implemented to amplify only coded padlock probes which have hybridized to a mutant allele target. The current state of the art in nucleotide detection uses either coded padlock probe PCR detection or RNAse H2-dependent PCR detection. These two detection schemes may be coupled together. These two processes together significantly improves assay specificity to detect single nucleotide mutations.

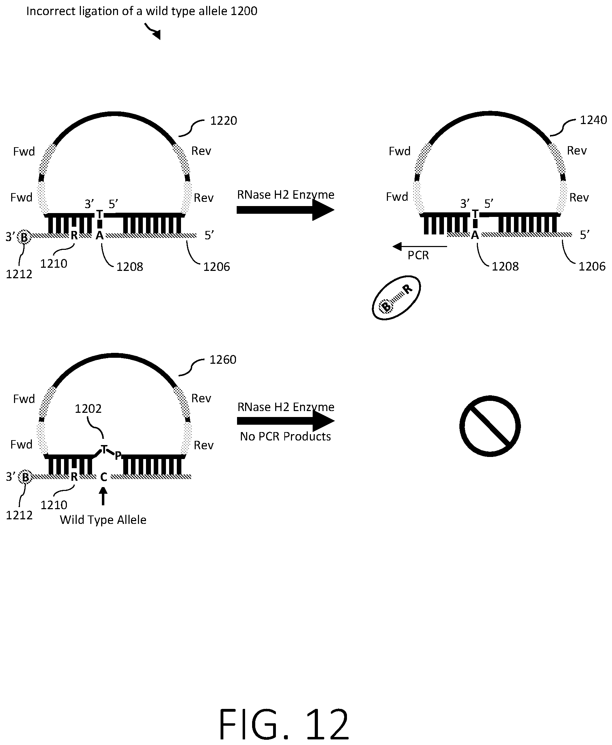

In a first layer of specificity, the highest fidelity ligase should circularize only coded padlock probes which correctly complement the mutant allele sequence. A next step may include denaturation and digestion which separates the hybridized sequence from the circularized coded padlock probes and then digests any linear material in the droplet. In a second layer of specificity, RNAse H2 primers may be presented to the circularized coded padlock probes. FIG. 12 is a schematic diagram of an example showing the use of RNAse H2 primers to increase specificity in a coded padlock probe ligation reaction. If the sequences 100% align between the ligated coded padlock probe 1220 and the RNAse H2 primer 1206, the primer may be cut with RNAse H2 which removes a blocking function 1212 allowing PCR to occur (1240).

If the high fidelity ligase makes an error during ligation, for example incorrectly sealing a coded padlock probe which hybridized to a wildtype allele having incorrectly matching nucleotides there will be a bulge in the coded padlock probe. After denaturation and digestion, RNAse H2 primers 1206 specific for a mutant allele may be presented to the circularized coded padlock probes. If the RNAse H2 primers 1206 hybridize to a wild type allele coded padlock probe 1260, this may create a site 1202 where mismatched nucleotides do not hybridize. This non-hybridized nucleotide pair prevents RNAse H2 release of the PCR blocking group 1212 and PCR does not occur for the incorrectly paired nucleotides on the wild type allele. After the RNAse H2 clean-up step, the droplets containing the circularized coded padlock probes may be returned to the original workflow at the amplification step (step 614 of FIG. 6).

In this configuration, the coded padlock probes in each coded padlock probe droplet A-J may be separated from the target mutant allele sequence or the incorrectly paired wildtype allele sequences by heating the reaction to 94-98°C for 20-30 seconds.

Droplet operations may be used to transport one droplet of rehydrated exonuclease I enzyme (described above) into contact with each coded padlock probe droplet A-J. Droplet operations may further be used to merge and pulse the exonuclease I enzyme with each coded padlock probe droplet A-J. This step may digest any linear coded padlock probes, any linear cfNA, any unused primers and any mutant or wildtype linear nucleic acid strands present in the droplet into 1-2 nucleotide bases.

Droplet operations may be used to transport each coded padlock probe droplets A-J to a dried reagent spot containing specific forward and reverse oligonucleotide RNAse H2 primers 1206. All primers and probes may be synthesized by Thermo Fisher (Waltham, MA) for example and stored at -20°C until use. Droplet operations may be used to pulse the coded padlock probe droplets A-J over their respective dried RNAse H2 primers spot to ensure complete rehydration and uniform mixing. Droplet operations may be used to transport a droplet of buffer to mix with each coded padlock probe droplet A-J. The buffer droplet may include 10 mM Tris pH 8.0, 1 mM EDTA, 100 mM NaCl, 0.1% Triton X-100. In another configuration, the reaction temperature of each coded padlock probe droplet A-J (step 614) may be lowered to 50-65°C for 20-40 seconds to allow the primers to anneal to the circularized coded padlock probes, forming rhPCRPrimers droplets A-J.

The RNAse H2 PCR primers may be specifically designed to be complementary to the 3'-arm of the coded padlock probes which previously hybridized to the target mutant allele. As shown in FIG. 12, the RNase H2 primers 1206, shown in dark grey, may have the same sequence as the 5' end of the mutant target nucleic acid strand and may have a nucleotide which matches the mutant nucleotide 1208. In panels 1220 and 1240, mutant nucleotide 1208 is an A, for example. Proceeding in the 3' direction of RNAse H2 primer 1206 may be a ribonucleotide 1210, and further in the 3' direction may be a blocking entity 1212. In one configuration, the blocking entity in the RNAse H2 PCR primer may be a three-carbon spacer moiety. In one configuration, the RNAse H2 PCR primers may include a chemical blocker such as a di-deoxy nucleotide moiety without an extendable hydroxyl group. In another configuration, the RNAse H2 Primer may be a GEN2 primer from Integrated DNA Technologies (Coralville, IA) where the highest fidelity template amplification is required. According to Integrated DNA Technologies,"the GEN2 primer may be represented by DDDDDDDDrDxxDM, where D represents a DNA base, r represents the RNA base, and x represents the blocker. Inserting a mismatched DNA base at the 3' end of the primer to create the "DM" combination ensures maximum effectiveness of the end block."

RNAse H2 enzyme may be introduced. In this step, droplet operations may be used to transport each rhPCR primers droplets A-J to a dried reagent spot containing an rhAmp (RNase H2 enzyme) genotyping master mix available from Integrated DNA Technologies (Coralville, IA), Tris-HCl, NaCl, MgCl₂, BSA, Triton X-100 and DNA Polymerase (available from Thermo Fisher (Waltham, MA)) for example. The rhPCRPrimers droplets A-J may be pulsed over their respective dried reagent spots to ensure complete rehydration and uniform mixing. This rehydration period may be followed by for example 4-10, 60 or 90 cycles of 10 seconds at 95°C and 90 seconds at 60°C. At this step, the rhAmp (RNAse H2 enzyme) may cleave the primer 5' to the single RNA base 1210, leaving an exposed 3'-OH. The segment may now be active as a primer and may be extended using the rehydrated DNA polymerase present in the droplet, step 614 of FIG. 6. If a mismatch is present near the RNA site, RNase H2 has a significantly decreased efficiency of cleavage and therefore the chance of PCR amplification occurring is further reduced. This provides a third layer of selectivity for the mutant target of interest. PCR amplification proceeds for 4-10 cycles or more than 10 cycles until reaction quenching occurs by the droplet operations transport of a 1 µL of EDTA which may merge with each rhPCRPrimers droplets A-J producing amplified droplets A-J.

In one configuration, complementary RNAse H2 PCR primers may be spotted in or on each detection feature of the CMOS array detector instead of the T₁R through T₁₅R reverse primers. The single nucleotide detection may proceed from the amplified droplets A-J as described above for fluorescence detection.

### 5.6.21 Indirect single nucleotide detection using TaqMan Probes

In one configuration, the coded padlock probe of FIG. 9 may be synthesized to include TaqMan probe sequences for detection purposes. The TaqMan probes may contain reporter and quencher sequences.

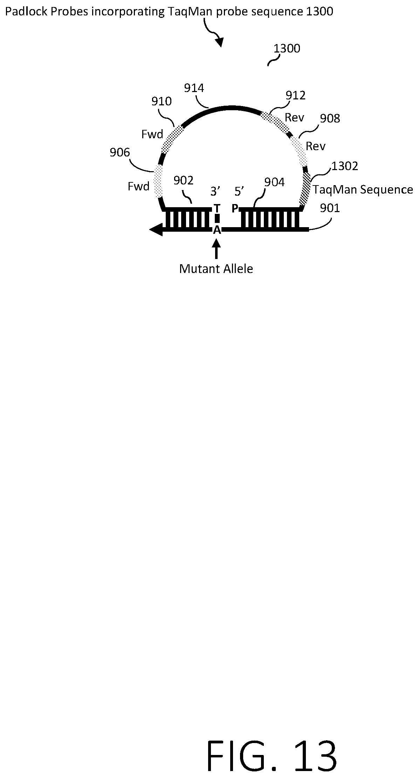

FIG. 13 is a schematic diagram of an example of a process 1300 including coded padlock probes incorporating TaqMan probe sequences. The forward 906 and reverse 908 PCR tag sequences, the forward 910 and reverse 912 universal amplification sequences and the non-extendable (spacer) sequence 914 may be the same as shown in FIG. 9. In this embodiment, a TaqMan probe sequence 1302 may be included in the coded padlock probe synthesis. The order of the sequences may be altered to fit various experimental needs. This disclosure includes various arrangements of the sequences in the coded padlock probes. In another configuration, a TaqMan probe sequence may be synthesized such that it is located on the 5'-arm of the coded padlock probe and is complementary to the 3' end of the mutant target sequence. In this configuration, the TaqMan probe may be designed to ensure it anneals to the correct allele. In one configuration, the TaqMan probes may be pre-spotted in the detection features. During PCR amplification these probes may anneal to the amplicon PCR product. In one configuration, the TaqMan probe may anneal to the duplex nucleic acid strand creating a third layer. In another configuration, TaqMan probes may be designed to provide two-color PCR. In one configuration, a VIC fluorescent tag may be used. In another embodiment, a SYBR Green or SYBR Gold fluorescent intercalating dye may be used as the fluorescing molecule. In the configuration of using two color PCR, the number of mutant target sequences detected may be doubled. The probe may bind to the nucleic acid between the forward and reverse primer. While the reporter and quencher may be bound to the probe, the quencher may absorb the fluorescence emitted by the reporter. During the extension phase of the PCR reaction the probe may be degraded, releasing the reporter and allowing its fluorescence to be detected. The advantage of the TaqMan method is that probes with different colored reporters may be combined in multiplex assays.

For both SYBR Green (SYBR Gold) and TaqMan methods, the amount of fluorescence in a sample may be detected in 'real-time' and plotted against the cycle number. The amount of fluorescence may be proportional to the amount of PCR product. The time point at which the fluorescence reaches a defined threshold is relative to the level of nucleic acid amplification. The design of real-time PCR experiments requires prior knowledge of the gene sequence and careful consideration of the types of controls to include.

### 5.6.22 Indirect Single Nucleotide Detection Using UMI Probes

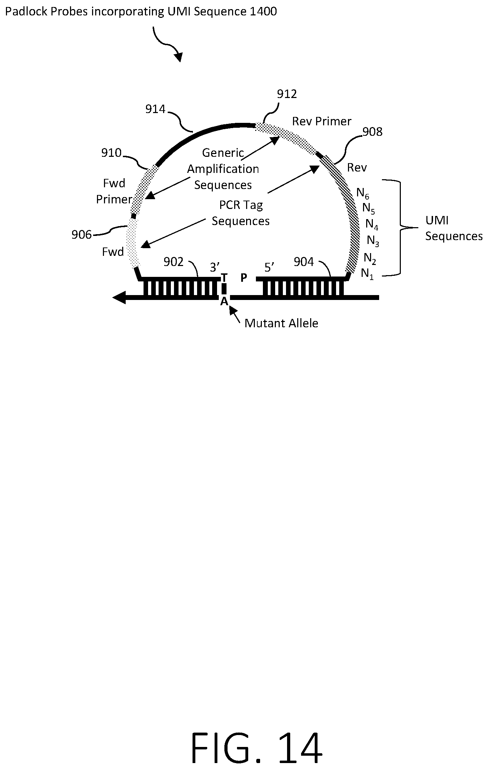

In one configuration, the coded padlock probe shown in FIG. 9 may be synthesized to include Unique Molecular Identifiers (UMI) or index barcodes and the forward and reverse PCR Tag sequences. For example, FIG. 14 is a schematic diagram of an example of a coded padlock probe incorporating UMI sequences. In this configuration, the UMIs or index barcodes may be used to provide a second level of selectivity.

In one configuration, the UMI sequence or barcode is from 5 to 25 nucleotides long, or 5 to about 20 nucleotides long, or about 5 to 15 nucleotides long. In another configuration, the UMI or barcode sequence may be more than 25 nucleotides long. In another configuration, the UMI or barcode sequence may be less than 5 nucleotides long.

In one configuration, the 3'-arm and the 5'-arm of the coded padlock probe may be synthesized to be complementary to the mutant target sequence of interest. In this configuration, each coded padlock probe which is synthesized to be complementary to one particular target may include a different UMI or barcode sequence. In this configuration, each UMI may be used to determine the total number of a specific target sequence which were present in the original sample. In another configuration, a UMI sequence may be used as a quality control check to determine if a mutation was present in the original sample or if it was artificially introduced during PCR processing.

In contrast with the code, which is a predetermined, known sequence, the barcode or UMI may in some instances be a random sequence.

FRET probes and other types of self-detecting probes may be used in a similar manner.

### 5.6.23 Indirect single nucleotide methylation detection

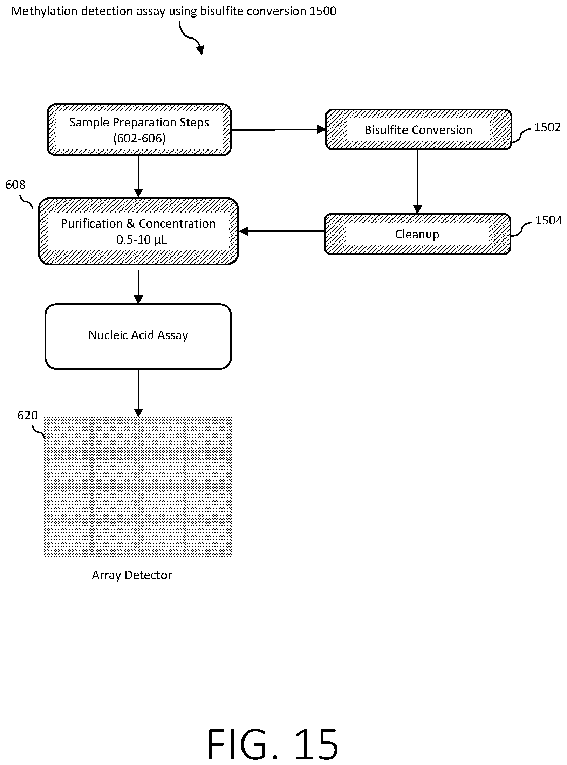

FIG. 15 is a flow diagram of an example of a process 1500, which is a process of methylation detection assay using bisulfite conversion. Process 1500 for methylation detection using bisulfite conversion is described below for a DMF system 400; however, it will be appreciated that other systems, such as robotics systems or other microfluidics systems, may be used. In one configuration of the assay, single nucleotide methylation may be detected using the indirect coded padlock probe method described above. In this configuration, the sample preparation steps 602-606 of assay workflow 600 shown in FIG. 6 may generally be followed in process 1500. The sample preparation steps of process 1500 may be the same with respect to collecting the blood sample (602), separating the plasma fraction from that sample (604) and using droplet operations to transport the sample droplets to a bead-based nucleic acid extraction step (606). In this configuration, the nucleic capture beads may generically capture all nucleic acid from the sample, both methylated and unmethylated.

In step 1502, a bisulfite conversion reagent droplet is prepared and the bisulfite conversion reaction is performed. For example, droplet operations may be used to transport one droplet of a buffer (e.g., Tris buffer) to merge with a dried sodium or ammonium bisulfite reagent spot. Droplet operations may be used to pulse the buffer droplet over the dried sodium or ammonium bisulfite reagent spot to ensure complete rehydration and uniform mixing. The rehydrated bisulfite-buffer droplet may be transported using droplet operations to merge with the magnetically bound macroscale NA-beads droplet (see step 606 of assay workflow 600). The macroscale NA-beads droplet may be incubated for 1.5 hours at room temperature for example with the bisulfite-buffer droplet to ensure complete deamination of unmethylated cytosine residues to uracil, leaving any 5-methyl cytosine or 5-hydroxymethyl cytosine residues unmodified. This step produces an HSO₃-NA droplet.

In step 1504, a magnetic bead cleanup protocol is performed. For example, while maintaining the magnetic field, a cleanup step may be performed where droplet operations may be used to replace any material not bound to the magnetic beads with buffer. For example, the beads may be washed with a Tris buffer to remove any material not bound to the beads such as any unreacted reagents or other contaminants. Droplet operations may be used to add enough volume of buffer to produce a 25 µL droplet, for example.

The HSO₃-NA droplet volume may be reduced as described in step 608 of FIG. 6. For example, while maintaining the magnetic field, droplet operations may be used to remove all but 0.5 to 10 µL, for example, of surrounding solution from the bound HSO₃-NA droplet to produce a NA-beads droplet having a droplet volume of 0.5-10 µL (500-10,000 nL), for example. In one configuration, the remaining volume in the HSO₃-NA droplet may be transported to a waste reservoir using droplet operations. Using on-board thermal controls, the NA-beads droplet may be heated to 80°C to release the bead-bound nucleic acid into the buffer solution, producing a 0.5-10 µL (500-10,000 nL) cfNA droplet. The cfNA droplet may proceed through the remaining steps in the nucleic acid assay as described above and shown in FIG. 6.

In another configuration, an enzyme-based methylation analysis technique such as NEBNext^{®} Enzymatic Methyl-seq (EM-seq^{™}) (New England Biolabs, NEB #E7120) may be used on cartridge to significantly reduce nucleic acid degradation.

### 5.6.24 Direct detection of single methylated bases using molecular wires

In one configuration, a molecular wire hybridization sequence may be used to detect a single methylation site on a single nucleotide in a strand of nucleic acid. Per the method of Roswell Technologies, a molecular wire made up of a protein alpha helix structure may be attached to a substrate using semiconductor technology. In the Roswell Technologies method, the protein wire is conductive and has a measurable electrical signal. A nucleic acid capture probe may be attached to the conductive protein wire, where the capture probe may be complementary to a single-stranded nucleic acid sequence of interest. In another example, a complex may be formed to bind streptavidin to the wire. A biotinylated capture probe may be bound to the streptavidin. The molecules attached to the protein wire may generate a background electrical signal. When any molecule such as another protein, a single nucleotide, a strand of nucleotides, ligands, antibodies or streptavidin, for example, binds to the wire or the complex attached to the wire, a transient but measurable change in the electrical signal is generated. According to Roswell Technologies (personal communication) the molecular wire may detect when only one single nucleotide base hybridizes to the complementary capture probe. When involving nucleic acid, the Roswell method is used only for nucleic acid sequencing. The Roswell method is not typically used to detect nucleic acid mutations or methylated nucleotides.

Making use of the Roswell method, a Roswell molecular wire may be attached to a semiconductor substrate, a complementary nucleic acid capture probe may be attached to the molecular wire and then the probe may be used to capture the complementary target nucleic acid sequence of interest. The Roswell method is unable to resolve the differences in methylation status of these captured target nucleic acid sequences as it detects only that an entity has bound but not any specifics about that entity.

A configuration of the present disclosure makes use of the Roswell molecular wire and the mutated nucleotide assay described above to directly detect the methylation status of a captured nucleic acid sequence.

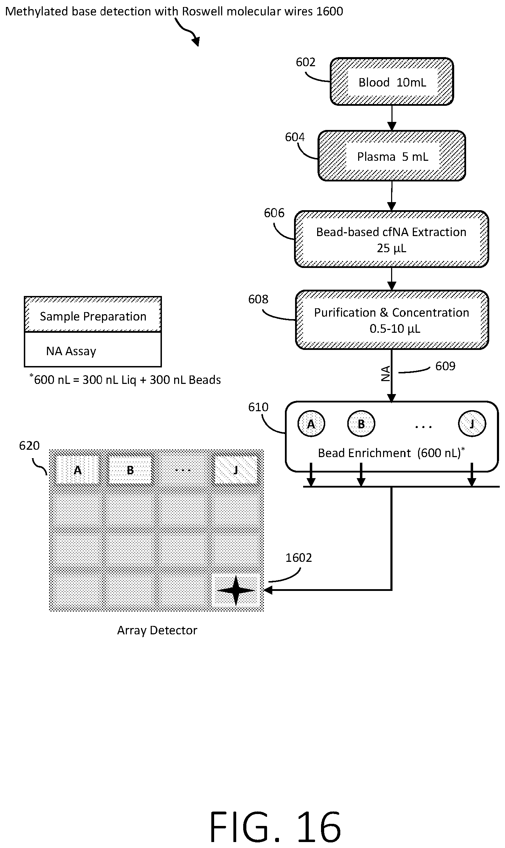

FIG. 16 is a flow diagram of an example of a process 1600, which is a workflow for a methylated base detection assay with Roswell molecular wires. For example, process 1600 makes use of the Roswell molecular wire and the mutated nucleotide assay described above to directly detect the methylation status of a capture nucleic acid sequence. Process 1600 for methylated base detection using Roswell molecular wires is described below for a DMF system 400; however, it will be appreciated that other systems, such as robotics systems or other microfluidics systems, may be used.

Process 1600 may be based on the assay workflow 600 shown in FIG. 6. For example, in process 1600, the steps of assay workflow 600 with respect to collecting the blood sample (602), separating the plasma fraction from that sample (604) and using droplet operations to transport the sample droplets to a bead-based cfNA extraction step (606) may be used. In this embodiment, the nucleic acid capture beads may generically capture all nucleic acid from the sample, both methylated and unmethylated. The next few steps of the assay workflow 600 apply to the presently described embodiment where the cfNA sample may be purified and concentrated (step 608) and droplet operations may be used to transport the concentrated droplet to a bead enrichment step. The droplets entering the bead enrichment step are referred to as ssNA droplets A-J.

In one configuration, droplet operations may be used to transport each of the ssNA droplets A-J to the region of the cartridge for a bead enrichment step (step 610 of FIG. 6). In this configuration, one or more bead pools out of the total number of bead pools may be used to capture nucleic acid sequences using the Roswell molecular wire direct detection method. In this configuration, one or more of the bead pools B_{A} - B_{J}, for example, may contain magnetic beads-streptavidin-biotin-capture probe complexes where the capture probe may be complementary to the potentially methylated target nucleic acid strand of interest. Upon reaching the magnetic beads, droplet operations may be used to ensure complete mixing of the droplets with the magnetic beads. The reaction temperature may be lowered to 50-65°C for 20-40 seconds to allow for optimal annealing of single stranded nucleic acid to the capture probes. In this configuration, the complementary capture probe may capture both methylated and unmethylated strands of the correct sequence. In a configuration described below, methylation discrimination may happen in a later step. In one configuration, the complementary capture probes may not discriminate against non-methylated nucleic acid, methylated strands of nucleic acid or any of its oxidized derivatives. Thus, the complementary capture probes may extract one or more of 5-methylcytosine (5mC), 5-hydroxymethylcytosine (5hmC), 5-formylcytosine (5fC), and 5-carboxylcytosine (5caC). As described above, droplet operations may be used to transport any unwanted material away from the magnetically bound beads which have captured the target nucleic acid strand of interest. For example, any molecules not bound to each of the target NA-capture probe-biotin-streptavidin-beads droplets may be replaced by Tris buffer, for example, using droplet operations. In one configuration, the desired target nucleic acid strand may be released from the magnetic beads by increasing the reaction temperature to 80°C for several minutes to release the bead-bound nucleic acid into the buffer solution. The operations in this embodiment may produce one or more droplets with single-stranded target nucleic acid which has hybridized to the complementary capture probe.

In an alternate configuration, droplet operations may be used to merge two Tris buffer droplets for example over a dehydrated Qiagen bead elution buffer reagent spot. Droplet operations may be used to pulse the Tris buffer, for example, droplets over the Qiagen bead elution buffer reagent spot to ensure complete rehydration and uniform mixing. This produces two elution buffer droplets. Keeping the magnetic field active, droplet operations may be used to merge and pulse one elution buffer droplet with each target NAprobe-beads droplet, causing the captured target nucleic acid sequences to elute into the droplet to produce one or more droplets containing double stranded target nucleic acid sequences.

In another configuration, the double-stranded nucleic acid formed from the hybridization of the target nucleic acid strands to the complementary capture probes may be denatured to release the target nucleic acid strands. In this configuration, the one or more target nucleic acid containing droplets may be heated to 94-98°C for 20-30 seconds to break hydrogen bonds between the individual nucleic acid strands.

In one configuration, the one or more droplets containing the single-stranded target nucleic acid strands may be transported using droplet operations directly to one or more sections of the array detector 620 dedicated for Roswell molecular wire detection (1602). Notice that in this configuration many steps in the previous assay may be skipped. Furthermore, the previous methods involve indirect detection whereas the Roswell molecular wire method is a direct detection method.

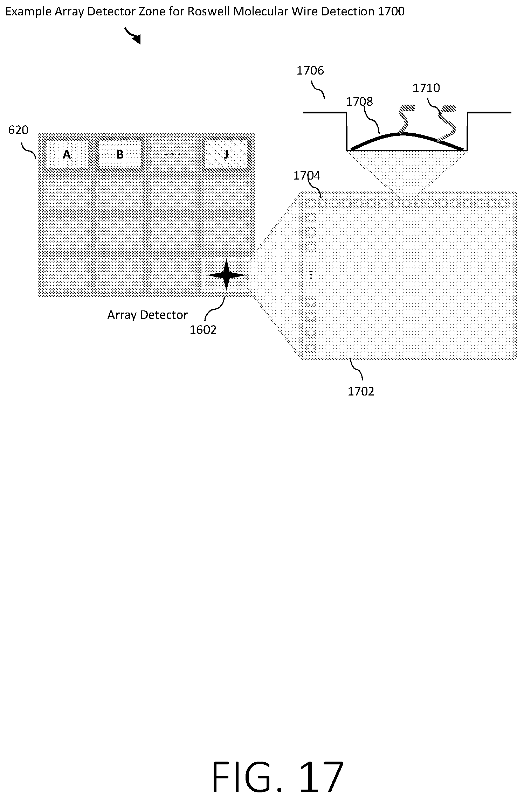

FIG. 17 is a schematic diagram of an example of a process 1700 that uses an array detector zone for Roswell molecular wire detection. For example, process 1700 provides an example of using a section 1602 of array detector 620 that is dedicated for Roswell molecular wire detection 1602. FIG. 17 shows an expanded view of array detector zone 1702 of array detector 620. In this view, the white boxes, 1704, illustrate a few of the hundreds of possible detection features located in an array detector zone. In one configuration, each array detector zone may contain a grid of 100 x 100 detection features 1704. In another configuration, each array detector zone may contain a grid of 10 x 10 reaction wells 1704. An example of individual reaction wells is shown in further detail in 1706. Each detection feature may contain a Roswell molecular wire 1708 shown in black which may be secured using semiconductor technology to each of the wells dedicated to molecular wire detection. In one configuration, the electrical current of the Roswell molecular wires may be collected before the droplets containing methylated nucleic acid reach the molecular wire detection feature. In one configuration, the one or more current readings may be stored on board the cartridge. In another configuration, the Roswell molecular wires may have an attached specific complementary capture probe 1710, designed to capture a nucleic acid target of interest. At this point in the assay, the complementary capture probe may capture both methylated and unmethylated target strands, including any oxidized derivatives as explained above. The capture probe may be a strand of nucleotides specifically synthesized to be complementary to and capture target nucleic acid strands of interest. In one embodiment, the number of nucleotides may range from 1 to 50. In another configuration, the number of nucleotides may range from 17 to 35. In another configuration, the number of nucleotides may be 25 to 30. In another configuration, a target strand of nucleic acid present in the sample may hybridize to the capture probe.

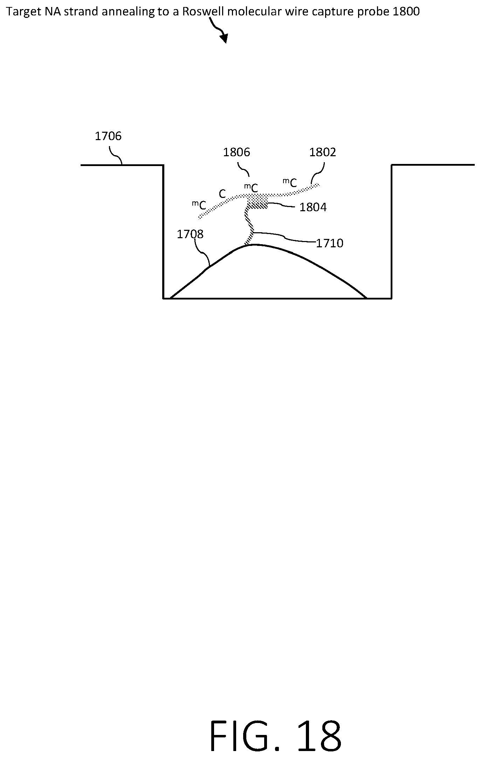

In another configuration, when, for example, droplet operations transport the one or more target nucleic acid containing droplets to the wire-containing detection feature, the droplets may flood their respective detection features. FIG. 18 is a schematic diagram of an example of a process 1800, which is an example of a target DNA strand annealing to a Roswell molecular wire capture probe. More specifically, process 1800 shows the Roswell molecular wire 1708 attached to a detection feature 1706 (illustrated here as a well). Process 1800 is described for a DMF system 400; however, it will be appreciated that other systems, such as robotics system or other microfluidics systems, may be used. In one configuration, the target nucleic acid sample droplets may be incubated with the complementary capture probe 1710-Roswell molecular wires-1708 for 20-40 seconds at 80 °C, as an example only, to ensure target nucleic acid strands 1802 anneal to the complementary capture probe 1710, as shown in FIG. 18. In one configuration, the electrical current of the Roswell molecular wires may be collected after the target nucleic acid containing droplets have incubated with the one or more molecular wires in or on each detection feature. In one configuration, the one or more current readings may be stored on board the cartridge. In this step, both methylated, unmethylated and oxidized derivatives of the target nucleic acid strands may be bound to the complementary capture probe.

In another configuration, droplet operations may be used to transport one droplet of Tris buffer for example over a dried reagent spot containing methylated cytosine binding domain proteins which specifically bind to methylated cytosines. Droplet operations may be used to pulse the buffer over the dried protein reagent spots to ensure complete rehydration and uniform mixing. Once the binding proteins have been completely rehydrated, droplet operations may be used to transport the rehydrated binding proteins to the molecular wire containing wells 1706. When the rehydrated binding protein reaches the wells, the protein may flood the wells containing target nucleic acid bound to complementary capture probes bound to the molecular wire, where the target nucleic acid may be methylated, unmethylated and/or their oxidized derivatives.

Process 1800 shows a target strand of DNA 1802 bound to the capture probe 1710. At the double stranded site where the nucleic acid strand has bound to the capture probe 1804, there is a methylated cytosine 1806, designated by ^{m}C. Additionally, the example strand of target nucleic acid contains two other methylated cytosines (^{m}C) and an unmethylated cytosine (C).

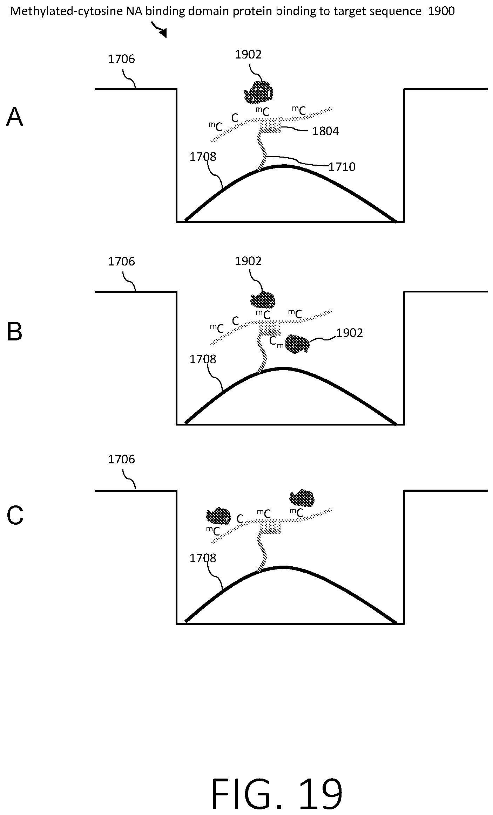

In another configuration, a methylated cytosine nucleic acid binding domain protein may be incubated with the captured target nucleic acid strand. FIG. 19 is a schematic diagram 1900 showing methylated-cytosine NA binding domain protein binding to a target sequence. In panel A, in one configuration, the binding proteins 1902 may selectively bind with the methylated cytosine residues which are bound in a double stranded fashion to the molecular wire capture probe. In one configuration, a capture probe may be synthesized such that only the target strand may contain one or more methylated sites and the capture probe is free of methylated sites. In this configuration, a protein may be used which will bind to a double stranded nucleic acid section where only one strand has a methylated cytosine. This binding may generate a change in electrical signal measured on the molecular wire. In one embodiment, the electrical signal increases. In another configuration, the electrical signal decreases. If there are no methylated cytosines present in the captured nucleic acid strand, then the binding protein may not bind and a change in electrical signal may not occur. In one configuration, the methylated-cytosine nucleic acid binding domain protein may bind to oxidized and unoxidized moieties of methylated cytosines. In another configuration, the methylated cytosine nucleic acid binding domain protein may bind to only the unoxidized methylated cytosines present in the target nucleic acid strand.

In one configuration, shown in FIG. 19 panel B, the capture probe may be synthesized to contain one or more methylated cytosines and to be complementary to a target nucleic acid strand with one or more methylated cytosines. In this configuration, a protein may be used which is only selective to double stranded nucleic acid where each strand contains at least one methylated cytosine. In one configuration, the only double stranded location would be at the target-probe hybridization site.

In another configuration, shown in FIG. 19 panel C, a protein may be used which binds to only single stranded methylated cytosines which would be located distally to the double stranded target-probe hybridized section of nucleic acid. In this configuration, the binding protein may attach to the one or more methylated sites on a single stranded section of the target nucleic acid strand which has not hybridized to a capture probe. This additional binding may generate a measurable change in signal on the Roswell molecular wire. In another configuration, more than one methylated site on the target nucleic acid strand may be detected using the change in electrical signal when more than one protein binds to the more than one methylated nucleotide. In one configuration, a protein may be used which binds to both single stranded and double stranded methylated cytosines. In a configuration of the present disclosure tunable proteins may be used to detect methylated cytosines at the hybridization site and/or methylated cytosines located distally from the hybridization site.

This disclosure presents a direct electrical detection method of methylated nucleic acid, detectable down to a single methylated cytosine nucleotide. In one configuration, the protein may be a binding protein. For example, these proteins may be binding proteins which may bind to methylated cytosines as part of the regulation of nucleic acid transcription.

These configurations are examples of direct detection of methylated nucleic acid.

In a similar configuration, an antibody, such as a monoclonal or polyclonal antibody, for example clone 33D3 (Diagenode, Denville, NJ), may bind to a methylated cytosine nucleotide which may generate a change in the detectable electrical signal at the molecular wire. In another configuration, a protein or antibody which has bound to a methylated cytosine, may be linked to an enzyme which may generate a fluorescent signal, presenting a direct optical method for detecting one or more methylated cytosine nucleotides. In another configuration, a protein or antibody which has bound to a methylated cytosine, may be linked to an enzyme which may generate a chemiluminescent signal, presenting a direct optical method for detecting down to single methylated cytosine nucleotides. In another configuration, a protein or antibody which has bound to a methylated cytosine, may be directly detected using PCR.

Coupling the Roswell molecular wire back-end detector with the currently disclosed front-end digital microfluidics technique for sample processing, handling and bead-based nucleic acid extraction provides a non-invasive direct detection method which does not require any modification of the target nucleic acid. This new technique may detect nucleic acid methylation status down to single methylated nucleotides with minimal upfront biochemical processing.

### 5.6.25 Direct detection of single methylated bases using molecular wires and coded padlock probes

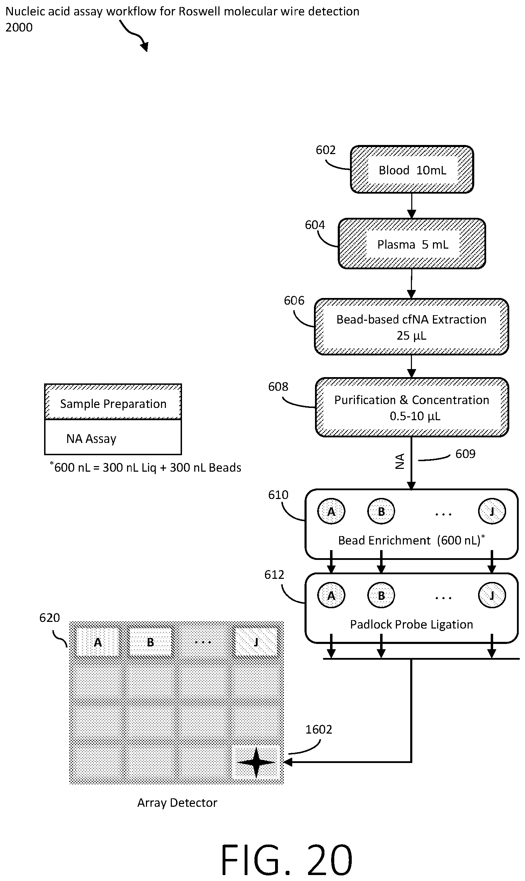

In one configuration, coded padlock probes with Roswell molecular wires may be used to indirectly detect the presence of methylated nucleotides. Several variations of this method are described below. In these configurations, sample preparation may be performed as previously described starting from whole blood sample collection (FIG. 6, step 602) and resulting in nucleic acid material concentrated on magnetic beads (FIG. 6, step 608). The bead pellet may then be resuspended in any convenient liquid volume, for example 1 µL, 2 µL, 5 µL, 10 µL, 25 µL, for direct transfer to the microfluidic cartridge. In one configuration, the bead pellet may be resuspended in a volume of more than 25 µL. In one configuration, the bead pellet may be resuspended in a volume between 1 and 25 µL. In one configuration, the bead pellet may be resuspended in a volume less than 1 µL. As described above, the beads may then be concentrated within a single unit volume droplet (e.g., 1 nL, 10 nL, 100 nL, 1000 nL, 10000 nL) and material may then be eluted from the beads using the Qiagen bead elution buffer reagent. Droplet operations may be used to transport the sample to a coded padlock probe ligation step, which will be described below. After the coded padlock probe ligation step the sample contents may proceed directly to one or more sections of the array detector 620 dedicated for Roswell molecular wire detection 1502, as shown, for example, in FIG. 20.

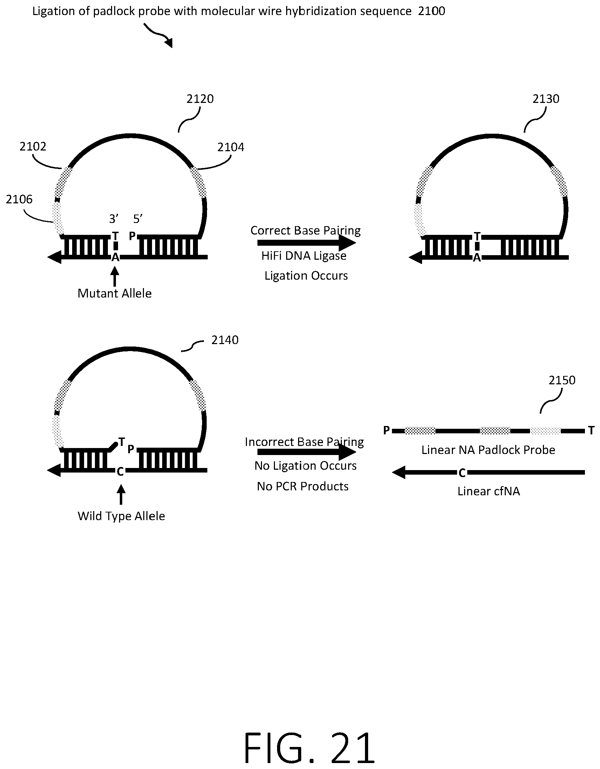

In one configuration, illustrated in FIG. 21, the coded padlock probes 2120 may include forward and reverse universal amplification primer sequences, shown in medium gray (2102, 2104 respectively). These universal amplification sequences may be the same as described above. In one configuration, the coded padlock probes may include a molecular wire hybridization sequence 2106 shown in light gray. This sequence may be used to hybridize to a complementary capture probe attached to the Roswell molecular wire. If the coded padlock probe encounters a target nucleic acid sequence having the correct base pairing, then HiFi DNA Ligase may seal the nick between the ends of the probe and circularize the coded padlock probe 2130. If the coded padlock probe incorrectly pairs with a wildtype allele strand 2140, no ligation occurs and only linear products 2150 may remain.

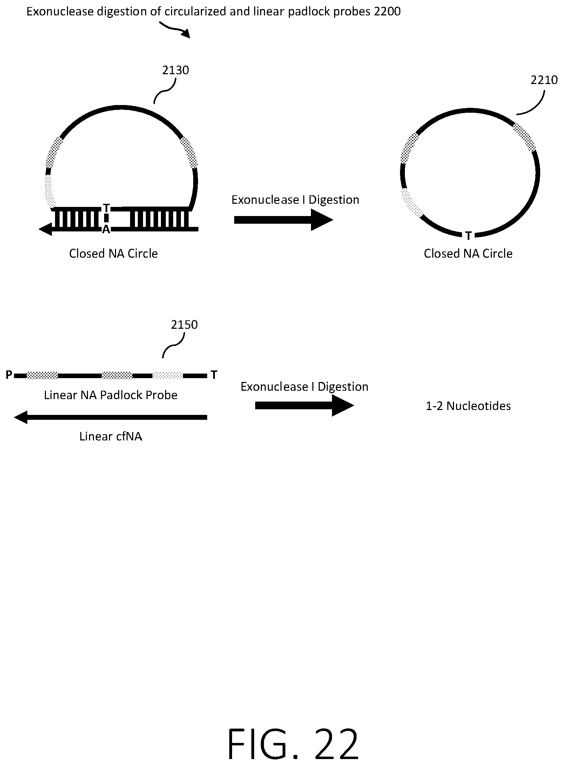

In another configuration, as shown in FIG. 22, the circularized coded padlock probe may proceed to the exonuclease digestion step, as described above. This step removes the target strand from the circularized coded padlock probe leaving a closed nucleic acid circle 2210 and removes any uncircularized material. Any uncircularized material 2150 present during the exonuclease digestion step may be broken into nucleotides of 1-2 bases in length.

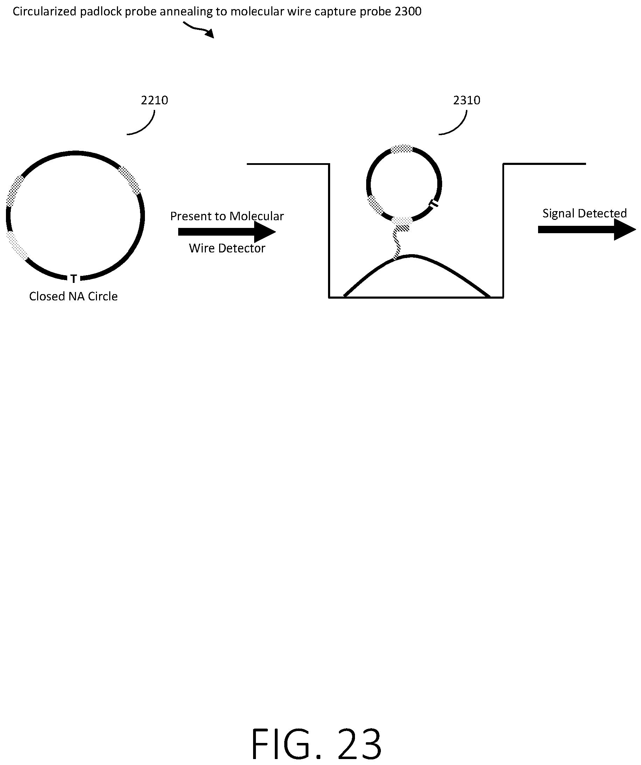

In one configuration, droplets containing coded padlock probes with a molecular wire hybridization sequence may be transported using, for example, droplet operations to the array of detection features containing the molecular wires. FIG. 23 is a illustrates a process 2300 of annealing a circularized coded padlock probe to a molecular wire capture probe. After flooding the molecular wire containing wells, the coded padlock probe containing droplets may incubate with the molecular wires. As shown in FIG. 23, the molecular wire hybridization sequence may hybridize with the molecular wire's capture probe 2310. Upon hybridization, a change in electrical signal may be measured. In one configuration the electrical signal increases upon hybridization with the coded padlock probe. In another embodiment the electrical signal decreases upon hybridization with the coded padlock probe. In another configuration, this method may be used to detect SNPs, insertions/deletions and fusions, for example.

### 5.6.26 Direct detection of methylated nucleic acid using protein probes for methylation-specific inhibition of ligation

Methylation-specific inhibition of ligation is a variation of a direct detection of single methylated bases assay by coupling coded padlock probes with Roswell molecular wires. In this method, sample preparation is performed as previously described for FIG. 6 starting from a 10 mL whole blood sample (step 602), separating and collecting the plasma fraction (step 604) and resulting in nucleic acid material concentrated on magnetic beads (step 606). The bead pellet may be resuspended in any convenient liquid volume for direct transfer to the microfluidic cartridge (e.g., 1 µL, 2 µL, 5 µL, 10 µL, 25 µL). The beads may then be concentrated (step 608) within a single unit volume droplet (e.g., 1 nL, 10 nL, 100 nL, 1000 nL, 10000 nL) and material may be eluted from the beads, for example using the Qiagen bead elution buffer.

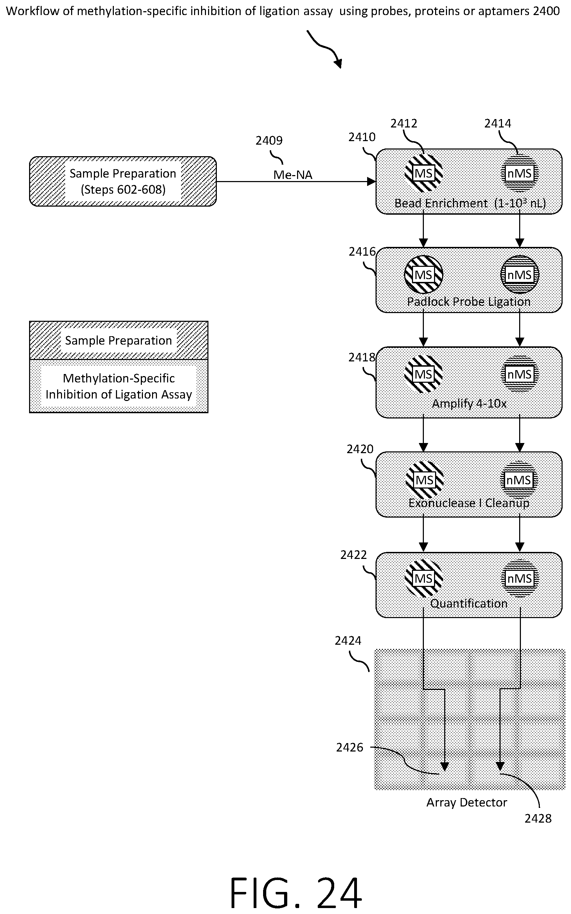

FIG. 24 is a flow diagram of an example of a process 2400 and showing a methylation-specific inhibition of ligation assay workflow using probes, proteins, and/or aptamers. Process 2400 is described for a DMF system 400; however, it will be appreciated that other systems, such as robotics system or other microfluidics systems, may be used.

In step 2410, a methylated nucleic acid sample is divided into two parallel stream for analysis of methylated and unmethylated targets. For example, droplet operations may be used to divide the sample into two parallel streams for bead enrichment, one for methylation sensitive or methylation specific analysis (MS) and one for non-methylation sensitive or specific analysis (nMS). The MS sample 2412 may be used to determine the methylation status of certain loci within the nucleic acid whereas the nMS sample 2414 may serve as a baseline reference enabling the fraction or percent of methylation to be determined at each locus of interest (e.g., 10%, 20%, 50%, 80%, 90%). In some configurations, the sample may be split evenly between MS and nMS and in some configurations the sample may be unevenly divided between the two types of analysis.

In this configuration, magnetic beads may be used as described above in the direct and indirect capture methods for reducing the sample volume producing a single droplet having a volume of approximately 1 to 1000 nL.

In step 2416, a ligation reaction may be performed using coded padlock probes. The probes may be circularized (i.e., ligated together) if a target matching the probe sequence is present in the sample (also as previously described). The MS sample 2412 may undergo a variation of this reaction in which the ligation efficiency is affected by the methylation status of the locus being interrogated. Methylation specificity may be provided by several different methods including 1) methylation-specific inhibition of ligation, 2) methylation-specific aptamers, 3) methylation-specific restriction enzymes or 4) methylation-specific immuno-precipitation.

### 5.6.27 Direct detection of methylated nucleic acid using methylation-specific inhibition of ligation

Methylation specific inhibition of ligation is based on the use of a probe or protein for example which binds specifically to methylated nucleic acid groups thereby interfering with the activity of the ligase and preventing the circularization of the coded padlock probes. For example, if an antibody or methyl CpG binding domain (MBD) or the Uhrf1pr domain is bound to the methyl-C targeted by the coded padlock probe it effectively prevents ligation of the probe. When compared to the amount of probe ligated in the reference sample the relative fraction of methylation at the specific locus can be determined.

In one configuration, the MS sample 2412 may be transported using droplet operations to a dried reagent spot containing for example monoclonal antibodies, polyclonal antibodies, nanobodies, MBD or Uhrf1pr proteins. Droplet operations may be used to pulse the MS sample 2412 with the dried reagent spot to ensure complete rehydration of the protein, producing a MS-Protein Droplet. In this droplet the protein or antibody may bind to sequences containing a methylated nucleotide. In another configuration, the nMS sample 2414 does not rehydrate and react with methyl binding domain proteins or antibodies selective to a methylated nucleotide.

In one configuration, the MS-Protein droplet may be transported using droplet operations to a dried reagent spot containing HiFi DNA Ligase and coded padlock probes complementary to the target sequence of interest, where the target sequence may or may not be methylated. As in examples above, the coded padlock probes may contain sequences which may not be extended (non-extendable sequence 914) which allow for linear double-stranded PCR products. Droplet operations may be used to pulse the droplet with the dried reagent spot to ensure complete rehydration, producing a MS-protein-probe droplet . In another configuration, the nMS sample 2414 may be transported using droplet operations to a dried reagent spot containing HiFi DNA Ligase and coded padlock probes complementary to the target sequence of interest, where the target sequence may or may not be methylated. Droplet operations may be used to pulse the droplet with the dried reagent spot to ensure complete rehydration, producing a nMS-probe droplet .

In one configuration, the rehydrated coded padlock probes may anneal to the target sequence of interest and ligation may occur if the target sequence is not methylated. In another configuration, if the target sequence is methylated, the proteins or antibodies bind to the one or more methylated nucleotides in the sample which blocks coded padlock probes from annealing and then ligating with the target sequence.

In step 2418, the MS-protein-probe droplet and the nMS-probe droplet may be transported to an appropriate zone of the PCB cartridge for a universal amplification step as described above (step 614). For example, in this process, the MS-protein-probe droplet and the nMS-probe droplet may each be transported to one of two dried reagent spots containing dried dNTPs, generic PCR primers which complement the universal, generic PCR primers (generic forward and reverse primer sequences 910 and 912) and DNA polymerase. Droplet operations may be used to pulse the MS-protein-probe droplet and the nMS-probe droplet over the dried reagent spots to ensure complete rehydration and uniform mixing, producing a pre-amplification MS droplet and a pre-amplification nMS droplet. After rehydration and mixing, the pre-amplification MS droplet and the pre-amplification nMS droplet may be heated in a PCR initialization step to 94-96°C for 2-10 minutes to activate the DNA polymerase and to denature any contaminates in the mixture. To begin the denaturation step of PCR cycle 1, the pre-amplification MS droplet and the pre-amplification MS droplet may be heated to 94-98°C for 20-30 seconds to break the hydrogen bonds between the coded padlock probe and the target sequence. This denatures the double-stranded nucleic acid sequence, separates the target nucleic acid sequence from the coded padlock probe and produces single-stranded products. The target nucleic acid sequence is a linear single-stranded product, while the coded padlock probe is a single-stranded circularized sequence. At this stage the rehydrated primers bind to the complementary sequences (generic forward and reverse primer sequences 910 and 912) of the coded padlock probe. The reaction temperature of the pre-amplification MS droplet and the pre-amplification nMS droplet may be lowered to 50-65°C for 20-40 seconds to allow the primers to anneal to the circularized coded padlock probes. After the primers hybridize to the forward and reverse universal amplification sequences on the coded padlock probe, the rehydrated DNA polymerase incorporates dNTPs onto the primers in the elongation step at 72-78°C for Taq DNA polymerase for approximately 2 minutes. The coded padlock probes may incorporate amplification blocking sequences as described above or the amplification may involve rolling circle amplification. In one configuration, this PCR amplification process may be repeated for 3 or more cycles to generate enough target nucleic acid to be detected. In other configurations, generic PCR amplification may proceed for 20 or more cycles or 40 or more cycles. After for example 4-10 amplification cycles, the pre-amplification MS droplet and the pre-amplification nMS droplet may be held at 72-78°C for 5-15 minutes to ensure complete strand extension. The universal amplification step produces linear duplex nucleic acid copies (see FIG. 10) containing the generic (universal) primer sequences 910 and 912 and the combinatorial forward and reverse PCR tag sequences 906 and 908. The resulting droplets are referred to as amplified MS droplet and amplified nMS droplet. This step produces excess nucleic acid in each droplet relative to that required to populate the detector array. The exact amount of nucleic acid in each droplet will be determined in a subsequent step, discussed below, and this nucleic acid will be diluted to a concentration range required by the fluorescence detector. Only the unmethylated target sequences in the MS analysis droplet may be amplified because the methylated sequences were blocked from coded padlock probe ligation by the methylation specific proteins or antibodies. Furthermore, in the nMS analysis droplet all or substantially all of the target sequences may be amplified because of the absence of any methylated specific proteins or antibodies.

The amount of PCR product generated may be a function of how many coded padlock probes hybridize to the same target sequence. As described above it may be desirable to tune the signal at the detector if it is known in advance that a target allele is over-represented in the sample to prevent detector saturation.

After amplification of the coded padlock probes, linear duplex PCR products may be formed (see FIG. 10, panel 1030) with universal primers and the one or more pairs of unique PCR tag sequences. The same forward and reverse universal primers may be used on every coded padlock probe in the assay to prevent bias, thereby ensuring all targets are amplified uniformly and to the same degree. PCR products may only be produced upon successful ligation by the HiFi DNA Ligase.

As shown in FIG. 9, panel 9300, when the coded padlock probe anneals to a wildtype allele, the ligase may not be able to seal the buckled nick due to the incorrect base pairing and therefore no PCR products may be produced. This process may be dependent on the fidelity of the ligase enzyme connecting only the correctly paired probe and target molecules. A high-fidelity ligase, commercially available may be selected for this reaction. In the event the ligase incorrectly seals the nick of a wildtype allele pair, a cleanup step using the RNAse H2 protocol may be used as described above to prevent incorrect amplification in step 2418.

In step 2420, an exonuclease cleanup process is performed to remove any unreacted single-stranded nucleic acid. For example, an exonuclease 1 enzymatic reaction may be performed as describe above in step 616 of assay workflow 600.

For example, each of the amplified MS droplet and amplified nMS droplet may be transported using droplet operations to a dried exonuclease I spot for sample cleanup. In this step, droplet operations may be used to pulse the amplified MS droplet and amplified nMS droplet with the dried exonuclease I spot to ensure rehydration and mixing, producing a digested MS droplet and a digested nMS droplet.

In one configuration, each of the digested MS droplet and the digested nMS droplet may be heated at 80°C for 1 minute to fully inactivate the exonuclease I enzyme, producing an MS cleanup droplet and an nMS cleanup droplet.

In step 2422, a quantification process is performed. For example, the amount of nucleic acid present in each MS cleanup droplet and nMS cleanup droplet may be quantified by incorporating a fluorescent dye into the dsNA strands and measuring the change in fluorescence as described above in step 618 of assay workflow 600. Quantification may be used to determine whether dilution of a droplet is required to produce the desired nucleic acid concentration to send to the detector. If it is determined that dilution of the MS cleanup droplet and/or nMS cleanup droplet is required, droplet operations may be used to dispense the appropriate volume of buffer from a buffer reservoir and transport this volume of buffer needed to appropriately dilute the MS cleanup droplet and/or the nMS cleanup droplet. Droplet operations may be used to pulse the buffer with the MS cleanup droplet and nMS cleanup droplet to ensure appropriate dilution of the target nucleic acid in the sample.

For example, the MS cleanup droplet and/or the nMS cleanup droplet may be transported using droplet operations to a quantification section of the PCB cartridge. Droplet operations may be used to separate 1 nL from each of the cleanup droplets and transport each droplet separately to a dried reagent spot containing SYBR Green (or SYBR Gold) master mix. Droplet operations may be used to pulse the MS and nMS cleanup droplets over the dried dye spot to ensure complete rehydration and uniform mixing of the SYBR Green (SYBR Gold) intercalating dye, producing a MS quantification droplet and a nMS quantification droplet. Immediately after the MS and nMS cleanup droplets come in contact with the dried SYBR Green (SYBR Gold) master mix reagent spot, the fluorescence of each drop may be captured using an on-cartridge CCD camera at an example wavelength of 520 nm for 5 minutes.

In one configuration, the fluorescence of each of the MS and nMS quantification droplets may be compared to an embedded, laboratory-generated, standard curve of PCR cycles as a function of fluorescence for varying nucleic acid concentrations as described in step 618 of assay workflow 600.

After the total amount of nucleic acid in each of the MS and nMS quantification droplets is determined, a calculation may be performed by the processor to determine the exact dilution necessary to ensure the nucleic acid concentration in each droplet is within the range dictated by the fluorescence detector.

In another configuration, 3 or 4 or 5 or more than 5 nucleic acid standards of known concentration may be spotted on the cartridge and may be used to generate a standard curve in-situ as described in step 618 of assay workflow 600. The amount of nucleic acid in each of the MS and nMS quantification droplets may be compared to the standard curve to determine the amount of nucleic acid in each MS and nMS quantification droplet.

In another configuration, one concentrated standard nucleic acid spot may be dried on the cartridge, rehydrated and then diluted into 3, or 4, or 5, or more than 5 standard drops that may be used in a quantification process to generate a standard curve of nucleic acid concentration vs. fluorescence signal as describe in step 618 of assay workflow 600. The amount of nucleic acid in each of the MS and nMS quantification droplets may be compared to the standard curve to determine the amount of nucleic acid in each MS and nMS quantification droplet.

In this configuration, droplet operations may be used to dispense one buffer droplet from the on-board buffer reservoir and then transport the buffer droplet to merge with the dried, concentrated nucleic acid spot. Droplet operations may be used to pulse the buffer droplet over the concentrated nucleic acid standard spot to ensure complete rehydration and unform mixing. In another embodiment, droplet operations may be used to separate the rehydrated concentrated nucleic acid standard droplet into 3, or 4, or 5, or more than 5 droplets. In another configuration, Droplet operations may be used to transport for example Tris buffer from the on-board buffer reservoir to the first rehydrated, concentrated nucleic acid spot. In another configuration, droplet operations may be used to transport buffer droplets to each of the 3, or 4, or 5, or more than 5 nucleic acid standard droplets to dilute them to the appropriate concentration. In another configuration, one droplet of buffer may be dispensed from the on-board buffer reservoir and transported using droplet operations to merge with one dried spot of SYBR Green (SYBR Gold) intercalating dye. Droplet operations may be used to pulse the one droplet of buffer over the dried dye spot to ensure complete rehydration and uniform mixing.

Droplet operations may be used to split the one drop of dyed buffer into the same number droplets as the number of nucleic acid standard droplets. In one configuration, the dyed buffer may be split into 3, or 4, or 5, or more than 5 droplets.

Droplet operations may be used to transport each of the dyed buffer droplets to merge with each of the nucleic acid standard droplets. Droplet operations may be used to pulse the merged dyed buffer and nucleic acid standard droplets to ensure uniform mixing. Once the nucleic acid has incorporated the intercalating dye, fluorescence may occur. In one configuration, a standard curve may be generated of nucleic acid concentration vs. fluorescence signal. In one configuration, the amount of nucleic acid in each of the MS and nMS quantification droplets (step 2422) may be compared to the standard curve to determine the amount of nucleic acid in each MS and nMS quantification droplet.

After the appropriate concentration of nucleic acid in each of the MS and nMS quantification droplets is obtained, a calculation may be performed by an on-board processor to determine the exact dilution necessary to ensure the nucleic acid concentration in each droplet may be within the range dictated by the fluorescence detector. In one configuration, if the fluorescence detector determines an appropriate amount of nucleic acid has been created in the amplification step 2418, droplet operations may be used to transport the MS and nMS quantification droplets to an appropriate zone of the array detector. In one configuration of the quantification step, if the processor determines there is insufficient nucleic acid present in any of the MS or nMS quantification droplets, an error flag may be called which stops the entire assay and returns an error message for the end user.

In one configuration, if the fluorescence detector determines there is excess nucleic acid present in the sample such that the droplet would saturate the array detector, a calculation may be performed by the instrument to determine the appropriate dilution needed for each droplet. Amplifying the sample nucleic acid to more than the amount needed by the detector may be a preferred configuration. Based on the dilution factor determined in the quantification step 2422, a calculation may be performed to determine what volume of for example Tris buffer may be needed to appropriately dilute each of the quantification droplets. In one configuration, droplet operations may be used to transport the appropriate volume of for example Tris buffer to each quantification droplet. Droplet operations may be used to pulse each of the quantification droplets with its respective dilution buffer droplet to ensure uniform mixing and dilution, producing a MS diluted droplet and an nMS diluted droplet.

In step 2422, droplet operations may be used to separate a small volume approximately 1 to 10 pL from each MS and nMS diluted droplet for analysis, producing a MS picoscale droplet and an nMS picoscale driplet. Droplet operations may be used to transport each picoscale droplet to a dried reagent spot containing all the requisite reagents, except the PCR primers and the fluorescent intercalating dye, required for PCR-initiated fluorescence detection on the array detector 2424. These reagents may, for example, include TaqPath ProAmp master mix, Taq DNA polymerase, each of the four dNTPs, stabilizers, buffers, enzymes such as RNAse H2 enzyme, and MgCl₂. Droplet operations may be used to pulse each of the picoscale droplet to ensure complete rehydration and uniform mixing of the reagents with the nucleic acid in the sample. This step produces a MS detection droplet and an nMS detection droplet. In an alternative configuration, the MS picoscale droplet and nMS picoscale droplet may be transported to an appropriate section of the array detector to be detected using a Roswell molecular wire analysis. A detection method using a Roswell molecular wire analysis is described hereinbelow.

In one configuration, standard engineering protocols may be used to transfer each of the MS and nMS detection droplets to the X-1 Active Matrix Digital Microfluidics (AM-DMF) CMOS chip as described above in step 618 of assay workflow 600. In one configuration the CMOS detector chip is fabricated using glass. In another configuration, the CMOS detector chip is fabricated using silicon. A PCB can accommodate a minimum drop size in the nanoliter volume range. However, below droplet volumes of approximately 100 nL, PCBs reach their manufacturing limitations and cannot be used for smaller volumes. A silicon or glass CMOS chip can effectively process picoliter sized droplet volumes. In order to increase the signal to noise ratio it may be important to minimize the sample volume and maximize the sample concentration as discussed previously. Therefore, one configuration may include transferring the MS and nMS Detection Droplets to the CMOS chip using droplet operations for the final detection steps on the CMOS array detector 2424. CMOS array detector 2424 is essentially the same as CMOS array detector 620, which is described in more detail hereinabove with reference to FIG. 6 and FIG. 7, array detector 708.

After transfer to the CMOS chip, droplet operations may be used to transport each of the MS and nMS detection droplets to a specific zone of the array detector 2424. FIG. 7 provides a more detailed schematic of the design of a detection zone in the CMOS array detector. For example, droplet operations may be used to transport each MS and nMS detection droplet to the appropriate detector zone, shown in FIG. 24 as zones 2426 and 2428, respectively. When the droplet lands in the detector zone, it may flood the detection features located in that zone. Droplet operations may be used to pulse the liquid in each detector zone and in each detection feature to ensure complete rehydration and uniform mixing of each MS and nMS detection droplet with the dried forward and reverse primers and the intercalating dye pre-spotted in or on each detection feature. Immediately upon rehydration and mixing with the nucleic acid containing droplets, the intercalating dye may begin to fluoresce. This fluorescence may be used to detect the presence of mutant allele targets and is discussed further below.

In one configuration, a unique PCR tag may be used for each separate target sought as described hereinabove. In one configuration, each target may be detected by a unique combination of two primers reused over all zones which significantly reduces the number of primers needed. The unique combinatorial PCR tags (906 and 908) on the coded padlock probes are shown in light gray in FIG. 9. The combination of forward and reverse primers which hybridize to the unique PCR tags may be pre-spotted in each detection feature.

During the manufacture of the CMOS chip, an array of forward and reverse PCR primers and an intercalating dye such as SYBR Green, SYBR Gold or Eva Green may be spotted in each detection feature and dried using for example forced air as described above with reference to FIG. 11. In each zone, an array of fifteen, for example, forward PCR primers T₁F through T₁₅F may be spotted in or on each detection feature of the rows and an array of fifteen, for example, reverse primers T₁R through T₁₅R may be spotted in or on each detection feature of the columns. Therefore, each detection feature may contain a unique combination of at least two dried primers, at least one forward and at least one reverse primer. Any number of zones may be dedicated to this analysis method which serves to increase the number of targets detected.

In one configuration, the exact location of the combinatorial primers is known as described above with reference to FIG. 11. If the matching target arrives from a MS or nMS detection droplet and floods the corresponding detection feature, PCR amplification may occur. After a sufficient quantity of target molecules are generated and after a sufficient quantity of intercalating dye is incorporated a fluorescent signal may occur. The result may be a binary detection output signal. While T₁F+T₁R may be used in the upper left corner, for example, of each zone, a fluorescence signal from the upper left corner of different zones may indicate the presence of a different target. In one embodiment, one or more wells in each zone contain unique PCR tag sequences which may be used to detect the internal positive and negative control sequences.

In one configuration, droplet operations may be used to transport a droplet of buffer to a detection feature containing dried PCR primers and an intercalating dye. A fluorescence measurement may be taken at 520 nm for example and collected for approximately 5 minutes. The background fluorescence signal may be collected and stored on the cartridge.

In one configuration, droplet operations may be used to transport each of the MS and nMS detection droplets to the correct section of the array detector zones. Droplet operations may then be used to pulse each of the MS and nMS detection droplets to rehydrate and uniformly mix the combinatorial PCR primers and the intercalating dye with the nucleic acid sample. Immediately upon rehydration and mixing with the nucleic acid containing droplets, the intercalating dye may begin to fluoresce in the range of the background noise.

In one configuration, PCR amplification may be performed to increase the amount of nucleic acid in each droplet until a fluorescence signal is obtained beyond that of the background noise. In this configuration, the wells in each zone may be heated to 95°C for 5 minutes to activate the nucleic acid polymerase and to denature any contaminants in the flooded wells. In one configuration, the zones may be heated to 94-98°C for 20-30 seconds to convert the double-stranded nucleic acid present in the droplet to single-stranded nucleic acid. In an configuration, the rehydrated primers pre-spotted in the detection feature may anneal to the complementary sequence of the PCR tags in the original coded padlock probes. In one configuration, the temperature of the zones may be reduced to 50-65°C to allow for optimal primer annealing. In another configuration, the temperature of each zone may be increased to 72-78°C to allow optimal template extension by Taq polymerase. This step may proceed for a certain length of time. In one configuration, 15-20 PCR cycles may be performed. Subsequently a fluorescence measurement may be collected using a standard fluorescence detector. In another configuration, PCR amplification may be allowed to continue for 2 additional cycles before another fluorescence measurement is collected. In one configuration, a fluorescence measurement may be collected after cycle 32 for example. In one configuration, a fluorescence measurement may be collected after 45 cycles. In one configuration, a fluorescence measurement may be collected after every PCR cycle. In one configuration, a fluorescence measurement may be collected after every third PCR cycle. The fluorescence signal as a function of PCR cycle number may be stored on cartridge. The on-cartridge processor may include an algorithm which determines when the fluorescence signal increases above a predetermined threshold indicating a positive result for that target. In one configuration, a fluorescence map may be made corresponding to specifically mapped detection features and which may be used to detect the presence of mutant target alleles. Since a precise map of each zone may be created at the time of reagent spotting, one may precisely determine which target mutant allele is fluorescing.

In one configuration, a fluorescence endpoint assay may be conducted where PCR amplification may be allowed to proceed for 45 cycles. In this configuration, a fluorescence signal may be collected after the 45 cycles has completed.

The signal from the MS detection droplet where methylated sequences were inhibited from ligating may be compared to that of the nMS detection droplet where the target sequences were allowed to ligate regardless of methylation status. Comparing these two signals provides a percent measure of methylation status of the target sequence of interest.

As described above, the MS and nMS picoscale droplets were combined with reagents necessary for PCR based detection. In another configuration, the MS and nMS picoscale droplets may instead be transported to an appropriate zone of the array detector for detection by Roswell molecular wires. This configuration may proceed as described above.

For this analysis method, the original complementary coded padlock probes used in step 2416, may contain a Roswell molecular wire hybridization sequence 2106 as shown in FIG. 21 instead of or in addition to the universal amplification sequences and in addition to or instead of the PCR tag sequences (906, 908) shown in FIG. 9.

In one configuration, droplets containing coded padlock probes with a molecular wire hybridization sequence may be transported using droplet operations to the array of detection features containing the molecular wires (see FIG. 17). After flooding the molecular wire containing wells, the coded padlock probe containing droplets may incubate with the molecular wires. As shown in FIG. 23, the molecular wire hybridization sequence may hybridize with the molecular wires capture probe. Upon hybridization, a change in electrical signal may be measured. In one configuration the electrical signal increases upon hybridization with the coded padlock probe. In another configuration the electrical signal decreases upon hybridization with the coded padlock probe. The change in electrical signal between the MS picoscale droplet and the nMS picoscale droplet may be used to determine the percent methylation of the target sequence.

### 5.6.28 Direct detection of methylated nucleic acid using aptamers for methylation-specific inhibition of ligation

The disclosure provides a method using methylation-specific aptamers for the indirect detection of 5-methylcytosine (5-mC) found in CpG dinucleotide motifs in cell free nucleic acid fragments. Aptamers may be created that bind specifically to 5-mC and thus may be used to elucidate the state of methylation of CpGs. The binding specificity is independent of the 5'- and 3'-NA sequence flanking the CpG dinucleotide with the exception of when CpGs are in close proximity with one another. Because of their smaller footprint relative to antibodies and methyl domain binding proteins, aptamers may be used to improve the resolution of detecting clusters of methylated sites in close proximity. Aptamers can be created to bind to a single 5-mCpG or to two or more adjacent 5-mCpGs or to multiple 5-mCpG that have one or more nucleotides inserted between the 5-mCpGs. A collection of aptamers can be employed to differentiate methylated and non-methylated CpGs that are found alone or near other CpGs.

The aptamers may be used in combination with coded padlock probes or restriction endonucleases. Padlock ligation probes may be designed to hybridize to the nucleic acid sequences flanking the methylated or unmethylated CpG. In combination with aptamers, the presence or absence of coded padlock probe hybridization may provide a mechanism to identify the methylation status of specific 5-mCs. If an aptamer is bound to a 5-mC, it may prevent the annealing of the coded padlock probe to that region. If the coded padlock probe is unable to anneal to the target nucleic acid sequence, ligation of the probe will not occur leading to no signal for that specific 5-mC. If the CpG dinucleotide is unmethylated, the coded padlock probe can hybridize to the nucleic acid target sequence and ligation can occur producing a positive signal. Methylation-specific aptamers may be used on a digital microfluidics device to detect proteins using for example PCR or a Roswell molecular wire method as described above.

Cell free nucleic acid may be purified from plasma and potentially methylated nucleic acid sequences of interest can be enriched or sub-fractionated with the use of complementary oligonucleotides and magnetic beads. The methylated nucleic acid sample may then be divided into two samples. A first sample may be for the detection and quantitation of the target nucleic acid independent of methylation status. This may be accomplished by annealing and ligation of nucleic acid target specific coded padlock probes as described in earlier disclosures. A second sample may be incubated with 5-mC binding aptamers which may bind to all or substantially all of the 5-mC bases in the sample. A coded padlock probe specific for the target sequence can be added to the sample. If the aptamer has bound to the 5-mC, this may disrupt the annealing of the methylated target nucleic acid to the coded padlock probe which may block coded padlock probe ligation which may prevent a signal from being generated downstream. The difference in signal between the sample treated with aptamers and the signal from the sample not treated with aptamers may be a measure of the extent of methylation at the specific cytosine residue.

This method is similar to that described above for the direct detection of methylated nucleic acid using protein probes for methylation-specific inhibition of ligation. The steps described above with reference to FIG. 24 will be used for both methods involving methylation-specific inhibition of ligation. In one configuration, sample preparation may be carried out using the methods described above (see FIG. 6, steps 602 - 608) where cell-free nucleic acid is separated from plasma and extracted, purified and concentrated using a bead-based method. In another configuration, droplet operations may be used to split the sample into two parallel streams for bead enrichment (step 2410 of FIG. 24), one for methylation sensitive or methylation specific analysis (MS) and one for non-methylation sensitive or specific analysis (nMS), producing an MS droplet 2412 and an nMS droplet 2414, respectively. The MS droplet 2412 may be used to determine the methylation status of certain loci within the nucleic acid whereas the nMS droplet 2414 may be used for the detection and quantitation of the target nucleic acid independent of the methylation status. The nMS sample may serve as a baseline reference enabling the fraction or percent of methylation to be determined at each locus of interest (e.g., 10%, 20%, 50%, 80%, 90%). In some configurations the sample may be split evenly between MS and nMS droplets and in some configurations the sample may be unevenly divided between the two types of analysis.

In this configuration, magnetic beads coupled with droplet operations may be used as described above for reducing the sample volume producing two single droplets each having a volume of approximately 1 to 1000 nL (2310).

In one configuration, the MS droplet 2412 may be transported using droplet operations to a dried reagent spot containing for example 5-mC aptamers. Droplet operations may be used to pulse the MS droplet 2412 with the dried reagent spot to ensure complete rehydration of the aptamer, producing an MS-aptamer droplet. In this droplet the singular or multiple different aptamer types may bind to sequences containing a methylated cytosine nucleotide. In one configuration, the nMS sample 2414 may not interact with 5-mC aptamers since it may be used as a reference sample for comparison. At this step of the assay an MS-aptamer droplet and an nMS droplet may have been produced.

In one configuration, both the MS-aptamer droplet and the nMS droplet may be transported using droplet operations to a dried reagent spot containing HiFi DNA Ligase and coded padlock probes complementary to the target sequence of interest, where the target sequence may or may not be methylated. As in examples above, the coded padlock probes may contain sequences which may not be extended (e.g., non-extendable sequence 914) which allow for linear double stranded PCR products. Droplet operations may be used to pulse each of the MS-aptamer droplet and the nMS droplet with each of dried reagent spots to ensure complete rehydration, producing an MS-aptamer-probe droplet and an nMS-probe droplet (step 2416).

In one configuration, the rehydrated coded padlock probes may anneal to the target sequence of interest and ligation may occur if the target sequence is not methylated. In another configuration, if the target sequence is methylated, the aptamers bind to one or more methylated nucleotides in the sample which blocks the coded padlock probe from annealing to the target sequence. In this embodiment, only the nMS-probe droplet (step 2416) may have circularized coded padlock probes.

In one configuration, the MS-aptamer-probe droplet and the nMS-probe droplet may be transported to an appropriate zone of the chip for a universal amplification step 2418 as described above (and in step 614 of FIG. 6). In this process, the MS-aptamer-probe droplet and the nMS-probe droplet may each be transported to one of two dried reagent spots containing dried dNTPs, generic PCR primers which complement the universal, generic PCR primers shown in medium gray on the complementary coded padlock probes (910 and 912 of FIG. 9) and DNA polymerase. Droplet operations may be used to pulse the MS-aptamer-probe droplet and the nMS-probe droplet over the dried reagent spots to ensure complete rehydration and uniform mixing, producing a pre-amplification MS droplet and a pre-amplification nMS droplet (step 2418). After rehydration and mixing, standard PCR protocols may be employed for 3 to 40 or more cycles to separate the coded padlock probes from the target sequences and then amplify the coded padlock probes to a point of excess concentration as described above.

The universal amplification step 2418 produces linear duplex nucleic acid copies (see FIG. 10, panel 103030) containing the universal primers (910 and 912) shown in medium gray and the combinatorial forward and reverse PCR detection primers (906 and 908) shown in light gray. The resulting droplets are referred to as amplified MS droplet and amplified nMS droplet (step 2418, panel 1030 of FIG. 10). This step may produce excess nucleic acid in each droplet relative to that required to populate the detector array. The exact amount of nucleic acid in each droplet will be determined in a subsequent step, discussed below, and this nucleic acid will be diluted to an appropriate concentration range as required by the fluorescence detector. To be clear, only the unmethylated target sequences in the MS analysis droplet may be amplified because the methylated sequences were blocked from coded padlock probe ligation by the methylation specific aptamers. Furthermore, in the nMS analysis droplet all or substantially all of the target sequences may be amplified because of the absence of any methylation-specific aptamers.

The amount of PCR product generated may be a function of how many coded padlock probes hybridize to the same target sequence. As described above, it may be desirable to tune the signal at the detector if it is known in advance that a target allele is over-represented in the sample to prevent detector saturation.

After amplification of the coded padlock probes, linear duplex PCR products may be formed (see FIG. 10, panel 1030). The same forward and reverse universal primers (medium gray) may be used on every coded padlock probe in the assay to prevent bias, thereby ensuring all targets are amplified uniformly & to the same degree. PCR products may only be produced upon successful ligation by the HiFi DNA Ligase. If an aptamer has bound to a 5-mC loci, this may disrupt the coded padlock probe from binding to the target sequence.

In one configuration, any coded padlock probes which have hybridized with a target sequence may be circularized. After amplification (step 2418) they may be linear, double-stranded nucleic acid sequences (see FIG. 10, panel 1030) which may be resistant to exonuclease I digestion. Any unreacted material remaining in the droplets may be a linear single stranded nucleic acid product which may be digested by the exonuclease I enzyme. As described above, each of the amplified MS droplet and amplified nMS droplet (step 2418) may be transported using droplet operations to a dried exonuclease I spot for sample cleanup (step 2420). In this step, droplet operations may be used to pulse the Amplified MS Droplet and Amplified nMS Droplet with the dried exonuclease I spot to ensure rehydration and mixing, producing a Digested MS Droplet and a Digested nMS Droplet (step 2420). The exonuclease I enzyme may digest into 1-2 nucleotide residues any unused PCR primers, unligated (open, not circularized) coded padlock probes, left over target nucleic acid sequences and any other linear single-stranded contents or contents with an exposed 3' end remaining in the droplet after amplification. The only products which may remain after reaction with the exonuclease I enzyme may include closed circular molecules or any double-stranded nucleic acid product. Remaining circularized molecules may not interfere with the subsequent steps in the single nucleotide mutation detection assay.

In one configuration, the Digested MS Droplet and the Digested nMS Droplet (step 2420) may be heated to fully inactivate the exonuclease I enzyme, producing an MS cleanup droplet and an nMS cleanup droplet. This step in combination with the former step may remove the need for bead-based purification because unwanted material may be reduced to small nucleotide residues. Furthermore, the heat treatment may remove the enzyme itself which may otherwise interfere with a PCR detection step, described below.

In one configuration as described above, it may be useful to determine the total amount of nucleic acid in each of the cleanup droplets (from step 2420) to ensure there is an appropriate amount of analyte for the specific detector. In step 2422, the amount of nucleic acid present in each cleanup droplet may be quantified by incorporating an intercalating fluorescent dye into the dsNA strands and measuring the difference in fluorescence relative to the background signal. This step may confirm enough amplification cycles have been performed to send the appropriate amount of nucleic acid to the sensor. In a preferred embodiment, excess nucleic acid may be produced in the amplification step 2418 and the quantification step 2422 may be used to determine what dilution of the droplet is needed to produce the desired nucleic acid concentration to send to the detector. In one configuration, if the calculation by the on-board processor determines an appropriate amount of nucleic acid has been created in the amplification step 2418, droplet operations may be used to transport the two sample droplets (step 2422) to an appropriate zone of the array detector 2424. As described above, if dilution is determined to be required, droplet operations may be used to dispense the calculated volume of buffer from the buffer reservoir and transport this volume of buffer to appropriately dilute the sample droplets. Droplet operations may be used to pulse the buffer with each droplet to ensure appropriate dilution of the target nucleic acid in the sample, producing an MS dilution droplet and an nMS dilution droplet (step 2422). In one configuration of the quantification step, if the processor determines there is insufficient nucleic acid present in any of the sample droplets, an error flag may be called which stops the entire assay and returns an error message for the end user.

Droplet operations may be used to separate a small volume approximately 1 to 10 pL from each MS and nMS diluted droplets (step 2422) for analysis, producing an MS picoscale droplet and an nMS picoscale droplet. Droplet operations may be used to transport each picoscale droplet to a dried reagent spot containing all the requisite reagents, except the PCR primers and the fluorescent intercalating dye, required for PCR-initiated fluorescence detection on the array detector 2424. These reagents include TaqPath ProAmp Master Mix, Taq DNA Polymerase, each of the four dNTPs, stabilizers, buffers, enzymes such as RNAse H2 enzyme, and MgCl₂. Droplet operations may be used to pulse each of the picoscale droplets to ensure complete rehydration and uniform mixing of the reagents with the nucleic acid in the sample. This step produces an MS detection droplet and an nMS detection droplet. In an alternative embodiment, the MS picoscale droplet and nMS picoscale droplet may be transported to an appropriate section of the array detector to be detected using a Roswell molecular wire analysis. This detection method is described below the fluorescence detection method immediately below.

In one configuration as described above, each of the MS and nMS detection droplets may be transferred to the X-1 Active Matrix Digital Microfluidics (AM-DMF) CMOS chip for the final detection steps on the CMOS array detector 2424. A detailed description of the CMOS array detector 620 may be found above.

After being transferred to the CMOS chip, droplet operations may be used to transport each of the MS and nMS detection droplets to a specific zone of the array detector 2424. FIG. 7 provides a more detailed schematic of the design of the CMOS array detector.

Droplet operations may be used to transport each MS and nMS detection droplet to the appropriate detector zone, shown for example as zones 2426 and 2428 respectively. When the droplet lands in the detector zone, it may flood the detection features located in that zone. Droplet operations may be used to pulse the liquid in each detector zone and in each detection feature to ensure complete rehydration and uniform mixing of each MS and nMS detection droplet with the combination of dried forward and reverse primers, any necessary PCR reagents not already present in the droplets and the intercalating dye pre-spotted in or on each detection feature.

In one configuration, the exact location of the combinatorial primers is known. If the matching target arrives from a MS or nMS detection droplet and floods the corresponding detection feature, PCR amplification may occur. After a sufficient quantity of target molecules are generated and after a sufficient quantity of intercalating dye is incorporated a fluorescent signal may occur. The result may be a binary detection output signal.

In one configuration, droplet operations may be used to transport a droplet of buffer to a detection feature containing dried PCR primers and an intercalating dye. A fluorescence measurement may be taken at 520 nm for example and collected for approximately 5 minutes. The background fluorescence signal may be collected and stored on the cartridge.

In one configuration, droplet operations may be used to transport each of the MS and nMS detection droplets to the correct section of the array detector zones. Droplet operations may then be used to pulse each of the MS and nMS droplets to rehydrate and uniformly mix the combinatorial PCR primers and the intercalating dye with the nucleic acid sample. Immediately upon rehydration and mixing with the nucleic acid containing droplets, the intercalating dye may begin to fluoresce in the range of the background noise.

In one configuration, PCR amplification may be performed to increase the amount of nucleic acid in each droplet until a fluorescence signal is obtained beyond that of the background noise. In one configuration, 15-45 PCR cycles may be performed. Subsequently a fluorescence measurement may be collected using a standard fluorescence detector. In another embodiment, PCR amplification may be allowed to continue for 2 additional cycles before another fluorescence measurement is collected. In one configuration, a fluorescence measurement may be collected after cycle 32 for example. In one configuration, a fluorescence measurement may be collected after 45 cycles. In one configuration, a fluorescence measurement may be collected after every PCR cycle. In one configuration, a fluorescence measurement may be collected after every third PCR cycle. The fluorescence signal as a function of PCR cycle number may be stored on cartridge. The on-cartridge processor may include an algorithm which determines when the fluorescence signal increases above a predetermined threshold indicating a positive result for that target. In one configuration, a fluorescence map may be made corresponding to specifically mapped detection features and which may be used to detect the presence of methylated target nucleotides. Since a precise map of each zone may be created at the time of reagent spotting, one may precisely determine which methylated target allele is fluorescing.

In one configuration, a fluorescence endpoint assay may be conducted where PCR amplification may be allowed to proceed for 45 cycles. In this embodiment, a fluorescence signal may be collected after the 45 cycles has completed.

Signal from the MS detection droplet where methylated sequences were inhibited from ligating may be compared to that of the nMS detection droplet where the target sequences were allowed to ligate regardless of methylation status. The difference in signal between the MS detection droplet and the nMS detection droplet provides a measure of the extent of methylation at a specific cytosine.

In a previous step, the MS and nMS picoscale droplets were combined with reagents necessary for PCR based detection. In another embodiment, the MS and nMS picoscale droplets may instead be transported to an appropriate zone of the array detector for detection by Roswell molecular wires. This configuration proceeds as described above.

For this analysis method, the original complementary coded padlock probes used in step 2416, may contain a Roswell molecular wire hybridization sequence 2106 as shown in FIG. 21 instead of or in addition to the universal amplification sequences and in addition to or instead of the PCR tag sequences (906, 908) shown in FIG. 9.

Droplets containing coded padlock probes with a molecular wire hybridization sequence may be transported using droplet operations to the array of detection features containing the molecular wires (see FIG. 17). After flooding the molecular wire containing wells, the coded padlock probe containing droplets may incubate with the molecular wires. As shown in FIG. 23, the molecular wire hybridization sequence may hybridize with the molecular wire's capture probe. Upon hybridization, a change in electrical signal may be measured. In one embodiment the electrical signal increases upon hybridization with the coded padlock probe. In another embodiment the electrical signal decreases upon hybridization with the coded padlock probe. The change in electrical signal between the MS picoscale droplet and the nMS picoscale droplet may be used to determine the percent methylation of the target sequence.

### 5.6.29 Direct detection of methylated nucleic acid using methylation-specific restriction enzymes

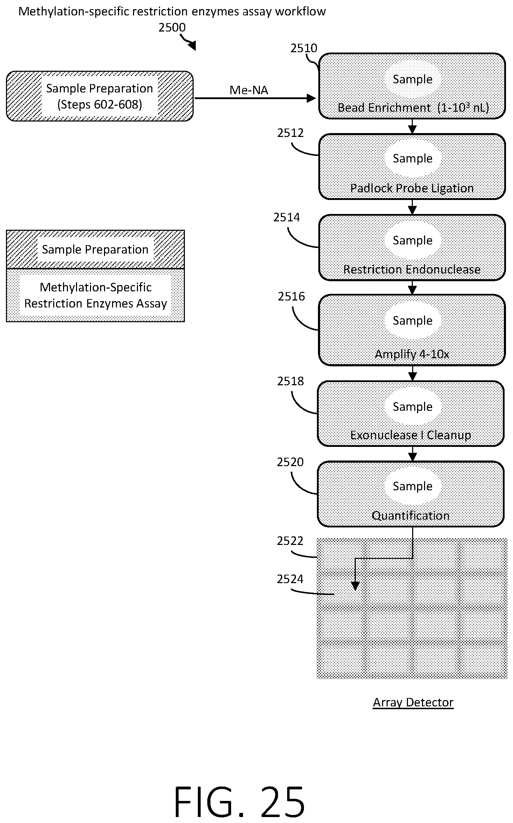

In another configuration, methylation-specific restriction enzymes may be used to detect the presence or absence of methylated nucleotides. This assay protocol, shown in FIG. 25, also known as methylation-specific multiplex ligation dependent probe amplification (MS-MLPA) is a PCR based technique for determining the methylation status of a particular locus. Methylation sensitive endonucleases such as Hhal may be used to cut the coded padlock probe-NA sample complex described above resulting in the inability to produce PCR product. If the target nucleic acid is methylated, Hhal is unable to cut the fragment which may then be subsequently amplified and detected by PCR. FIG. 25 is a flow diagram showing an example of a process 2500 of detecting methylated nucleic acid using methylation-specific restriction enzymes. Process 2500 is described for a DMF system 400; however, it will be appreciated that other systems, such as robotics system or other microfluidics systems, may be used.

In this configuration, the steps described above for methylation-specific inhibition of ligation may be followed where plasma is extracted from a sample of whole blood, followed by bead-based cfNA extraction, purification, concentration (steps 602-608 of FIG. 6) and a bead enrichment step 2510 to produce an enrichment droplet 2510.

In step 2512, droplet operations may be used to transport the enrichment droplet 2510 (possible enrichment volumes of 1, 10, 100, 1000 nL) into contact with a dried reagent spot containing HiFi DNA Ligase and coded padlock probes complementary to the target sequence of interest, where the target sequence may or may not be methylated. As in examples above, the coded padlock probes may contain sequences which may not be extended (non-extendable sequence 914) which allow for linear double stranded PCR products. Droplet operations may be used to pulse the droplet with the dried reagent spot to ensure complete rehydration, producing a probe droplet. In one configuration, the rehydrated coded padlock probes may anneal to the target sequence of interest and ligation may occur regardless of methylation status of the target sequence.

In step 2514, in one configuration, the probe droplet may be transported to an appropriate zone of the PCB chip for the restriction endonuclease step. In this configuration, the probe droplet may be transported using droplet operations to a dried spot of HHal restriction enzyme, for example. Droplet operations may be used to pulse the droplet over the dried reagent to ensure complete rehydration. The probe droplet and the enzyme may incubate to ensure complete reaction. In this configuration, the restriction enzyme will cut the double stranded nucleic acid at a specific GCGC recognition sequence. This particular HHal restriction enzyme will cut any double stranded GCGC sequence that is not methylated at the first cytosine. This enzyme will not restrict a methylated nucleic acid GCGC sequence. The action of this restriction enzyme breaks the circularized coded padlock probe into an open circle. This open circle, non-methylated nucleic acid sequence is now not available for PCR amplification, whereas the methylated circularized strand is available for PCR amplification. This circularized coded padlock probe annealed to the methylated target is referred to as the restriction probe droplet. In another configuration, other restriction enzymes may be used to restrict other specific recognition sequences.

In step 2516, in one configuration, the restriction probe droplet may be transported to an appropriate zone of the PCB chip for a universal amplification step as described above (step 614 of FIG. 6). In this process, the restriction probe droplet may be transported to a dried reagent spot containing dried dNTPs, generic PCR primers which complement the universal, generic PCR primers shown in medium gray on the complementary coded padlock probes (910 and 912) and DNA Polymerase. Droplet operations may be used to pulse the restriction probe droplet over the dried reagent spot to ensure complete rehydration and uniform mixing, producing a pre-amp restriction probe droplet. After rehydration and mixing, the pre-amp restriction probe droplet may be heated in a PCR initialization step to 94-96°C for 2-10 minutes to activate the DNA polymerase and to denature any contaminates in the mixture. To begin the denaturation step of PCR cycle 1, the pre-amp restriction probe droplet may be heated to 94-98°C for 20-30 seconds to break the hydrogen bonds between the coded padlock probe and the target sequence. This denatures the double-stranded nucleic acid sequence, separates the target nucleic acid sequence from the coded padlock probe and produces single-stranded products. The target nucleic acid sequence is a linear single-stranded product, while the coded padlock probe is a single-stranded circularized sequence. At this stage the rehydrated primers bind to the complementary sequences (in medium gray, 910 and 912) of the coded padlock probe. The reaction temperature of the pre-amp restriction probe droplet 2516 may be lowered to 50-65°C for 20-40 seconds to allow the primers to anneal to the circularized coded padlock probes. After the primers hybridize to the forward and reverse universal amplification sequences on the coded padlock probe, the rehydrated DNA polymerase incorporates dNTPs onto the primers in the elongation step at 72-78°C for Taq DNA polymerase for approximately 2 minutes. The coded padlock probes may incorporate amplification blocking sequences as described above or the amplification may involve rolling circle amplification. In one configuration, this PCR amplification process may be repeated for 3 or more cycles to generate enough target nucleic acid to be detected. In other configurations, generic PCR amplification may proceed for 20 or more cycles or 40 or more cycles. After for example 4-10 amplification cycles, the pre-amp restriction probe droplet may be held at 72-78°C for 5-15 minutes to ensure complete strand extension. The universal amplification step 2516 produces linear duplex nucleic acid copies (FIG. 10, 1030) containing the universal primers 910 and 912 shown in medium gray and the combinatorial forward and reverse PCR detection primers 906 and 908 shown in light gray. The resulting droplets may be referred to as amplified restriction probe droplet. This step produces excess nucleic acid in each droplet relative to that required to populate the detector array. The exact amount of nucleic acid in each droplet will be determined in a subsequent step, discussed below, and this nucleic acid will be diluted to a concentration range required by the fluorescence detector.

The amount of PCR product generated may be a function of how many coded padlock probes hybridize to the same target sequence. As described above it may be desirable to tune the signal at the detector if it is known in advance that a target allele is over-represented in the sample to prevent detector saturation.

After amplification of the coded padlock probes, linear duplex PCR products may be formed (FIG. 10) with universal primers (medium gray) and the one or more pairs of unique PCR tag sequences (light gray). The same forward and reverse universal primers (medium gray) may be used on every coded padlock probe in the assay to prevent bias, thereby ensuring all targets may be amplified uniformly and to the same degree. PCR products may only be produced upon successful ligation by the HiFi DNA Ligase. We have chosen the highest fidelity ligase commercially available. In the event the ligase incorrectly seals the nick of a wildtype allele pair, a cleanup step using the RNAse H2 protocol may be used as described above to prevent incorrect amplification in step 2516. This assay describes the use of methylation sensitive restriction enzymes to assess the state of methylation in a nucleic acid sequence. The coded padlock probes will be the correct complementary sequence.

In one configuration, any coded padlock probes which have hybridized with a target sequence may be circularized. After amplification they may be linear, double-stranded nucleic acid sequences (FIG. 10) which may be resistant to exonuclease I digestion. Any unreacted material remaining in the droplets may be a linear single stranded nucleic acid product which may be digested by the exonuclease I enzyme. This difference in sensitivity to the exonuclease I enzyme may provide an excellent method for differentiating desired products from waste and may allow for an efficient cleanup step. The remaining single-stranded contents of the amplified restriction probe droplet (step 2516) may be digested by the exonuclease I enzyme which may cleave individual ssNA nucleotides in the 3' to 5' direction. This may include any unreacted open (not circular) coded padlock probes, any unreacted forward and reverse primers, left over target sequences and any other contents in the droplet which may be single-stranded or have an exposed 3' end. The exonuclease digestion may reduce any susceptible components to 1-2 nucleotide residues. Double stranded PCR amplicons and circularized coded padlock probes may not be digested.

In step 2518, in one configuration, the amplified restriction probe droplet (step 2516) may be transported using droplet operations to a dried exonuclease I spot for sample cleanup. In this step, droplet operations may be used to pulse the amplified restriction probe droplet with the dried exonuclease I spot to ensure rehydration and mixing, producing a digested restriction probe droplet. At the end of the reaction time, the exonuclease I enzyme may digest unused PCR primers, unligated coded padlock probes and target nucleic acid into 1-2 nucleotide segments. Linear, single-stranded nucleic acid remaining in the droplet may also be digested into 1-2 nucleotide segments. The only products which may remain after reaction with the exonuclease I enzyme may include closed circular molecules or any double-stranded nucleic acid product. Remaining circularized molecules may not interfere with the subsequent steps in the single nucleotide mutation detection assay.

In one configuration, the digested restriction probe droplet may be heated at 80°C for 1 minute to fully inactivate the exonuclease I enzyme, producing a restriction cleanup droplet. This step, in combination with the former step, may remove the need for bead-based purification because unwanted material may be reduced to small nucleotide residues. Furthermore, the heat treatment may remove the enzyme itself which may otherwise interfere with a PCR detection step, described below.

In one configuration, it may be useful to determine the total amount of nucleic acid in each of the restriction cleanup droplets. When determining the presence or absence of each of the target nucleic acid sequences, it may be important to have the appropriate amount of nucleic acid for the detector. If there is insufficient nucleic acid, a real concern when sampling few target nucleic acid strands, the sensor may fail to produce a detectable signal. If there is too much nucleic acid in the sample, the sensor may saturate.

In step 2520, the amount of nucleic acid present in each restriction cleanup droplet may be quantified by incorporating a fluorescent dye into the dsNA strands and measuring the change in fluorescence. When surrounded by only water or aqueous buffer solutions, a fluorescent dye exposed to an excitation wavelength will release the energy as heat or vibrations. When in the presence of nucleic acid, certain fluorescent dyes such as SYBR Green (SYBR Gold) may bind in the hydrophobic regions. When exposed to an appropriate excitation wavelength, the energy absorbed by the fluorescent dye may be released as light at a higher wavelength. This method may be an extremely sensitive, fast and powerful way of quantifying the presence of nucleic acid, resolving on the order of picograms of nucleic acid per 100 µL sample volume. This step may confirm enough amplification cycles have been performed to send the appropriate amount of nucleic acid to the sensor. In a preferred configuration, excess nucleic acid may be produced in the amplification step 2516 and the quantification step 2520 may be used to determine what dilution of the droplet is needed to produce the desired nucleic acid concentration to send to the detector. Droplet operations may be used to dispense the appropriate volume of buffer from the buffer reservoir and transport this volume of buffer needed to appropriately dilute the restriction cleanup droplet. Droplet operations may be used to pulse the buffer with the restriction cleanup droplet to ensure appropriate dilution of the target nucleic acid in the sample.

Droplet operations may be used to transport 1 nL of for example Tris buffer over a dried spot of SYBR Green (SYBR Gold) master mix. Droplet operations may be used to pulse the buffer droplet over the dried dye spot to ensure complete rehydration and uniform mixing. The fluorescence of this droplet may be measured using a CCD camera, for example, at 520 nm to obtain the background fluorescence signal due to only the buffer in the restriction cleanup droplets and the intercalating dye. After measurement, the fluorescence is stored on board and this droplet is transported to a waste reservoir.

In another configuration, each of the restriction cleanup droplets may be transported using droplet operations to a quantification section of the PCB cartridge. In a quantification step 2520, the amount of total nucleic acid in each of the restriction cleanup droplets may be determined using a fluorescence measurement. In one configuration, droplet operations may be used to separate 1 nL from the restriction cleanup droplet and transport the droplet to a dried reagent spot containing SYBR Green (SYBR Gold) master mix. Droplet operations may be used to pulse the restriction cleanup droplet over the dried dye spot to ensure complete rehydration and uniform mixing of the SYBR Green (SYBR Gold) intercalating dye, producing a restriction quantification droplet. Immediately after the restriction cleanup droplets come in contact with the dried SYBR Green (SYBR Gold) master mix reagent spot, the fluorescence of each drop may be captured using an on-cartridge CCD camera at an example wavelength of 520 nm for 5 minutes.

In one configuration, the fluorescence of each of the restriction quantification droplets may be compared to an embedded, laboratory-generated, standard curve of PCR cycles as a function of fluorescence for varying nucleic acid concentrations. In this configuration, a standard curve of PCR cycles as a function of fluorescence for varying nucleic acid concentrations may be stored on cartridge. The fluorescence of each of the restriction quantification droplets may be compared to the stored standard curve to determine the total nucleic acid present in each droplet. After the total amount of nucleic acid in the restriction quantification droplets is determined, a calculation may be performed by the processor to determine the exact dilution necessary to ensure the nucleic acid concentration in each droplet is within the range dictated by the fluorescence detector.

In another configuration, 3 or 4 or 5 or more than 5 nucleic acid standards of known concentration may be spotted on the cartridge and may be used to generate a standard curve in-situ. In this configuration, one droplet of buffer may be dispensed from the on-board buffer reservoir using droplet operations and then transported to a dried spot of intercalating dye, for example SYBR Green (SYBR Gold) master mix.

Droplet operations may be used to separate the rehydrated SYBR Green (SYBR Gold) master mix reagent spot into the same number of drops as the number of dried nucleic acid standards spotted on the cartridge. In a configuration, the number of rehydrated intercalating dye spots may be 3 or 4 or 5 or more than 5 rehydrated dye droplets. Droplet operations may be used to transport each of the rehydrated SYBR Green (SYBR Gold) master mix droplets to merge with each of the nucleic acid standard spots. Droplet operations may be used to pulse each of the buffer droplets over each of the nucleic acid standard spots. After complete rehydration and uniform mixing, fluorescence may occur. In one configuration, a standard curve may be generated of nucleic acid concentration vs. fluorescence signal. In one configuration, the amount of nucleic acid in the restriction quantification droplets may be compared to the standard curve to determine the amount of nucleic acid in the restriction quantification droplet.

In another configuration, one concentrated standard nucleic acid spot may be dried on the chip, rehydrated and then diluted into 3, or 4, or 5, or more than 5 standard drops. In this configuration, droplet operations may be used to dispense one buffer droplet from the on-board buffer reservoir and then transport the buffer droplet to merge with the dried, concentrated nucleic acid spot. Droplet operations may be used to pulse the buffer droplet over the concentrated nucleic acid standard spot to ensure complete rehydration and unform mixing. In another configuration, droplet operations may be used to separate the rehydrated concentrated nucleic acid standard droplet into 3, or 4, or 5, or more than 5 droplets. In another configuration, droplet operations may be used to transport for example Tris buffer from the on-board buffer reservoir to the first rehydrated, concentrated nucleic acid spot. In another configuration, droplet operations may be used to transport buffer droplets to each of the 3, or 4, or 5, or more than 5 nucleic acid standard droplets to dilute them to the appropriate concentration. In another configuration, one droplet of buffer may be dispensed from the on-board buffer reservoir and transported using droplet operations to merge with one dried spot of SYBR Green (SYBR Gold) intercalating dye. Droplet operations may be used to pulse the one droplet of buffer over the dried dye spot to ensure complete rehydration and uniform mixing.

Droplet operations may be used to split the one droplet of dyed buffer into the same number droplets as the number of nucleic acid standard droplets. In one configuration, the dyed buffer may be split into 3, or 4, or 5, or more than 5 droplets.

Droplet operations may be used to transport each of the dyed buffer droplets to merge with each of the nucleic acid standard droplets. Droplet operations may be used to pulse the merged dyed buffer and nucleic acid standard droplets to ensure uniform mixing. Once the nucleic acid has incorporated the intercalating dye, fluorescence may occur. In one configuration, a standard curve may be generated of nucleic acid concentration vs. fluorescence signal. In one configuration, the amount of nucleic acid in the restriction quantification droplet may be compared to the standard curve to determine the amount of nucleic acid in the restriction quantification droplet.

After the appropriate concentration of nucleic acid in the restriction quantification droplet is obtained, a calculation may be performed by an on-board processor to determine the exact dilution necessary to ensure the nucleic acid concentration in each droplet may be within the range dictated by the fluorescence detector. In one configuration, if the fluorescence detector determines an appropriate amount of nucleic acid has been created in the amplification step 2516, droplet operations may be used to transport the restriction quantification droplet to an appropriate zone of the array detector 2522. In one configuration of the quantification step, if the processor determines there is insufficient nucleic acid present in the restriction quantification droplet, an error flag may be called which stops the entire assay and returns an error message for the end user.

In one configuration, if the fluorescence detector determines there is excess nucleic acid present in the sample such that the drop would saturate the PCR array detector, a calculation may be performed by the instrument to determine the appropriate dilution needed for each droplet. Amplifying the sample nucleic acid to more than the amount needed by the detector may be a preferred embodiment. Based on the dilution factor determined in the quantification step 2520, a calculation may be performed to determine what volume of for example Tris buffer may be needed to appropriately dilute the restriction quantification droplet. In one configuration, droplet operations may be used to transport the appropriate volume of for example Tris buffer to the restriction quantification droplet. Droplet operations may be used to pulse the restriction quantification droplet with its respective dilution buffer droplet to ensure uniform mixing and dilution, producing a restriction diluted droplet.

Droplet operations may be used to separate a small volume approximately 1 to 10 pL from the restriction diluted droplet for analysis, producing a restriction picoscale droplet. Droplet operations may be used to transport each restriction picoscale droplet to a dried reagent spot containing all the requisite reagents, except the PCR primers and the fluorescent intercalating dye, required for PCR-initiated fluorescence detection on the array detector 2522. These reagents include TaqPath ProAmp master mix, Taq DNA polymerase, each of the four dNTPs, stabilizers, buffers, enzymes such as RNAse H2 enzyme, and MgCl₂. Droplet operations may be used to pulse the restriction picoscale droplet to ensure complete rehydration and uniform mixing of the reagents with the nucleic acid in the sample. This step produces a restriction detection droplet. In an alternative configuration, the restriction picoscale droplet may be transported to an appropriate section of the array detector to be detected using a Roswell molecular wire analysis. This detection method is described below the fluorescence detection method immediately below.

In one configuration, standard engineering protocols may be used to transfer the restriction detection droplet to the X-1 Active Matrix Digital Microfluidics (AM-DMF) CMOS chip. In one embodiment the CMOS detector chip may be fabricated using glass. In another configuration, the CMOS detector chip may be fabricated using silicon. A PCB can accommodate a minimum drop size in the nanoliter volume range. However, below droplet volumes of approximately 100 nL, PCBs reach their manufacturing limitations and cannot be used for smaller volumes. A silicon or glass CMOS chip can effectively process picoliter sized droplet volumes. In order to increase the signal to noise ratio it may be important to minimize the sample volume and maximize the sample concentration as discussed previously. Therefore, one configuration may include transferring the restriction detection droplet to the CMOS chip for the final detection steps on the CMOS array detector 2522. A detailed description of the CMOS array detector 620 may be found above.

After being transferred to the CMOS chip, droplet operations may be used to transport the restriction detection droplet to a specific zone of the array detector 2522, where the location shown in 2522 is for example purposes only.

Droplet operations may be used to transport the restriction detection droplet to the appropriate detector zone, shown for example as zone 2524. When the droplet lands in the detector zone, it may flood the detection features located in that zone. Droplet operations may be used to pulse the liquid in each detector zone and in each detection feature to ensure complete rehydration and uniform mixing of the restriction detection droplet with the dried forward and reverse primers and the intercalating dye pre-spotted in or on each detection feature. Immediately upon rehydration and mixing with the nucleic acid containing droplets, the intercalating dye may begin to fluoresce. This fluorescence may be used to detect the presence of mutant allele targets and is discussed further below.

In one configuration, a unique PCR tag may be used for each separate target sought as described above. In one configuration, each target may be detected by a unique combination of two primers reused over all zones which significantly reduces the number of primers needed. The unique combinatorial PCR tags (906 and 908) on the coded padlock probes are shown in light gray in FIG. 9. The combination of forward and reverse primers which hybridize to the unique PCR tags may be pre-spotted in or on each detection feature.

During the manufacture of the CMOS chip, an array of forward and reverse PCR primers and an intercalating dye such as SYBR Green, SYBR Gold or Eva Green may be spotted in each detection feature and dried using for example forced air. In each zone, an array of fifteen, for example, forward PCR primers T₁F through T₁₅F may be spotted in or on each detection feature of the rows and an array of fifteen, for example, reverse primers T₁R through T₁₅R may be spotted in or on each detection feature of the columns. Therefore, each detection feature may contain a unique combination of at least two dried primers, at least one forward and at least one reverse primer. Any number of zones may be dedicated to this analysis method which serves to increase the number of targets detected.

In one configuration, the exact location of the combinatorial primers is known. If the matching target arrives from a restriction detection droplet and floods the corresponding detection feature, PCR amplification may occur. After a sufficient quantity of target molecules are generated and after a sufficient quantity of intercalating dye is incorporated a fluorescent signal may occur. The result may be a binary detection output signal (signal vs. no signal). While T₁F+T₁R may be used in the upper left corner, for example, of each zone, a fluorescence signal from the upper left corner of different zones may indicate the presence of a different target. In one configuration, one or more wells in each zone contain unique PCR tag sequences which may be used to detect the internal positive and negative control sequences.

In one configuration, droplet operations may be used to transport a droplet of buffer to a detection feature containing dried PCR primers and an intercalating dye. A fluorescence measurement may be taken at 520 nm for example and collected for approximately 1 or more seconds or 10 or more seconds or 30 or more seconds or 60 or more seconds. The background fluorescence signal may be collected and stored on the cartridge.

In one configuration, droplet operations may be used to transport the restriction detection droplet to the correct section of the array detector zones. Droplet operations may then be used to pulse the restriction detection droplet to rehydrate and uniformly mix the combinatorial PCR primers and the intercalating dye with the nucleic acid sample. Immediately upon rehydration and mixing with the nucleic acid containing droplets, the intercalating dye may begin to fluoresce in the range of the background noise.

In one configuration, PCR amplification may be performed to increase the amount of nucleic acid in each droplet until a fluorescence signal is obtained beyond that of the background noise. In this configuration, the wells in each zone may be heated to 95°C for 5 minutes to activate the DNA polymerase and to denature any contaminants in the flooded wells. In one configuration, the zones may be heated to 94-98°C for 20-30 seconds to convert the double stranded nucleic acid present in the droplet to single-stranded nucleic acid. In a configuration, the rehydrated primers pre-spotted in the detection feature may anneal to the complementary sequence of the PCR tags in the original coded padlock probes. In one configuration, the temperature of the zones may be reduced to 50-65°C to allow for optimal primer annealing. In another configuration, the temperature of each zone may be increased to 72-78°C to allow optimal template extension by Taq polymerase. This step may proceed for a certain length of time. In one configuration, 15-20 PCR cycles may be performed. Subsequently a fluorescence measurement may be collected using a standard fluorescence detector. In another configuration, PCR amplification may be allowed to continue for 2 additional cycles before another fluorescence measurement is collected. In one configuration, a fluorescence measurement may be collected after cycle 32 for example. In one configuration, a fluorescence measurement may be collected after 45 cycles. In one configuration, a fluorescence measurement may be collected after every PCR cycle. In one configuration, a fluorescence measurement may be collected after every third PCR cycle. The fluorescence signal as a function of PCR cycle number may be stored on cartridge. The on-cartridge processor may include an algorithm which determines when the fluorescence signal increases above a predetermined threshold indicating a positive result for that target. In one configuration, a fluorescence map may be made corresponding to specifically mapped detection features and which may be used to detect the presence of mutant target alleles. Since a precise map of each zone may be created at the time of reagent spotting, one may precisely determine which target mutant allele is fluorescing.

In one configuration, a fluorescence endpoint assay may be conducted where PCR amplification may be allowed to proceed for 45 cycles. In this configuration, a fluorescence signal may be collected after the 45 cycles has completed.

In a previous step, the restriction picoscale droplet was combined with reagents necessary for PCR based detection. In another configuration, the restriction picoscale droplet may instead be transported to an appropriate zone of the array detector for detection by Roswell molecular wires. This configuration proceeds as described above.

For this analysis method, the original complementary coded padlock probes used in step 2512, may contain a Roswell molecular wire hybridization sequence 2106 as shown in FIG. 21 instead of or in addition to the universal amplification sequences and in addition to or instead of the PCR tag sequences.

Restriction picoscale sample droplets containing circularized coded padlock probes with a molecular wire hybridization sequence may be transported using droplet operations to the array of detection features containing the molecular wires. After flooding the molecular wire containing wells, the coded padlock probe containing restriction picoscale sample droplets may incubate with the molecular wires. As shown in FIG. 23, the molecular wire hybridization sequence may hybridize with the molecular wire's capture probe. Upon hybridization, a change in electrical signal may be measured. In one configuration the electrical signal increases upon hybridization with the coded padlock probe. In another configuration the electrical signal decreases upon hybridization with the coded padlock probe.

### 5.6.30 Direct detection of methylated nucleic acid using methylation-specific immuno-precipitation

Methylation specific immuno-precipitation has previously been developed and applied to NGS based methylation analysis. For example, the cfMeDIP-seq method performs enrichment based on methylation status prior to sequencing. That is, only those fragments containing a methylated site may be pulled out from the original sample for the sequencing reaction. In one aspect of the present invention, MeDIP is applied to one of the subsamples and the presence of a particular target sequence may be determined by PCR. Although this technique cannot identify the specific location of a methylated nucleic acid base it can provide information that at least one or no methylated sites were located within a particular distance of a specific locus as defined by the average fragment size (e.g., 150 bp).

In some configurations, the probe may consist of both a capture sequence and decoder sequence. The capture sequence may be complementary to the target sequence and may be used to determine whether the target was present in the original sample. The decoder sequence may be used to determine the identity of the capture sequence (and bead) among an array of beads (and sequences) which may have been randomly distributed among an array of bead-capture wells. In some configurations, one or more ion-sensitive field-effect transistor (ISFET) sensors may be located in or on each detection feature and may be used to detect whether a target has been captured and may be used to detect the decoding process. The target or decode oligonucleotide may be hybridized to the capture probe which serves to prime the nucleic acid for subsequent extension. In some configurations the primed nucleic acid strand may be extended by adding one or more nucleotides (dNTPs) in the presence of a DNA Polymerase enzyme. The identity of the next unpaired bases or bases may be known through design of the probe, enabling control over the number and types of bases that are added. For example, if A bases are flanking the primed nucleic acid then T bases may be incorporated, thereby releasing protons per molecule which may be detected as a change in pH by the ISFET sensors. Alternatively, all four bases may be added to extend any sequence to its full length. In some embodiments the decode oligonucleotide may be extended towards the bead along one strand of the nucleic acid complex and the target is extended away from the bead on the complementary strand. The combination of multiple copies of each capture probe attached to each bead and the extension of each strand by multiple bases (e.g., 10 bases, or 20 bases, or 30 bases, or 40 bases) provides an amplification effect which improves the reliability of detection using the ISFET sensors. Sensitivity may be further improved by using captured target probes as primers for performing an amplification reaction such as rolling circle amplification.

The disclosure enables efficient reaction kinetics because all of the beads may be contained within a small volume (i.e., droplet) which can be mixed enabling rapid and efficient hybridization of capture probes and targets as compared to a traditional microarray in which the beads and/or capture probes are arrayed upon a surface. Thus, convective mass transfer can be used to accelerate the binding kinetics versus the (mostly) diffusion-limited transfer which is typical with planar microarrays. Decoding the identity of the beads following sample hybridization and random filling of wells is a major innovative aspect of the present invention. In contrast to the approach used by Illumina and others, bead decoding is performed at the time of use following target hybridization rather than as an aspect of the manufacturing process.

A further aspect of the present disclosure is that providing the sensor array and beads as separate components (i.e., rather than as a finished and decoded array) enables greater flexibility for the supplier with respect to managing inventory, multiple product configurations and shelf life. That is, the content of the assay which is defined by the beads and capture probes can be provided separately from the sensor device enabling the supplier to mix and match content depending on the goals of the particular assay. Additionally, economies of scale may be provided by using a single universal sensor device which is common to an entire class of assay types.

In a further aspect of the disclosure, additional techniques for discriminating between methylated and non-methylated nucleic acid may be provided. For example, methyl-binding proteins may be used to attach tags of a known sequence specifically in the vicinity of CpG sites and chemically fusing the nucleic acid strands to create a triple-stranded construct. PCR may then be performed on the tags and quantitation may then be performed to determine the level of methylation at each particular locus.

In another configuration, the method of the Roswell molecular wires may be used as described above for direct detection in the methylation-specific immuno-precipitation method.

### 5.6.31 Detection of methylated cytosine using dual hybridization probes and methylation sensitive endonuclease digestion

In a variation of the ligation mediated assays / workflows described above for detection of methylated nucleotides in a target sequence of interest, a dual probe approach in combination with a methylation sensitive endonuclease may be used. In this approach, a methylation conversion reaction (e.g., a bisulfite conversion) reaction is not used. For example, a cell-free DNA sample may be divided into two parallel streams for analysis of methylated and unmethylated targets.

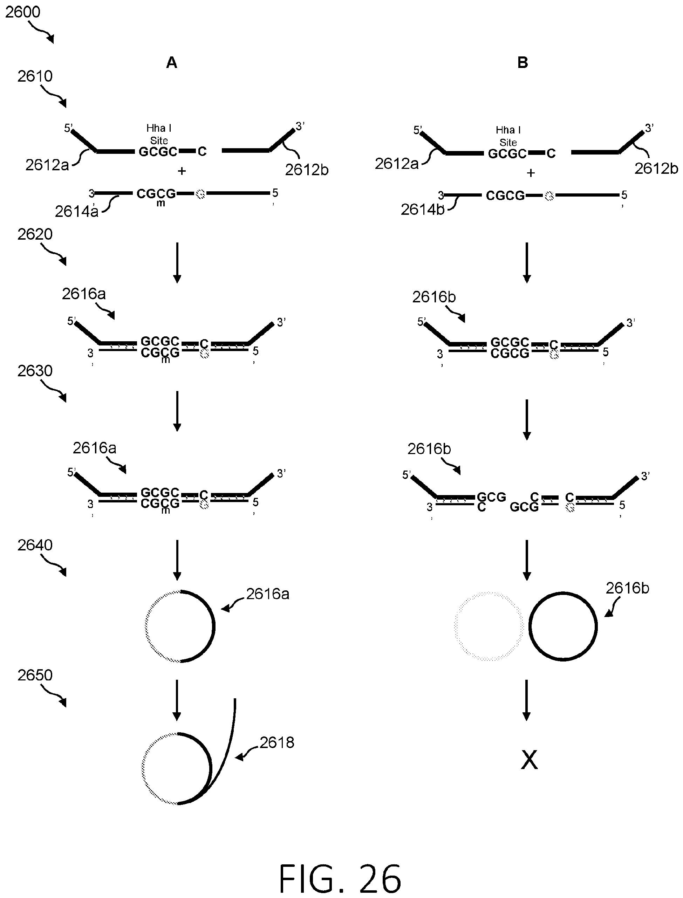

FIG. 26 illustrates a process 2600 of using dual hybridization probes in combination with a methylation sensitive endonuclease digestion reaction to differentiate a methylated target sequence and an unmethylated target sequence. In one example, sample preparation for input into process 2600 may be a performed as previously described for FIG. 6 starting from a 10 mL whole blood sample (step 602), separating, collecting the plasma fraction (step 604) resulting in nucleic acid material concentrated on magnetic beads (step 606), and purifying and concentrating the nucleic acid material (step 608); however, it will be appreciated that other systems, such as robotics systems or other microfluidics systems, may be used. Process 2600 may include, but is not limited to, the following steps and unspecified additional steps.

In step 2610, nucleic acid sample, (e.g., a cell-free DNA sample) may be divided into two parallel streams for analysis of methylated target (panel A) and unmethylated target (panel B) and hybridized with a pair of ligation probes 2612a and 2612b. Hybridization probes 2612a and 2612b are designed to hybridize adjacent to a target site of interest. Hybridization probe 2612a includes, for example, a primer sequence (not shown), a sample index (not shown), a code element (not shown), and sequences for a methylation sensitive endonuclease site that flank a target site of interest. Hybridization probe 2612b includes, for example, a primer sequence (not shown), a sample index (not shown), a code element (not shown). For example, in panel A, a methylated target 2614a is hybridized with ligation probes 2612a and 2612b; and in panel B, an unmethylated target 2614b is hybridized with ligation probes 2612a and 2612b.

In step 2620, a ligation reaction is performed. For example, ligation probes 2612a and 2612b hybridized to methylated target 2614a immediately adjacent to each other leaving no gap that can be ligated to produce a ligated dual probe strand 2616a. Ligation probes 2612a and 2612b hybridized to unmethylated target 2614b in a manner that generates a gap that is not ligated in the presence of the DNA ligase and generates an unmethylated target 2616b.

In step 2630, a methylation sensitive endonuclease digestion reaction is performed. The methylation sensitive endonuclease will cut at an unmethylated site but will not cut at a methylated site. In this example, the methylation sensitive endonuclease is Hha I. For example, in the presence of Hha I the methylated target 2616a is not cut and the unmethylated target 2616bb is cut at the restriction endonuclease site to generate two fragments.

In step 2640, a single-stranded DNA ligation reaction is performed to generate circularized molecules. The single-stranded ligation creates an intact primer site that can be used in a rolling circle amplification reaction. In one example, the single-stranded DNA ligase is Circligase. In this step, the uncut methylated target 2616a is circularized to create a circular recognition element 2616a that includes an intact RCA primer site. The Hha I cut unmethylated target 2616b is circularized to create two circular modified recognition elements 2616b that do not include an intact RCA primer sites.

In step 2650, the circularized modified recognition element 2616a is amplified in a rolling circle amplification reaction to generate a detection product 2618.

### 5.6.32 Variant detection using ligation of coded randomers

In a variation of the padlock probe-mediated methods of detecting a single nucleotide variant in a target sequence, a probe that includes a code element combined with a targeting or recognition element (i.e., a "code element probe") may be used to incorporate unknown dNTPS at a variant site of interest, thereby querying the site of interest for nucleotide usage at that site. In one example, the variant of interest is a single nucleotide polymorphism (SNP). In one example, the variant of interest is an insertion/deletion (indel) site where all substitutions may be evaluated.

In one configuration, the recognition element may be designed to hybridize to a target sequence one base proximal to the variant of interest. In one configuration, the recognition element may be designed to hybridize to a target sequence two bases proximal to the variant of interest. A set of query sequences or "randomers" may then be used to query the variant of interest for the nucleotide present at that site. The set of randomers may include all four nucleotide bases that may be possible at that variant site. In one configuration, each randomer terminates in one of the four possible bases. In another configuration, each randomer terminates with a dinucleotide. In this configuration, 16 combinations of possible variants may be probed.

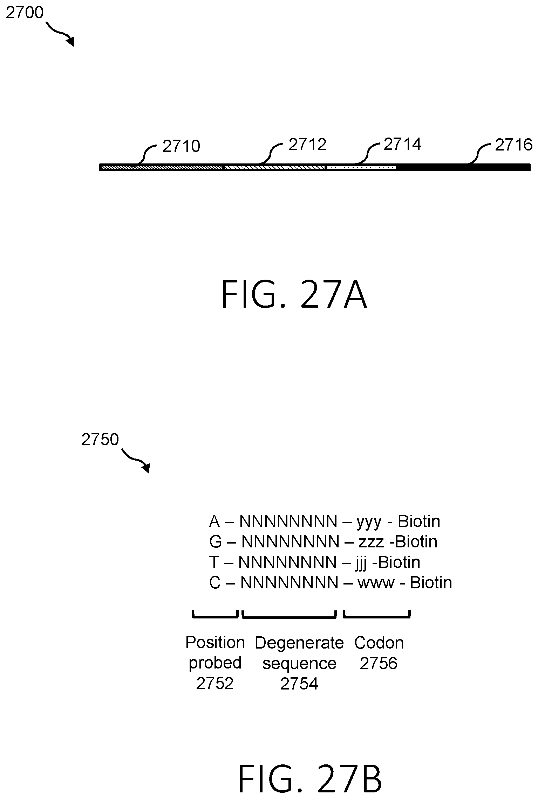

FIG. 27A is a schematic diagram of an example of a code element probe 2700 designed to query a target sequence for a variant of interest. The probe may include, for example, a target specific recognition element 2710 for recognizing and hybridizing to a target of interest and a code element 2716 that may be used in a transformation event / coding event to associate the code element with the target sequence, and subsequently used as a surrogate for detection of the target sequence. Probe 2700 may further include a primer sequence 2712 and a sample index sequence 2714.

A probe sequence may also include a UMI (not shown). In one configuration, the UMI may be used in a next generation sequencing (NGS) readout process to minimize intrinsic sequencing error rate.

FIG. 27B is a schematic diagram of an example of a set of randomers 2750 that may be used to query (probe) a variant site of interest in a targeted sequence. The set of randomers 2750 includes all four bases, A, G, T, and C as "position probes" 2752 for querying the variant site. Each position probe 2752 is coupled to a degenerate (wobble) sequence 2754 and a codon 2756 that is associated to the position probed. For example, the codon yyy is associated to a position that incorporates an A; a codon zzz is associated to a position that incorporates a G; a codon jjj is associated with a position that incorporates a T; and a codon www is associated with a position that incorporates a C. In this example, each randomer in the set of randomers 2750 terminates in a single position code 2752.

Each randomer may also include a capture tag that may be used in a subsequent cleanup step in the assay. The capture tag may, for example, be linked to the randomer using a cleavable linkage. In this example, the capture tag is a biotin molecule that is linked via a cleavable linkage to each randomer.

In one configuration, a rolling circle amplification process may be used to generate a detection product for determining the identity of a nucleotide variant at a variant site of interest in a target sequence. The rolling circle amplification process may, for example, be used to produce a "nanoball" that may then be converted into digital counts of a variant event.

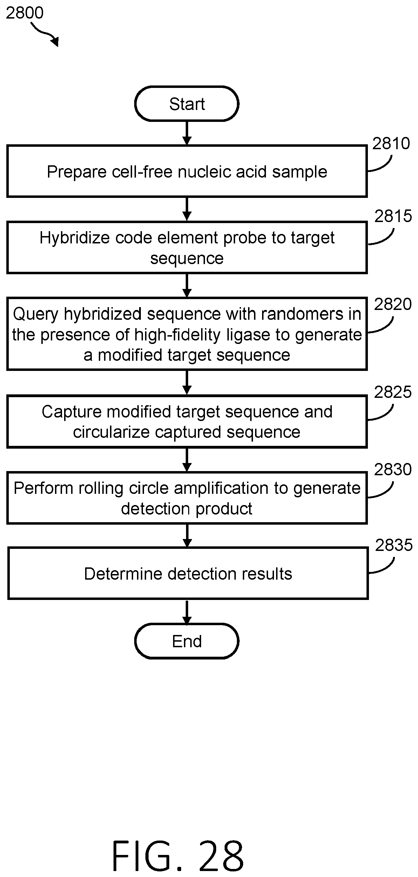

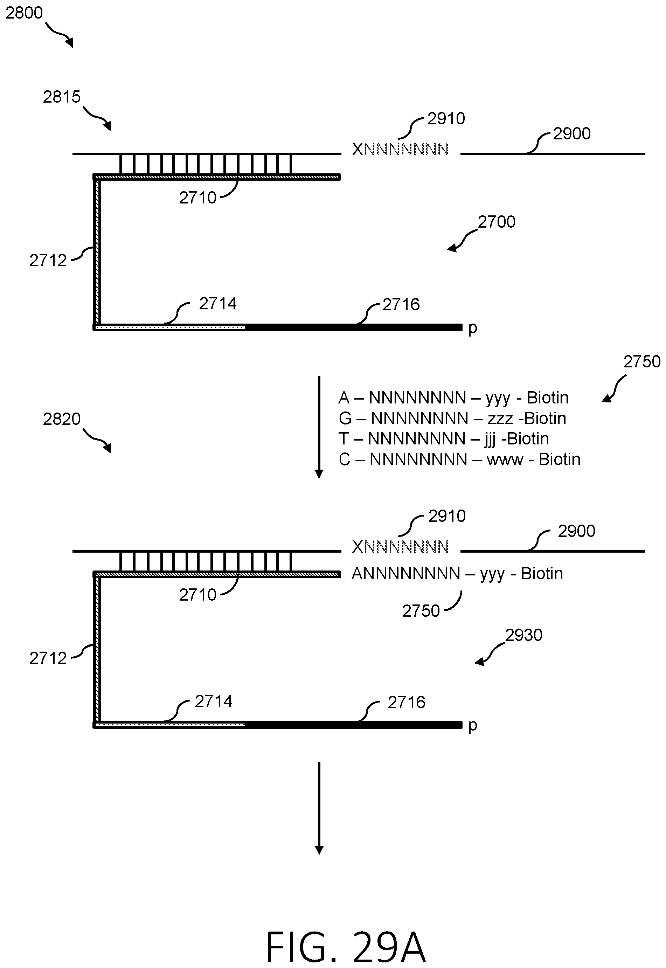

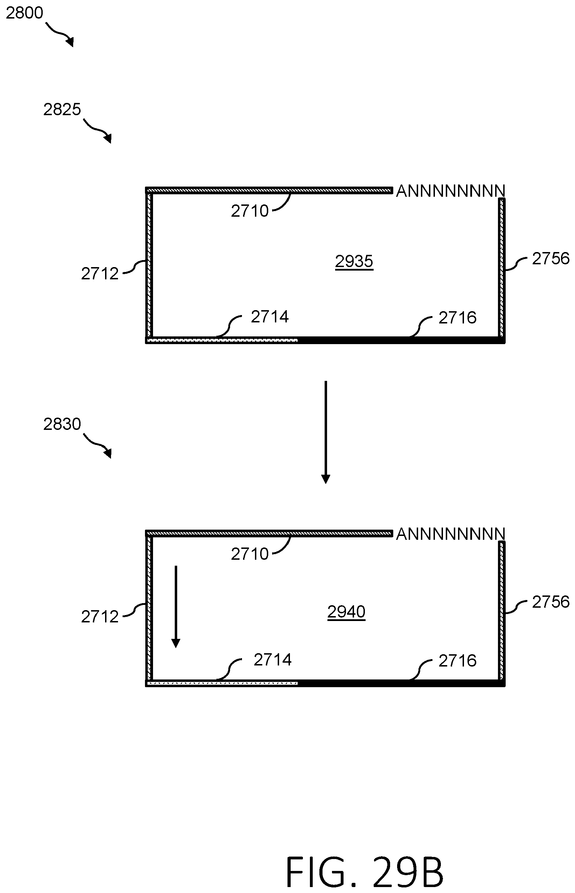

FIG. 28 is a flow diagram of an example of a method 2800 of using a probe that includes a code element combined with a recognition element and a set of coded randomers to query a variant of interest in a target sequence. Method 2800 may include, but is not limited to, the following steps and unspecified additional steps. Some of the steps of method 2800 are shown pictorially in FIG. 29A and FIG. 29B.

In step 2810, a cell-free nucleic acid sample is prepared. In one example, sample preparation may be performed as previously described for FIG. 6 starting from a 10 mL whole blood sample (step 602), separating and collecting the plasma fraction (step 604), extracting nucleic acid material on magnetic beads (step 606), purification and concentration (step 608), and bead enrichment (step 610); however, it will be appreciated that other systems, such as robotics systems or other microfluidics systems, may be used.

In step 2815, a code element probe is hybridized to a target sequence of interest. For example, code element probe is hybridized to a target sequence of interest that includes a variant site of interest. This step is also shown pictorially in FIG. 29A.

In step 2820, the hybridized sequence is queried with a set of randomers in the presence of a high-fidelity ligase to generate a modified recognition element sequence. For example, a randomer that perfectly matches the target site with the variant of interest is hybridized to the target sequence. In the presence of the high-fidelity ligase, the hybridized randomer is ligated to the code element probe to generate a modified recognition element that now includes the sample index, the code element, the variant specific codon, and a capture tag. This step is also shown pictorially in FIG. 29A.

In step 2825, the modified recognition element sequence is captured and circularized. For example, the capture tag on the modified recognition element sequence may be used to capture the modified recognition element sequence. In one example, the capture tag is a biotin molecule that is linked to the modified recognition element sequence via a cleavable linkage. In one example, a streptavidin bead-based cleanup protocol may be used to capture and purify the modified recognition element sequence. At the end of the cleanup protocol, the biotin capture tag is removed from the modified recognition element sequence in a cleavage reaction. The modified recognition element sequence is then circularized in a ligation reaction using, for example, Circligase. This step is also shown pictorially in FIG. 29B.

In step 2830, the circularized modified recognition element is amplified to generate a detection product. In one example, the circularized modified recognition element is amplified using a rolling circle amplification reaction. The detection product now includes the sample index, the code element, and the variant specific codon that is unique to the base that was incorporated into the modified recognition element sequence. This step is also shown pictorially in FIG. 29B.

In step 2835, the detection results are determined. For example, the specific codon that is unique to the base that was incorporated into the modified recognition element sequence in step 7120 may be decoded to determine the identity of the incorporated nucleotide. Any means of determining the sequence of the variant specific codon and code element (and other elements such as the sample index) may be used as described herein.

FIG. 29A and FIG. 29B illustrate steps of method 2800 of FIG. 28. In step 2815, code element probe 2700 is hybridized to a target sequence of interest 2900 that includes a variant site of interest 2910, where X is the variant of interest and N are sequence nucleotides.

In step 2820, the hybridized sequence is queried (probed) with a set of randomers 2750 in the presence of a high-fidelity ligase to generate a modified recognition element sequence 2930. In this example, the randomer that includes an A nucleotide is hybridized to the variant of interest. In the presence of the high-fidelity ligase, the hybridized randomer is ligated to the code element probe to generate a modified recognition element that now includes the code element.

In step 2825, the modified recognition element sequence 2930 is captured and circularized. For example, the capture tag on the modified recognition element sequence 2930 may be used to capture the modified recognition element sequence. In one example, the capture tag is a biotin molecule that is linked to the modified recognition element sequence via a cleavable linkage. In one example, a streptavidin bead-based cleanup protocol may be used to capture and purify the modified recognition element sequence 2930. At the end of the cleanup protocol, the biotin capture tag is removed from the modified recognition element sequence in a cleavage reaction. The modified recognition element sequence is then circularized using, for example, Circligase to produce a circular modified recognition element sequence 2935.

In step 2830, the circularized modified recognition element is amplified to generate a detection product 2940. In one example, the circularized modified recognition element 2935 is amplified using a rolling circle amplification reaction using primer sequence 2712. The detection product 2940 now includes the sample index 2714, the code element 2716, and the variant specific codon 2756 that is unique to the base that was incorporated into the modified recognition element sequence.

The variant detection assay described above examines a double-strand DNA, i.e., the randomers may probe both the sense and antisense strand. If a randomer that terminates with a dinucleotide is used, both nucleotides must match the variant sequence. Since the randomers incorporated in to both the sense and antisense strand of the modified recognition element sequence using the same code element probe, both the strands include the same code element. This approach may provide a means for identification of mismatches and error correction.

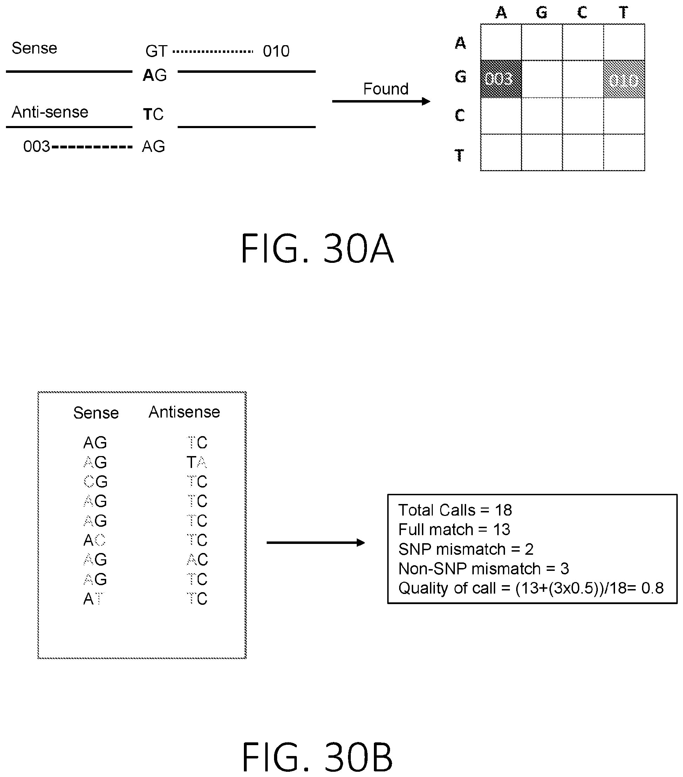

FIG. 30A and FIG. 30B are panels of a schematic diagram showing an example of identifying mismatches and error correction in a variant sequence detection process. Referring now to FIG. 30A, a sense strand includes a dinucleotide AG at the variant site and an anti-sense strand includes a corresponding dinucleotide TC at the variant site. In the detection of the variant in the sense strand a randomer comprising a GT is required. In this example, the randomer comprising a GT dinucleotide is associated with a codon "010". In the detection of the variant in the antisense strand, a randomer comprising an AG dinucleotide is required. In this example, the randomer comprising an AG dinucleotide is associated with a codon "003". This can be represented by a two-dimensional matrix of positive calls (e.g., either positive for 003 or 010).

Referring now to FIG. 30B, quality scores can be assigned to each call to further qualify the correctness of a call, i.e., the ability of the ligase to consistently recognize both the sense and anti-sense strands. In this example, a 50% weight to a single non-SNP mismatch was assigned.

### 5.7 Immunoassays

### 5.7.1 Immuno-PCR protein detection assay

In a variation of the above assays, digital microfluidics techniques may be used for immunoassays detecting proteins. In one configuration, ELISA based Immuno-PCR methods may be coupled with DMF on microchips to reduce sample and reagent volumes and improve detection sensitivity and specificity over the current state of the art.

FIG. 31 is a flow diagram of an example of an immuno-PCR assay workflow 3100, wherein a capture antibody is used to detect a protein bound to a target sequence. One configuration of the immuno-PCR assay may start from the plasma sample generated in step 604 (see FIG. 6). In one configuration, in step 3102, a capture antibody bound to a magnetic bead may immobilize the protein of interest when mixed with the plasma sample. In step 3104, a detection antibody may bind to the protein of interest forming a sandwich complex. In step 3106, in one configuration, the detection antibody may be coupled with an enzyme to participate in ELISA optical detection. In another configuration, the detection antibody may bind with one or more enzyme-coupled secondary antibodies which may participate in ELISA optical detection. In step 3108, in another configuration, the detection antibody may be coupled to a detector oligonucleotide which may participate in immuno-PCR detection. These configurations will be described below. In one configuration, approximately 100 µL of the plasma sample may be transferred from the sample collection reservoir to the electrowetting instrument cartridge for further analysis.

In one configuration, a droplet of for example BUF037A (Bio-Rad, Hercules, CA) buffer or PBS/Tween buffer may be transported from the buffer reservoir using droplet operations to merge with a dried spot of capture antibodies designed to capture the protein of interest. Droplet operations may be used to pulse the buffer over the dried spot of capture antibodies to ensure complete rehydration, producing a capture antibodies droplet (step 3102).

Rehydrated capture antibodies droplet may be transported to merge with a spot of magnetic beads purchased from for example Beckman Coulter Life Sciences (Indianapolis, IN) and pre-dried onto the PCB cartridge. Droplet operations may be used to pulse the rehydrated capture antibodies droplet over the dried spot of magnetic beads to ensure complete rehydration of the beads. The magnetic beads and rehydrated capture antibody droplet may be incubated to allow the capture antibodies to completely bind to the magnetic beads, producing a capture antibodies bead droplet.

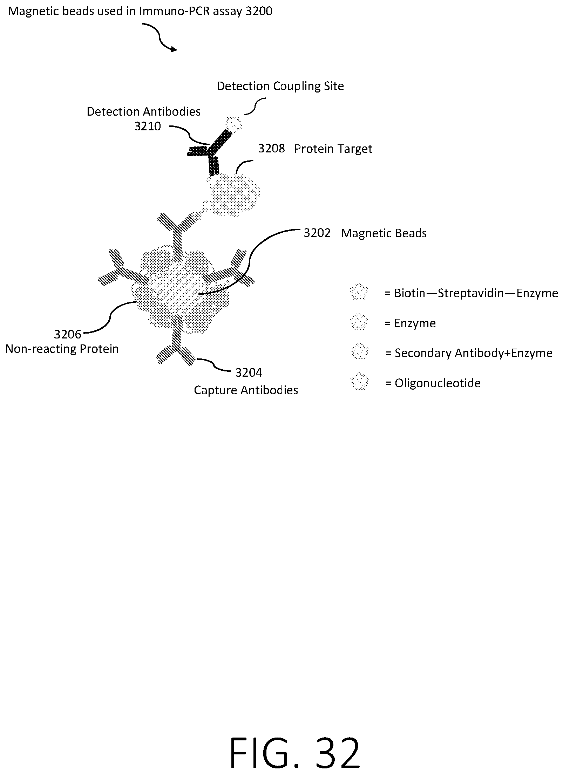

This arrangement may be seen in FIG. 32, where 3202 represents the magnetic beads and 3204 represents the capture antibodies bound to the magnetic beads.

In one configuration, the capture antibodies may be covalently bound to the magnetic beads. In another configuration the capture antibodies may be bound to the magnetic beads. In another configuration, the capture antibodies may be bound to the magnetic beads using a streptavidin-biotin non-covalent attachment method. Subsequently a magnetic field may be applied to the magnetic beads which may secure them to the PCB surface. Droplet operations may then be performed to dispense and then transport a droplet of BUF037A for example to merge with the capture antibodies-beads droplet (step 3102) to wash away any unbound capture antibodies replacing them with buffer, producing a droplet of approximately 25 µL. In one configuration, droplet operations may be used to dispense one droplet of BUF037A buffer for example from the buffer reservoir and then transport the droplet into contact with a dried spot of BSA protein (ThermoFisher Scientific, Waltham, MA), for example. Droplet operations may be used to pulse the BUF037A buffer over the BSA spot to ensure complete rehydration, producing a BSA droplet.

Droplet operations may be used to transport the rehydrated BSA droplet to merge with the magnetically bound capture antibodies-bead droplet. Droplet operations may be performed to pulse the two droplets. This step allows the BSA protein (represented as 3206) to coat the unoccupied surface of the magnetic beads 3202, producing a BSA-capture antibodies beads droplet. Any non-reacting protein for example casein or gelatin may be used instead of or in addition to BSA. BSA will be used henceforth as an example only.

Droplet operations as described above may be used to replace any material not bound to the magnetic beads with a wash buffer such as BUF037A buffer for example or PBS/Tween buffer.

In one configuration, droplet operations may be used to separate 10 µL of the stored plasma sample and then transport the plasma to merge with the magnetically bound BSA-capture antibodies-beads droplet. In another configuration, the volume of the plasma used may be less than 10 µL. In a preferred configuration the volume of the plasma may be between 10 µL and 20 µL. In another configuration, the volume of the plasma may be equal to 20 µL. In another configuration, the volume of the plasma may be greater than 20 µL. The plasma droplet may incubate with the BSA-capture antibodies-beads droplet for a predetermined time, allowing the protein of interest to bind to the capture antibodies. This produces an analyte-capture antibodies-beads droplet. In one configuration, only the protein of interest 3208 may bind to the capture antibody. Since the unoccupied surface of the magnetic beads may have been coated with a non-reacting protein, the protein of interest may not bind to the surface of the magnetic bead. In another configuration, another wash step may be performed using droplet operations to replace any droplet contents not bound to the beads with BUF037A buffer for example or PBS/Tween buffer.

In another configuration, droplet operations may be used to dispense one droplet of BUF037A buffer for example or PBS/Tween buffer from the buffer reservoir and then transport the buffer droplet to merge with a dried reagent spot containing specific detection antibodies designed to bind with a different epitope of the analyte of interest. Droplet operations may then be used to pulse the buffer droplet over the dried reagent spot to ensure complete mixing, producing a detection antibodies droplet (step 3104).

Droplet operations may be used to transport this rehydrated detection antibodies droplet to merge with the magnetically bound analyte-capture antibodies-beads droplet. Droplet operations may be used to pulse the analyte-capture antibodies-beads droplet and the detection antibodies droplet.

The two droplets may be incubated for a predetermined time to ensure the detection antibodies 3210 bind to the analyte of interest, producing a sandwiched analyte bead droplet.

Droplet operations may be used to wash away any material not bound to the magnetic beads, such as unbound detection antibodies, replacing the material with BUF037A or PBS/Tween buffer for example.

In another aspect, the components may be eluted from the magnetic beads producing a sandwiched analyte droplet.

In one configuration, the detection antibodies 3210 may be pre-coupled with for example biotin. Therefore, in this configuration the sandwiched analyte droplet may contain biotin. In this method a modified protocol from AssayGenie (Dublin, IE), for example, may be used. In this configuration, one droplet of BUF037A or PBS/Tween buffer for example may be dispensed from the buffer reservoir using droplet operations. This buffer droplet may then be transported to a dried reagent spot containing streptavidin which may be coupled with an enzyme such as alkaline phosphatase (ALP). Droplet operations may be used to pulse the sandwiched analyte droplet with the dried streptavidin-ALP reagent spot, producing a detection-biotin-streptavidin-ALP droplet.

Droplet operations may be used to transport the detection-biotin-streptavidin-ALP droplet to the immunoassay dedicated portion of the detector, shown for example purposes as 622 in FIG. 6. In this configuration, wells in the array detector zone dedicated to immunoassays may contain dried pre-spotted enzyme substrate which when acted upon by the enzyme produces visible light available for optical detection of the protein analyte of interest. In this configuration, 4-methylumbelliferyl phosphate (MUP) may be spotted in the designated wells of the designated detector zone. Droplet operations may be used to transport the detection-biotin-streptavidin-ALP droplet to the wells containing MUP. Droplet operations may then be used to pulse the reagents to ensure complete rehydration and uniform mixing. Once the MUP has been rehydrated, the components may be incubated in the dark or in standard lighting conditions, producing a detection-biotin-streptavidin-ALP-MUP droplet.

In another step of this configuration, droplet operations may be used to dispense one droplet of buffer from the buffer reservoir and transport the droplet to a dried spot of NaOH. Droplet operations may be used to pulse the buffer droplet over the NaOH to ensure complete rehydration and uniform mixing. Upon rehydration the concentration of the NaOH droplet may be 0.75 M.

The rehydrated NaOH droplet may be transported using droplet operations to the one or more wells containing the detection-biotin-streptavidin-ALP-MUP droplet. Upon reaction with the NaOH, the ALP-MUP enzyme cascade reaction may be quenched. At this point, the absorbance may be measured at a preferred wavelength of 405 nm.

In another configuration, the streptavidin may instead be coupled with horseradish peroxidase (HRP), producing a detection-biotin-streptavidin-HRP droplet. Droplet operations may be used to transport the detection-biotin-streptavidin-HRP droplet to the immunoassay dedicated portion of the detector, for example 622. In this configuration, hydrogen peroxide and 3,3',5,5'-tetramethylbenzidine (TMB) may be spotted in the designated wells of the designated detector zone. Droplet operations may be used to transport the detection-biotin-streptavidin-HRP droplet to the wells containing hydrogen peroxide and TMB. Droplet operations may be used to pulse the detection-biotin-streptavidin-HRP Droplet with the dried hydrogen peroxide and TMB reagent spot to ensure complete rehydration of the reagents and uniform mixing.

The droplet may be incubated in the dark, producing a detection-biotin-streptavidin-HRP-TMB droplet.

In another step of this configuration, droplet operations may be used to dispense one droplet of buffer from the buffer reservoir and transport the droplet to a dried spot of H₂SO₄. Droplet operations may be used to pulse the buffer droplet over the H₂SO₄ to ensure complete rehydration and uniform mixing.

The rehydrated H₂SO₄ droplet may be transported using droplet operations to the one or more wells containing the detection-biotin-streptavidin-HRP-TMB droplet. Upon reaction with the H₂SO₄, the HRP-hydrogen peroxide-TMB enzyme cascade reaction may be quenched. At this point, the absorbance may be measured by a spectrophotometer using 450 nm as the primary wavelength and optionally 630 nm as the reference wavelength, where 610 nm to 650 nm is acceptable.

Many suitable enzyme-substrate combinations may be useful. For example, in addition to HRP and ALP, enzymes such as β-galactosidase, acetylcholinesterase, and catalase may be bound to the detection antibody. Additionally, substrates such as 5-Bromo-4-chloro-3-indolyl phosphate with nitro blue tetrazolium (BCIP/NBT) and Fast Red (ThermoFisher Scientific, Waltham, MA) may be pre-spotted in the immuno-assay array of detection features.

In one configuration, the detection antibodies 3210 may be directly coupled with an enzyme such as alkaline phosphatase which may participate in an ELISA assay. In this configuration, the sandwiched analyte droplet may be transported using droplet operations to a dedicated portion of the detector, shown for example purposes as 622 in FIG. 6. In this configuration, wells in the array detector zone dedicated to immunoassays may contain MUP spotted in the designated wells of the designated detector zone. Droplet operations may be used to transport the sandwiched analyte droplet to the wells containing MUP. Droplet operations may then be used to pulse the reagents to ensure complete rehydration and uniform mixing. Once the MUP has been rehydrated, the components may be incubated in the dark, producing a sandwiched analyte-ALP-MUP droplet.

In another step of this configuration, droplet operations may be used to dispense one droplet of buffer from the buffer reservoir and transport the droplet to a dried spot of NaOH. Droplet operations may be used to pulse the buffer droplet over the NaOH to ensure complete rehydration and uniform mixing. Upon rehydration the concentration of the NaOH droplet may be 0.75 M.

The rehydrated NaOH droplet may be transported using droplet operations to the one or more wells containing the sandwiched analyte-ALP-MUP droplet. Upon reaction with the NaOH, the ALP-MUP enzyme cascade reaction may be quenched. At this point, the absorbance may be measured at a preferred wavelength of 405 nm.

### 5.7.2 Detection antibody directly coupled to enzyme

In one configuration, the detection antibodies 3210 may be directly coupled with an enzyme such as horseradish peroxidase which may participate in an ELISA assay. In this configuration, the sandwiched analyte droplet may be transported using droplet operations to a dedicated portion of the detector, shown for example purposes as 622 in FIG. 6. In this configuration, wells in the array detector zone dedicated to immunoassays may contain hydrogen peroxide and TMB may be spotted in the designated wells of the designated detector zone. Droplet operations may be used to transport the sandwiched analyte droplet to the wells containing hydrogen peroxide and TMB. Droplet operations may be used to pulse the sandwiched analyte droplet with the dried hydrogen peroxide and TMB reagent spot to ensure complete rehydration of the reagents and uniform mixing.

The droplet may be incubated in the dark, producing a sandwiched analyte-HRP-TMB droplet.

In another step of this configuration, droplet operations may be used to dispense one droplet of buffer from the buffer reservoir and transport the droplet to a dried spot of H₂SO₄. Droplet operations may be used to pulse the buffer droplet over the H₂SO₄ to ensure complete rehydration and uniform mixing.

The rehydrated H₂SO₄ droplet may be transported using droplet operations to the one or more wells containing the sandwiched analyte-HRP-TMB droplet. Upon reaction with the H₂SO₄, the HRP-hydrogen peroxide-TMB enzyme cascade reaction may be quenched. At this point, the absorbance may be measured by a spectrophotometer using 450 nm as the primary wavelength and optionally 630 nm as the reference wavelength, where 610 nm to 650 nm is acceptable.

### 5.7.3 Enzyme coupled to secondary antibody coupled to detection antibody

In another configuration, the detection antibody may be bound to a secondary antibody coupled with a secondary enzyme such as ALP or HRP. Optical detection may proceed as described above for each enzyme and its corresponding substrate. In one configuration, the antibodies discussed herein may be monoclonal antibodies or polyclonal antibodies or nanobodies or a mixture of two or three of each component.

### 5.7.4 Detector oligonucleotides

In one configuration, the detection antibodies may be coupled with a specific detector oligonucleotide synthesized by, for example, AlphaThera (Philadelphia, PA). In this configuration PCR may be used to quantify the amount of oligonucleotide present in the sample, providing an indirect detection method for the analyte of interest. In this configuration, droplet operations may be used to transport the sandwiched analyte droplet to an appropriate zone of the array detector reserved for PCR based immunoassays. It may be recalled that array detector zone 622 in FIG. 6 is reserved for non-PCR optical based immunoassays. Another array detector zone may be reserved for PCR based immunoassays.

In this configuration, droplet operations may be used to transport the sandwiched analyte droplet to the appropriate zone on the CMOS chip array detector. In or on each detection feature of the appropriate CMOS array detector zone may be spotted dried DNA polymerase, dried dNTPs, and PCR primers which complement the detector oligonucleotide. Droplet operations may be used to pulse the sandwiched analyte droplet over the dried reagent spot to ensure complete rehydration and uniform mixing, producing a sandwiched analyte PCR droplet. After rehydration and mixing, the sandwiched analyte PCR droplet may be heated in a PCR initialization step to 94-96°C for 2-10 minutes to activate the DNA polymerase and to denature any contaminates in the mixture. To begin the denaturation step of PCR cycle 1, the sandwiched analyte PCR droplet may be heated to 94-98°C for 20-30 seconds to denature any double stranded oligonucleotides.

The rehydrated primers bind to the complementary detector sequences. The reaction temperature of each sandwiched analyte PCR droplet may be lowered to 50-65°C for 20-40 seconds to allow the primers to anneal to the detector oligonucleotide sequence. After the primers hybridize to the detector oligonucleotide sequence, the rehydrated DNA Polymerase incorporates dNTPs onto the primers in the elongation step at 72-78°C for Taq DNA Polymerase for approximately 2 minutes for example.

In one configuration, this PCR amplification process may be repeated for 3 or more cycles to generate enough detector oligonucleotide sequences to be detected. In other configurations, generic PCR amplification may proceed for 20 or more cycles or 40 or more cycles. After 4-10 amplification cycles for example, each of the sandwiched analyte PCR droplets may be held at 72-78°C for 5-15 minutes to ensure complete strand extension. The amplification step produces linear duplex detector oligonucleotide copies. The resulting droplets are referred to as amplified sandwiched analyte PCR droplets. Standard quantitative PCR techniques may be used to detect the oligonucleotide strand attached to the analyte.

### 5.8 Samples

Examples of tissues from which NA may extracted using the techniques described herein may include solid tissue, lysed solid tissue, fixed tissue samples, whole blood, plasma, serum, dried blood spots, buccal swabs, other forensic samples, fresh or frozen tissue, biopsy tissue, organ tissue, cultured or harvested cells, and bodily fluids.

In various configurations, a sample may include a biological sample, such as whole blood, lymphatic fluid, serum, plasma, sweat, tear, saliva, sputum, cerebrospinal fluid, amniotic fluid, seminal fluid, vaginal excretion, serous fluid, synovial fluid, pericardial fluid, peritoneal fluid, pleural fluid, transudates, exudates, cystic fluid, bile, urine, gastric fluid, intestinal fluid, fecal samples, liquids containing single or multiple cells, liquids containing organelles, fluidized tissues, fluidized organisms, liquids containing multi-celled organisms, biological swabs and biological washes.

### 5.9 Targets

Targets may include any biological markers. Examples include biological markers for screening or diagnosing cancer. In one configuration, targets include a panel of methylation markers for diagnosing cancer. Examples of panels of probes which may be targeted are set for the in WO2019195268, entitled "Methylation markers and targeted methylation probe panels," and WO2020069350A1, entitled "Methylation markers and targeted methylation probe panel". Targets may be obtained from biopsies, circulating nucleic acid samples, or nucleic acids from other samples.

### 5.10 Diagnostics and screening

The methods of the disclosure may be used for screening or diagnosing a subject for a disease, such as cancer or for selecting a therapy for treating a disease, such as selecting a therapy for treating a cancer.

### 5.11 Examples

The disclosure provides a method for using dual hybridization probes in combination with a methylation sensitive endonuclease digestion reaction to differentiate a methylated target sequence and an unmethylated target sequence as described above with reference to FIG. 26. To demonstrate the feasibility of using a methylation sensitive endonuclease to differentiate a methylated target sequence and an unmethylated target sequence, the cutting event was evaluated using a qPCR assay. Briefly, a methylated target sample and an unmethylated target sample were digested with the methylation sensitive endonuclease Hha I for 30 minutes or 2 hours and then amplified in qPCR assay to determine the amount of amplifiable target present in each sample. In this assay, a methylated target should remain intact and amplifiable, while an unmethylated target should be cut and have less efficient amplification.

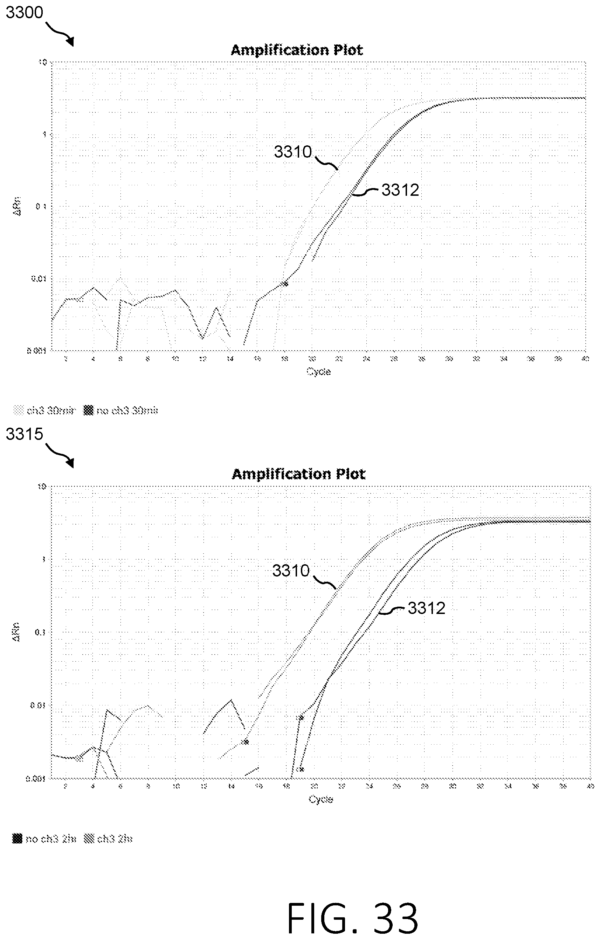

FIG. 33 is a pair of plots 3300 and 3315 showing qPCR amplification plots after 30 minutes and 2 hours, respectively, of cutting with Hha I. The data show that at a lower cycle value, the methylated target plot line 3310 had more material that was amplifiable than the unmethylated target plot line 3312, indicating that the unmethylated target was cleaved.

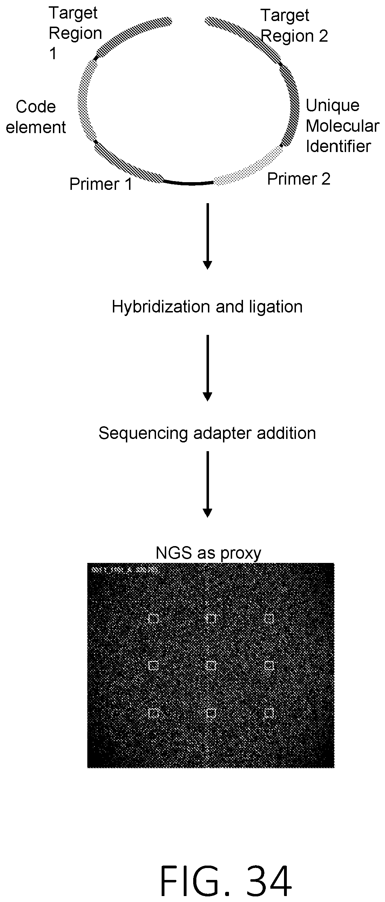

To demonstrate the feasibility of using a panel of code element probes (e.g., padlock probes) to detect different targets in a multiplexed hybridization / ligation assay, we used 12 different targets with 3 targets for testing for allele specificity and 14 probes with unique code elements were designed (some probes targeting multiple regions). As an assay read out, the steps of hybridization and ligation were performed followed by a step to introduce sequencing adapters to generate a library for next generation sequencing (NGS). NGS was used as a proxy for probe binding and ligation. Cluster codes and UMIs enabled counting of ligation events. FIG. 34 is a schematic diagram showing an example of a code element probe and some of the workflow steps in the multiplexed assay.

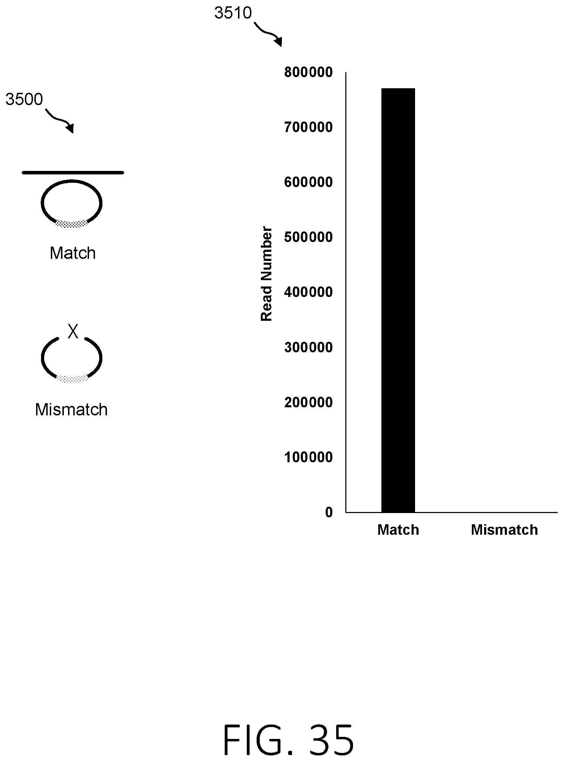

FIG. 35 is a schematic diagram 3500 and a plot 3510 showing the specificity of target interaction in the multiplexed assay. Schematic diagram 3500 shows a match and a mismatch between a probe and target sequence, where a match between a probe and a target results in a closing event, and a mismatch (X) does not. The dark gray arc and the light gray arc in the example probes represent different code elements. Plot 3510 shows the count of read number for the match and mismatch events. The data shows that the multiplexed assay provides high specificity of target interaction, i.e., about a 1000 to 1 read counts for matched vs mismatched.

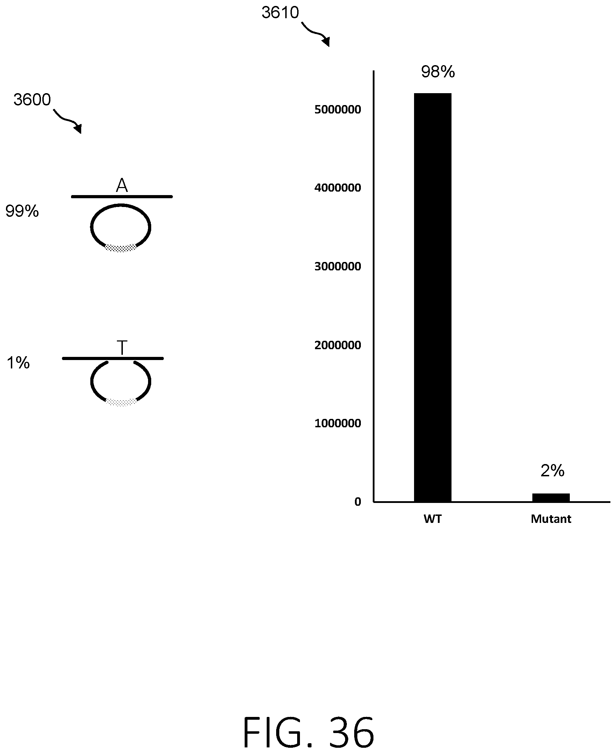

FIG. 36 is a schematic diagram 3600 and a plot 3610 detection of allele ratio in the multiplexed assay. Schematic diagram 3600 shows a match between a probe and a wildtype allele (A), and a mismatch between a probe and mutant allele (T), input into the assay at a ratio of 99% and 1%. The dark gray arc and the light gray arc in the example probes represent different code elements. Plot 3610 shows the read counts for the wildtype (WT) and mutant alleles. The data shows that the read count is to close to the expected ratio for wildtype (A) and mutant alleles (T), at 98% and 2%, respectively.

## Claims

1. A method of conducting a methylation specific assay for a set of targets, the method comprising:
(a) providing a sample comprising a set of targets;
(b) subjecting the sample to a recognition event, in which one or more targets in the set of targets in the sample is hybridized to a recognition element comprising a code from a set of codes, thereby yielding a set of coded targets comprising the one or more targets and the recognition element;
(c) subjecting each recognition element of the set of coded targets to a methylation specific transformation event, in which a molecular transformation of each recognition element of the set of coded targets produces a circular recognition element, thereby yielding a set of circular recognition elements comprising the code;
(d) subjecting each circular recognition element to rolling circle amplification to produce a set of amplified codes;
(e) subjecting each amplified code of the set of amplified codes to a detection event, wherein the detection event determines the presence of the coded targets.

2. The method of claim 1, further comprising:
(f) estimating a quantity of the target of the set of coded targets, wherein the estimating comprises counting the codes of the amplified codes of the set of amplified codes comprising the coded target.

3. The method of claim 1 or 2, wherein each code from the set of codes has a length of 3 to 75 nucleotides.

4. The method of any one of claims 1-3, wherein each code from the set of codes is generated from a 4-ary nucleotide alphabet of adenosine (A), cytosine (C), guanine (G) and thymine (T).

5. The method of any one of claims 1-3, wherein each code from the set of codes is generated from a 3-ary nucleotide alphabet of a set of three selected from the group consisting of A, C, G and T.

6. The method of any one of claims 1-5, wherein the recognition element comprises one or more universal primers.

7. The method of any one of claims 1-6, wherein the transformation event comprises ligating termini of the recognition element in the presence of the target but not in the absence of the target.

8. The method of any one of claims 1-7, further comprising subjecting the set of amplified codes to an exonuclease cleanup step.

9. The method of any one of claims 1-8, wherein the target of the set of targets comprises one or more mutated nucleic acid sequences.

10. The method of claim 9, wherein the one or more mutated nucleic acid sequences comprises:
(a) one or more point mutations;
(b) one or more substitutions;
(c) one or more insertions;
(d) one or more deletions;
(e) one or more copy number variations; or
(f) any combination of (a) to (e).

11. The method of any one of claims 1-8, wherein the target of the set of targets is a methylated nucleic acid sequence.

12. The method of any one of claims 1-11, wherein the detection event comprises obtaining a fluorescence measurement.

13. The method of any one of claims 1-12, wherein the set of coded targets further comprises one or more sequencing adapters for next generation sequencing, and wherein the method is a multiplexed method further comprising performing next generation sequencing.

14. The method of any one of claims 1-13, wherein the detection event comprises imaging with a fluorescence optical measurement system.

15. The method of any one of claims 1-14, wherein the method is performed on an assay substrate comprising wells.

## Patentansprüche

1. Ein Verfahren zum Durchführen eines methylierungsspezifischen Assays für einen Satz von Targets, wobei das Verfahren Folgendes beinhaltet:
(a) Bereitstellen einer Probe, die einen Satz von Targets beinhaltet;
(b) Unterziehen der Probe einem Erkennungsereignis, bei dem ein oder mehrere Targets in dem Satz von Targets in der Probe an ein Erkennungselement hybridisiert sind, das einen Code aus einem Satz von Codes beinhaltet, wodurch ein Satz von codierten Targets erhalten wird, der das eine oder die mehreren Targets und das Erkennungselement beinhaltet;
(c) Unterziehen jedes Erkennungselements des Satzes von codierten Targets einem methylierungsspezifischen Transformationsereignis, bei dem eine molekulare Transformation jedes Erkennungselements des Satzes von codierten Targets ein zirkuläres Erkennungselement erzeugt, wodurch ein Satz von zirkulären Erkennungselementen erhalten wird, der den Code beinhaltet;
(d) Unterziehen jedes zirkulären Erkennungselements einer Rolling-Circle-Amplifikation, um einen Satz von amplifizierten Codes zu erzeugen;
(e) Unterziehen jedes amplifizierten Codes des Satzes von amplifizierten Codes einem Detektionsereignis, wobei das Detektionsereignis das Vorhandensein der codierten Targets bestimmt.

2. Verfahren gemäß Anspruch 1, das ferner Folgendes beinhaltet:
(f) Schätzen einer Menge des Targets des Satzes codierter Targets, wobei das Schätzen das Zählen der Codes der amplifizierten Codes des Satzes amplifizierter Codes beinhaltet, die das codierte Target beinhalten.

3. Verfahren gemäß Anspruch 1 oder 2, wobei jeder Code aus dem Satz von Codes eine Länge von 3 bis 75 Nukleotiden aufweist.

4. Verfahren gemäß einem der Ansprüche 1-3, wobei jeder Code aus dem Satz von Codes aus einem quaternären Nukleotidalphabet von Adenosin (A), Cytosin (C), Guanin (G) und Thymin (T) erzeugt wird.

5. Verfahren gemäß einem der Ansprüche 1 bis 3, wobei jeder Code aus dem Satz von Codes aus einem ternären Nukleotidalphabet aus einem Satz von drei aus der aus A, C, G und T bestehenden Gruppe ausgewählten Nukleotiden gebildet ist.

6. Verfahren gemäß einem der Ansprüche 1-5, wobei das Erkennungselement einen oder mehrere Universalprimer beinhaltet.

7. Verfahren gemäß einem der Ansprüche 1-6, wobei das Transformationsereignis das Ligieren von Enden des Erkennungselements in Gegenwart des Targets, jedoch nicht in Abwesenheit des Targets, beinhaltet.

8. Verfahren gemäß einem der Ansprüche 1-7, das ferner das Unterziehen des Satzes amplifizierter Codes einem Exonuklease-Reinigungsschritt beinhaltet.

9. Verfahren gemäß einem der Ansprüche 1-8, wobei das Target des Satzes von Targets eine oder mehrere mutierte Nukleinsäuresequenzen beinhaltet.

10. Verfahren gemäß Anspruch 9, wobei die eine oder die mehreren mutierten Nukleinsäuresequenzen Folgendes beinhalten:
(a) eine oder mehrere Punktmutationen;
(b) eine oder mehrere Substitutionen;
(c) eine oder mehrere Insertionen;
(d) eine oder mehrere Deletionen;
(e) eine oder mehrere Kopienzahlvariationen; oder
(f) eine beliebige Kombination von (a) bis (e).

11. Verfahren gemäß einem der Ansprüche 1-8, wobei das Target des Satzes von Targets eine methylierte Nukleinsäuresequenz ist.

12. Verfahren gemäß einem der Ansprüche 1-11, wobei das Detektionsereignis das Erhalten einer Fluoreszenzmessung beinhaltet.

13. Verfahren gemäß einem der Ansprüche 1-12, wobei der Satz codierter Targets ferner einen oder mehrere Sequenzierungsadapter für die Sequenzierung der nächsten Generation beinhaltet und wobei das Verfahren ein gemultiplextes Verfahren ist, das ferner das Durchführen der Sequenzierung der nächsten Generation beinhaltet.

14. Verfahren gemäß einem der Ansprüche 1-13, wobei das Detektionsereignis das Abbilden mit einem optischen Fluoreszenzmesssystem beinhaltet.

15. Verfahren gemäß einem der Ansprüche 1-14, wobei das Verfahren auf einem Assay-Substrat durchgeführt wird, das Vertiefungen beinhaltet.

## Revendications

1. Un procédé pour effectuer un dosage spécifique de méthylation pour un ensemble de cibles, le procédé comprenant :
(a) la mise à disposition d'un échantillon comprenant un ensemble de cibles ;
(b) la soumission de l'échantillon à un événement de reconnaissance, où une ou plusieurs cibles dans l'ensemble de cibles dans l'échantillon sont hybridées à un élément de reconnaissance comprenant un code provenant d'un ensemble de codes, donnant ainsi un ensemble de cibles codées comprenant les une ou plusieurs cibles et l'élément de reconnaissance ;
(c) la soumission de chaque élément de reconnaissance de l'ensemble de cibles codées à un événement de transformation spécifique de méthylation, où une transformation moléculaire de chaque élément de reconnaissance de l'ensemble de cibles codées produit un élément de reconnaissance circulaire, donnant ainsi un ensemble d'éléments de reconnaissance circulaires comprenant le code ;
(d) la soumission de chaque élément de reconnaissance circulaire à une amplification par cercle roulant pour produire un ensemble de codes amplifiés ;
(e) la soumission de chaque code amplifié de l'ensemble de codes amplifiés à un événement de détection, dans lequel l'événement de détection détermine la présence des cibles codées.

2. Le procédé de la revendication 1, comprenant en outre :
(f) l'estimation d'une quantité de la cible de l'ensemble de cibles codées, dans lequel l'estimation comprend le comptage des codes parmi les codes amplifiés de l'ensemble de codes amplifiés comprenant la cible codée.

3. Le procédé de la revendication 1 ou de la revendication 2, dans lequel chaque code provenant de l'ensemble de codes a une longueur de 3 à 75 nucléotides.

4. Le procédé de l'une quelconque des revendications 1 à 3, dans lequel chaque code provenant de l'ensemble de codes est généré à partir d'un alphabet nucléotidique à 4 lettres constitué de l'adénosine (A), la cytosine (C), la guanine (G) et la thymine (T).

5. Le procédé de l'une quelconque des revendications 1 à 3, dans lequel chaque code provenant de l'ensemble de codes est généré à partir d'un alphabet nucléotidique à 3 lettres d'un ensemble de trois lettres sélectionnées dans le groupe constitué de A, C, G et T.

6. Le procédé de l'une quelconque des revendications 1 à 5, dans lequel l'élément de reconnaissance comprend une ou plusieurs amorces universelles.

7. Le procédé de l'une quelconque des revendications 1 à 6, dans lequel l'événement de transformation comprend la ligature de terminaisons de l'élément de reconnaissance en présence de la cible mais pas en l'absence de la cible.

8. Le procédé de l'une quelconque des revendications 1 à 7, comprenant en outre la soumission de l'ensemble de codes amplifiés à une étape de nettoyage par exonucléase.

9. Le procédé de l'une quelconque des revendications 1 à 8, dans lequel la cible de l'ensemble de cibles comprend une ou plusieurs séquences d'acide nucléique muté.

10. Le procédé de la revendication 9, dans lequel les une ou plusieurs séquences d'acide nucléique muté comprennent :
(a) une ou plusieurs mutations ponctuelles ;
(b) une ou plusieurs substitutions ;
(c) une ou plusieurs insertions ;
(d) une ou plusieurs délétions ;
(e) une ou plusieurs variations de nombre de copies ; ou
(f) toute combinaison de (a) à (e).

11. Le procédé de l'une quelconque des revendications 1 à 8, dans lequel la cible de l'ensemble de cibles est une séquence d'acide nucléique méthylé.

12. Le procédé de l'une quelconque des revendications 1 à 11, dans lequel l'événement de détection comprend l'obtention d'une mesure de fluorescence.

13. Le procédé de l'une quelconque des revendications 1 à 12, dans lequel l'ensemble de cibles codées comprend en outre un ou plusieurs adaptateurs de séquençage pour un séquençage de nouvelle génération, et dans lequel le procédé est un procédé multiplexé comprenant en outre la réalisation d'un séquençage de nouvelle génération.

14. Le procédé de l'une quelconque des revendications 1 à 13, dans lequel l'événement de détection comprend la réalisation d'images au moyen d'un système de mesure optique par fluorescence.

15. Le procédé de l'une quelconque des revendications 1 à 14, le procédé étant réalisé sur un substrat de dosage comprenant des puits.
